(19) Europäisches Patentamt
European Patent Office
Office européen des brevets

(11) Veröffentlichungsnummer: **0 407 342 A2**

(12) # EUROPÄISCHE PATENTANMELDUNG

(21) Anmeldenummer: **90810482.1**

(22) Anmeldetag: **27.06.90**

(51) Int. Cl.5: **C07D 403/10**, C07D 239/46, C07D 239/36, C07D 239/56, A61K 31/505

(30) Priorität: **06.07.89 CH 2509/89**

(43) Veröffentlichungstag der Anmeldung: **09.01.91 Patentblatt 91/02**

(84) Benannte Vertragsstaaten: **AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Herold, Peter, Dr.**
**Mattweg 19**
**CH-4144 Arlesheim(CH)**
Erfinder: **Bühlmayer, Peter, Dr.**
**Hangstrasse 18**
**CH-4144 Arlesheim(CH)**

(54) **Neue Pyrimidin Derivate.**

(57) Verbindungen der Formel

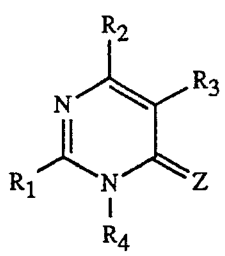

(I),

ihre Tautomere und Salze, worin
Z für O, S oder N(R) und R für Wasserstoff oder einen aliphatischen Kohlenwasserstoffrest steht;
$R_1$ einen gegebenenfalls substituierten aliphatischen Kohlenwasserstoffrest, einen cycloaliphatischen oder araliphatischen Kohlenwasserstoffrest oder einen aromatischen Rest bedeutet;
$R_2$ und $R_3$ unabhängig voneinander Halogen, Acyl, einen aromatischen Rest, gegebenenfalls substituiertes Amino, gegebenenfalls verestertes oder amidiertes Carboxy bedeuten; oder
$R_2$ für $-Z_1-R'_2$ und $R_3$ für $-Z_2-R'_3$ stehen, wobei $Z_1$ und $Z_2$ unabhängig voneinander für eine Bindung, O oder $S(O)_n$ stehen und n für 0, 1 oder 2 steht, und $R'_2$ und $R'_3$ unabhängig voneinander für Wasserstoff, einen araliphatischen oder aliphatischen Kohlenwasserstoffrest stehen, wobei letzterer gegebenenfalls substituiert und gegebenenfalls durch -O-oder $-S(O)_n-$ unterbrochen ist, wobei n für 0, 1 oder 2 steht; oder
$R_2$ und $R_3$ gemeinsam für Propylen oder Butylen oder für die Partialstruktur der Formel -CH=CH-CH=CH-, in der gegebenenfalls eine oder zwei der Methingruppen durch -N= ersetzt ist;
$R_4$ für eine Gruppe der Formel

EP 0 407 342 A2

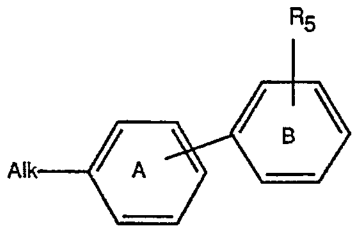

(Ia)

steht, in der
Alk einen zweiwertigen aliphatischen Kohlenwasserstoff bedeutet;
$R_5$ COOH, $SO_3H$, Halogenalkansulfamoyl, $PO_2H_2$, $PO_3H_2$ oder 5-Tetrazolyl bedeutet;
die Ringe A und B bzw. der durch $R_2$ und $R_3$ gemeinsam gebildete (hetero-)aromatische Ring unabhängig voneinander gegebenenfalls substituiert sind, können beispielsweise als Arzneimittelwirkstoffe verwendet werden.

# NEUE PYRIMIDIN-DERIVATE

Die Erfindung betrifft neue Pyrimidin-Derivate der Formel

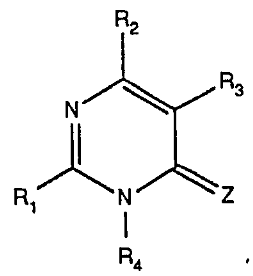

(I),

ihre Tautomere und Salze, worin
Z für O, S oder N(R) und R für Wasserstoff oder einen aliphatischen Kohlenwasserstoffrest steht;
$R_1$ einen gegebenenfalls substituierten aliphatischen Kohlenwasserstoffrest, einen cycloaliphatischen oder araliphatischen Kohlenwasserstoffrest oder einen aromatischen Rest bedeutet;
$R_2$ und $R_3$ unabhängig voneinander Halogen, Acyl, einen aromatischen Rest, gegebenenfalls substituiertes Amino, gegebenenfalls verestertes oder amidiertes Carboxy bedeuten; oder
$R_2$ für $-Z_1-R'_2$ und $R_3$ für $-Z_2-R'_3$ stehen, wobei $Z_1$ und $Z_2$ unabhängig voneinander für eine Bindung, O oder $S(O)_n$ stehen und n für 0, 1 oder 2 steht, und $R'_2$ und $R'_3$ unabhängig voneinander für Wasserstoff, einen araliphatischen oder aliphatischen Kohlenwasserstoffrest stehen, wobei letzterer gegebenenfalls substituiert und gegebenenfalls durch -O-oder $-S(O)_n-$ unterbrochen ist, wobei n für 0, 1 oder 2 steht; oder
$R_2$ und $R_3$ gemeinsam für Propylen oder Butylen oder für die Partialstruktur der Formel -CH = CH-CH = CH-, in der gegebenenfalls eine oder zwei der Methingruppen durch -N = ersetzt ist;
$R_4$ für eine Gruppe der Formel

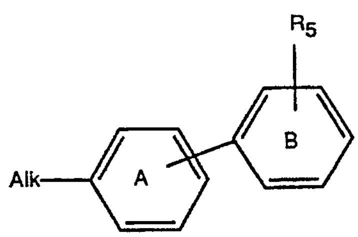

(Ia)

steht, in der
Alk einen zweiwertigen aliphatischen Kohlenwasserstoff bedeutet;
$R_5$ COOH, $SO_3H$, Halogenalkansulfamoyl, $PO_2H_2$, $PO_3H_2$ oder 5-Tetrazolyl bedeutet; die Ringe A und B bzw. der durch $R_2$ und $R_3$ gemeinsam gebildete (hetero-)aromatische Ring unabhängig voneinander gegebenenfalls substituiert sind;
Verfahren zu ihrer Herstellung, pharmazeutische Präparate und ihre Verwendung.

Verbindungen der Formel I können als Protonentautomere vorliegen. Bedeutet z.B. $R_2$ Hydroxy, können entsprechende Verbindungen im Gleichgewicht mit den tautomeren 4-Oxo-Derivaten stehen.

Die Verbindungen der Formel I können als, insbesondere pharmazeutisch verwendbare, Salze vorliegen. Weisen die erfindungsgemässen Verbindungen mindestens ein basisches Zentrum auf, können sie somit Säureadditionssalze bilden. Diese werden beispielsweise mit anorganischen Säuren, wie Mineralsäuren, z.B. Schwefelsäure, einer Phosphor- oder Halogenwasserstoffsäure, oder mit organischen Carbonsäuren, wie gegebenenfalls, z.B. durch Halogen, substituierte ($C_1$-$C_4$-)Alkancarbonsäuren, z.B. Essigsäure, wie gegebenenfalls ungesättigte Dicarbonsäuren, z.B. Oxal-, Malon-, Bernstein-, Malein-, Fumar-, Phthal-, oder Terephthalsäure, wie Hydroxycarbonsäuren, z.B. Ascorbin-, Glykol-, Milch-, Äpfel-, Wein- oder Citronensäure, wie Aminosäuren, z.B. Asparagin- oder Glutaminsäure, Benzoesäure oder mit organischen Sulfonsäuren, wie gegebenenfalls, z.B. durch Halogen, substituierte ($C_1$-$C_4$-)Alkan- oder Arylsulfonsäuren, z.B. Methan- oder Toluolsulfonsäure, gebildet. Entsprechende Säureadditionssalze können auch mit einem gegebenenfalls zusätzlich vorhandenen basischen Zentrum gebildet werden. Ferner können die erfindungsgemässen

Verbindungen mit einer aciden Gruppe ($R_5$ = z.B. COOH oder 5-Tetrazolyl) Salze mit Basen bilden. Geeignete Salze mit Basen sind beispielsweise Metall-, wie Alkali- oder Erdalkalimetallsalze, z.B. Natrium-, Kalium- oder Magnesiumsalze, oder Salze mit Ammoniak oder von substituierten organischen Aminen, wie Morpholin, Thiomorpholin, Piperidin, Pyrrolidin, wie Mono-, Di- bzw. Triniederalkylminen oder Mono-, Di- bzw. Trihydroxyniederalkylaminen, z.B. Mono-, Di- oder Triethanolamin. Mononiederalkylamine sind beispielsweise Ethyl- oder tert.-Butylamin. Diniederalkylamine sind z.B. Diethyl- oder Dipropylamin, und als Triniederalkylamine kommen z.B. Triethyl-, Tributylamin oder Dimethylpropylamin in Betracht. Weiterhin können entsprechende innere Salze gebildet werden. Umfasst sind ferner für pharmazeutische Verwendungen nicht geeignete Salze, da diese beispielsweise für die Isolierung bzw. Reinigung erfindungsgemässer Verbindungen oder deren pharmazeutisch verwendbarer Salze eingesetzt werden.

Ein aliphatischer Kohlenwasserstoffrest ist beispielsweise Niederalkyl, Niederalkenyl und in zweiter Linie Niederalkinyl.

Ein cycloaliphatischer Kohlenwasserstoffrest stellt beispielsweise Cycloalkyl und in zweiter Linie Cycloalkenyl dar.

Als araliphatischer Rest kommt insbesondere Phenylniederalkyl, ferner -niederalkenyl und -niederalkinyl in Frage.

Ein aromatischer Rest bedeutet beispielsweise einen carbocyclischen oder heterocyclischen aromatischen Rest, insbesondere Phenyl, ferner Naphthyl, oder insbesondere einen entsprechenden 5- oder 6-gliedrigen und monocyclischen Rest, der bis zu vier gleiche oder verschiedene Heteroatome, wie Stickstoff-, Sauerstoff- bzw. Schwefelatome, vorzugsweise ein, zwei, drei oder vier Stickstoffatome, ein Sauerstoff- oder ein Schwefelatom, aufweist. Entsprechende 5-gliedrige Heteroarylreste sind z.B. monoaza-, diaza-, triaza-, tetraaza-, monooxa- oder monothia-cyclische Arylreste, wie Pyrrolyl, Pyrazolyl, Imidazolyl, Triazolyl, Tetrazolyl, Furyl und Thienyl, während als entsprechende 6-gliedrige Reste insbesondere Pyridyl in Frage kommt.

Acyl bedeutet insbesondere Niederalkanoyl, Phenylniederalkanoyl oder gegebenenfalls substituiertes Benzoyl.

Substituiertes Amino ist beispielsweise durch einen aliphatischen oder araliphatischen Kohlenwasserstoffrest, wie Niederalkyl, Niederalkenyl, Niederalkinyl, Phenylniederalkyl, -niederalkenyl oder -niederalkinyl, unabhängig voneinander mono- oder disubstituiert oder durch einen zweiwertigen aliphatischen Kohlenwasserstoffrest, wie Niederalkylen oder Niederalkylenoxyniederalkylen, disubstituiertes Amino, beispielsweise Niederalkyl-, Niederalkenyl-, Niederalkinyl-, Phenylniederalkyl-, Phenylniederalkenyl-, Phenylnieder alkinyl-, Diniederalkyl-, N-Niederalkyl-N-Phenylniederalkyl-, Di-phenylniederalkylamino, Niederalkylenamino oder Niederalkylenoxyniederalkylenamino.

Verestertes Carboxy bedeutet beispielsweise Carboxy, welches durch einen aliphatischen Alkohol verestert ist, wobei dieser sich von einem aliphatischen Kohlenwasserstoffrest ableitet, wie Niederalkyl, Niederalkenyl und in zweiter Linie Niederalkinyl, und der gegebenenfalls durch -O- unterbrochen ist, wie Niederalkoxyniederalkyl, -niederalkenyl und -niederalkinyl, beispielsweise Niederalkoxy-, Niederalkoxyniederalkoxy- oder Niederalkenyloxy-carbonyl.

Amidiertes Carboxy ist beispielsweise Carbamoyl, in welchem die Aminogruppe, beispielsweise wie vorstehend angeben, gegebenenfalls durch einen aliphatischen oder araliphatischen Kohlenwasserstoffrest unabhängig voneinander mono- oder disubstituiert oder durch einen zweiwertigen aliphatischen Kohlenwasserstoffrest, der gegebenenfalls durch -O- unterbrochen ist, disubstituiert ist, wobei als zweiwertiger aliphatischer Kohlenwasserstoffrest, der gegebenenfalls durch -O- unterbrochen sein kann, insbesondere Niederalkylen oder Niederalkylenoxyniederalkylen in Frage kommt.

Ein aliphatischer Kohlenwasserstoffrest, der durch -O- unterbrochen ist, bedeutet insbesondere Niederalkoxyniederalkyl, -niederalkenyl oder -niederalkinyl, Niederalkenyloxyniederalkyl, -niederalkenyl oder -niederalkinyl, während als aliphatischer Kohlenwasserstoffrest, der durch $-S(O)_n-$ unterbrochen ist, insbesondere Niederalkylthioniederalkyl, -niederalkenyl oder -niederalkinyl, Niederalkansulfinylniederalkyl, -sulfonylniederalkyl, Niederalkenylthioniederalkyl, -sulfinylniederalkyl, -sulfonylniederalkyl, Niederalkinylthioniederalkyl, -sulfinylniederalkyl, -sulfonylniederalkyl.

Eine Partialstruktur der Formel -CH = CH-CH = CH-, in der eine oder zwei der Methingruppen durch -N = ersetzt ist, bedeutet beispielsweise -N = CH-CH = CH-, -CH = N-CH = CH-, -CH = CH-N = CH-, -CH = CH-CH = N-, -N = CH-N = CH- oder -CH = N-CH = N-.

Ein zweiwertiger aliphatischer Kohlenwasserstoffrest bedeutet beispielsweise Niederalkylen oder Niederalkyliden.

Halogenalkansulfamoyl bedeutet insbesondere Halogenniederalkansulfamoyl.

Substituenten eines aliphatischen Kohlenwasserstoffs, der gegebenenfalls durch O oder $S(O)_n$ unterbrochen ist, sind beispielsweise ausgewählt aus Halogen, gegebenenfalls verethertem Hydroxy, $S(O)_n$-R,

gegebenenfalls substituiertem Amino oder gegebenenfalls verestertem oder amidiertem Carboxy. Bevorzugte Substituenten eines aliphatischen Kohlenwasserstoffs $R_1$ sind z.B. Halogen oder Hydroxy. Entsprechende substituierte aliphatische Kohlenwasserstoffreste weisen vorzugsweise einen Substituenten auf, der in erster Linie bei längerkettigen Resten in höherer als der $\alpha$-Stellung lokalisiert ist. Als bevorzugte aliphatische Kohlenwasserstoffreste seien z.B. Halogenniederalkyl, ferner -niederalkenyl, -niederalkinyl, Hydroxyniederalkyl, ferner -niederalkenyl oder -niederalkinyl, genannt.

Verethertes Hydroxy bedeutet z.B. mit einem aliphatischen Alkohol verethertes Hydroxy, insbesondere Niederalkoxy oder Niederalkenyloxy, und steht ebenso für einen Phenylniederalkoxy- oder Phenoxyrest.

(Hetero-)Aromatische Reste, einschliesslich der Ringe A und B sowie, falls $R_2$ und $R_3$ für die Partialstruktur der Formel -CH=CH-CH=CH- stehen, in der gegebenenfalls eine oder zwei der Methingruppen durch -N= ersetzt ist, der durch diese Partialstruktur gebildete (hetero-)aromatische Ring, sind insbesondere, sofern nicht abweichend definiert, jeweils unsubstituiert oder ein- oder mehrfach, z.B. zwei- oder dreifach, insbesondere z.B. durch einen Substituenten ausgewählt aus Halogen, gegebenenfalls verethertes Hydroxy, $S(O)_m$-R und einen gegebenenfalls, z.B. durch Halogen oder Hydroxy, substituierten Kohlenwasserstoffrest, der gegebenenfalls durch -O- unterbrochen ist, substituiert. Bevorzugt ist unsubstituiertes oder ein- oder mehrfach, z.B. zwei- oder dreifach, z.B. durch einen Substituenten ausgewählt aus der Gruppe bestehend aus Niederalkyl, Niederalkoxy, Halogen, Trifluormethyl und Hydroxy, substituiertes Phenyl.

Vor- und nachstehend sind ungesättigte aliphatische, cycloaliphatische und araliphatische Substituenten in erster Linie nicht über das C-Atom, von dem eine Mehrfachbindung ausgeht, mit einem aromatischen Rest verknüpft.

Die vor- und nachstehend verwendeten Allgemeindefinitionen haben, sofern nicht abweichend definiert, folgende Bedeutungen:

Der Ausdruck "Nieder" bedeutet, dass entsprechende Gruppen und Verbindungen jeweils insbesondere bis und mit 7, vorzugsweise bis und mit 4, Kohlenstoffatome enthalten.

Halogen ist insbesondere Halogen mit Atomnummer bis und mit 35, wie Fluor, Chlor oder Brom, und umfasst ferner Iod.

Niederalkyl ist insbesondere $C_1$-$C_7$-Alkyl und bedeutet z.B. Methyl, Ethyl, n-Propyl, Isopropyl, n-Butyl, Isobutyl, sek.-Butyl, tert.-Butyl und umfasst ferner entsprechende Pentyl-, Hexyl- und Heptylreste. Bevorzugt ist $C_1$-$C_4$-Alkyl.

Niederalkenyl bedeutet insbesondere $C_3$-$C_7$-Alkenyl und ist z.B. 2-Propenyl oder 1-, 2-oder 3-Butenyl. Bevorzugt ist $C_3$-$C_5$-Alkenyl.

Niederalkinyl ist insbesondere $C_3$-$C_7$-Alkinyl und bedeutet vorzugsweise Propargyl.

Cycloalkyl ist insbesondere $C_3$-$C_7$-Cycloalkyl und bedeutet z.B. Cyclopropyl, Cyclobutyl, Cyclopentyl, Cyclohexyl und Cycloheptyl. Bevorzugt ist Cyclopentyl und Cyclohexyl.

Cycloalkenyl ist insbesondere $C_3$-$C_7$-Cycloalkenyl und bedeutet vorzugsweise Cyclopent-2-, -3-enyl, Cyclohex-2- und -3-en-yl.

Phenylniederalkyl ist insbesondere Phenyl-$C_1$-$C_4$-alkyl und bedeutet vorzugsweise Benzyl, 1- und 2-Phenethyl, während Phenylniederalkenyl und Phenylniederalkinyl insbesondere Phenyl-$C_3$-$C_5$-alkenyl und -alkinyl bedeuteten, insbesondere 3-Phenylallyl und 3-Phenylpropargyl.

Pyrrolyl ist z.B. 2- oder 3-Pyrrolyl. Pyrazolyl ist 3- oder 4-Pyrazolyl. Imidazolyl ist 2-oder 4-Imidazolyl. Triazolyl ist z.B. 1,3,5-1H-Triazol-2-yl oder 1,3,4-Triazol-2-yl. Tetrazolyl ist z.B. 1,2,3,4-Tetrazol-5-yl, Furyl ist 2- oder 3-Furyl und Thienyl ist 2- oder 3-Thienyl, während als Pyridyl 2-, 3- und 4-Pyridyl in Frage kommt.

Niederalkanoyl bedeutet insbesondere $C_1$-$C_7$-Alkanoyl und ist z.B. Formyl, Acetyl, Propionyl, Butyryl, Isobutyryl oder Pivaloyl. Bevorzugt ist $C_2$-$C_5$-Alkanoyl.

Phenylniederalkanoyl bedeutet insbesondere Phenyl-$C_2$-$C_7$-alkanoyl und ist z.B. Phenylacetyl oder 2- oder 3-Phenylpropionyl. Bevorzugt ist Phenyl-$C_2$-$C_4$-alkanoyl.

Niederalkylen bedeutet insbesondere $C_1$-$C_7$-Alkylen, ist geradkettig oder verzweigt und bedeutet insbesondere Methylen, Ethylen, Propylen oder Butylen sowie 1 ,2-Propylen, 2-Methyl-1,3-propylen und 2,2-Dimethyl-1,3-propylen. Bevorzugt ist $C_2$-$C_5$-Alkylen.

Niederalkylenoxyniederalkylen bedeutet insbesondere $C_1$-$C_4$-Alkylenoxy-$C_2$-$C_4$-alkylen, vorzugsweise Ethylenoxyethylen.

Niederalkylamino bedeutet insbesondere $C_1$-$C_7$-Alkylamino und ist z.B. Methyl-, Ethyl-, n-Propyl- und Isopropyl-amino. Bevorzugt ist $C_1$-$C_4$-Alkylamino.

Niederalkenylamino bedeutet vorzugsweise $C_3$-$C_5$-Alkenylamino, wie Allyl- und Methallylamino.

Niederalkinylamino bedeutet vorzugsweise $C_3$-$C_5$-Alkinylamino, wie Propargylamino.

Phenylniederalkylamino bedeutet vorzugsweise Phenyl-$C_1$-$C_4$-alkylamino, insbesondere Benzyl-, 1- und 2-Phenylethylamino.

Phenylniederalkenylamino bedeutet vorzugsweise Phenyl- $C_3$-$C_5$-alkenyl-amino, insbesondere Phenylallylamino und 3-Phenylmethallylamino.

Phenylniederalkinylamino bedeutet vorzugsweise Phenyl-$C_3$-$C_5$-alkinylamino, insbesondere 3-Phenylpropargylamino.

Diniederalkylamino bedeutet insbesondere Di-$C_1$-$C_4$-alkylamino, wie Dimethyl-, Di-ethyl-, Di-n-propyl-, Methyl-propyl-, Methyl-ethyl-, Methyl-butyl-amino und Dibutylamino.

N-Niederalkyl-N-phenylniederalkylamino bedeutet insbesondere N-$C_1$-$C_4$-Alkyl-N-phenyl-$C_1$-$C_4$-alkylamino, vorzugsweise Methyl-benzyl-amino und Ethyl-benzyl-amino.

Diphenylniederalkylamino bedeutet insbesondere Di-phenyl-$C_1$-$C_4$-alkyl-amino, vorzugsweise Dibenzylamino und Di-phenylethyl-amino.

Niederalkylenamino bedeutet insbesondere $C_2$-$C_6$-Alkylenamino, wie 1-Aziridinyl, 1-Azetidinyl, 1-Pyrrolidinyl, 1-Piperidinyl oder 1-Azepidinyl, während als Niederalkylenoxyniederalkylenamino insbesondere 4-Morpholinyl in Frage kommt.

Niederalkoxy bedeutet insbesondere $C_1$-$C_7$-Alkoxy und ist z.B. Methoxy, Ethoxy, n-Propyloxy, Isopropyloxy, n-Butyloxy, Isobutyloxy, sek.-Butyloxy, tert.-Butyloxy und umfasst ferner entsprechende Pentyloxy-, Hexyloxy- und Heptyloxyreste. Bevorzugt ist $C_1$-$C_4$-Alkoxy.

Niederalkenyloxy bedeutet insbesondere $C_3$-$C_7$-Alkenyloxy und ist z.B. Allyloxy oder But-2-en- oder But-3-enyloxy. Bevorzugt ist $C_3$-$C_5$-Alkenyloxy.

Niederalkoxyniederalkyl bedeutet insbesondere $C_1$-$C_4$-Alkoxy-$C_1$-$C_4$-alkyl, wie 2-Methoxy-ethyl, 2-Ethoxy-ethyl, 2-n-Propyloxy-ethyl oder Ethoxymethyl.

Niederalkoxyniederalkenyl bedeutet insbesondere $C_1$-$C_4$-Alkoxy-$C_3$-$C_4$-alkenyl, wie 3-Methoxy-allyl, 3-Ethoxy-allyl oder 2-n-Propyloxy-allyl, während als Niederalkoxyniederalkinyl bedeutet insbesondere $C_1$-$C_4$-Alkoxy-$C_3$-$C_4$-alkinyl, wie 3-Methoxypropargyl, in Frage kommt.

Niederalkoxycarbonyl bedeutet insbesondere $C_2$-$C_8$-Alkoxycarbonyl und ist z.B. Methoxy-, Ethoxy-, Propyloxy- oder Pivaloyloxy-carbonyl. Bevorzugt ist $C_2$-$C_5$-Alkoxycarbonyl.

Niederalkoxyniederalkoxycarbonyl bedeutet insbesondere $C_1$-$C_4$-Alkoxy-$C_1$-$C_4$-alkoxycarbonyl, vorzugsweise Ethoxy-ethoxycarbonyl, Methoxyethoxycarbonyl und Isopropyloxy-ethoxycarbonyl.

Niederalkenyloxycarbonyl bedeutet insbesondere $C_3$-$C_5$-Alkenyloxy-carbonyl, vorzugsweise Allyloxycarbonyl, während Niederalkinyloxycarbonyl insbesondere $C_3$-$C_5$-Alkinyloxy-carbonyl, wie Propargyloxycarbonyl, bedeutet.

Niederalkenyloxyniederalkyl bedeutet insbesondere $C_3$-$C_5$-Alkenyloxy-$C_1$-$C_4$-niederalkyl, wie 2-Allylethyl, 2- oder 3-Allyl-propyl.

Niederalkenyloxyniederalkenyl bedeutet insbesondere $C_3$-$C_5$-Alkenyloxy-$C_3$-$C_5$-alkenyl, wie 3-Allyloxyallyl, während Niederalkenyloxyniederalkinyl insbesondere $C_3$-$C_5$-Alkenyloxy-$C_3$-$C_5$-alkinyl, wie 3-Allyl-propargyl, bedeutet.

Niederalkylthio bedeutet insbesondere $C_1$-$C_7$-Alkylthio und ist z.B. Methyl-, Ethyl-, n-Propyl-, Isopropyl-, n-Butyl-, Isobutyl-, sek.-Butyl- oder tert.-Butylthio. Bevorzugt ist $C_1$-$C_4$-Alkylthio.

Niederalkansulfinyl bzw. -sulfonyl bedeutet insbesondere $C_1$-$C_7$-Alkansulfinyl bzw. -sulfonyl und ist z.B. Methan-, Ethan-, n-Propan- oder Isopropan-sulfinyl bzw. -sulfonyl. Bevorzugt ist $C_1$-$C_4$-Alkansulfinyl bzw. -sulfonyl.

Niederalkenylthio bedeutet insbesondere $C_3$-$C_5$-Alkenylthio, wie Allylthio, während Niederalkinylthio insbesondere $C_3$-$C_5$-Alkinylthio, wie Propargylthio, bedeutet.

Niederalkylthioniederalkyl bedeutet insbesondere $C_1$-$C_4$-Alkylthio-$C_1$-$C_4$-alkyl, wie Ethylthiomethyl, 2-Ethylthio-ethyl, 2-Methylthioethyl, 2-Isopropylthioethyl, während als Niederalkansulfinyl- bzw. Niederalkansulfonylniederalkyl insbesondere entsprechende $C_1$-$C_4$-Alkansulfinyl- bzw. $C_1$-$C_4$-Alkansulfonyl-$C_1$-$C_4$-alkylreste in Frage kommen.

Niederalkylthioniederalkenyl bzw. -niederalkinyl bedeutet insbesondere $C_1$-$C_4$-Alkylthio-$C_3$-$C_5$-alkenyl bzw. -alkinyl.

Niederalkenylthioniederalkyl bedeutet insbesondere $C_3$-$C_5$-Alkenylthio-$C_1$-$C_4$-alkyl, wie 2-Allylthioethyl oder 3-Allylpropyl, während als Niederalkenylsulfinyl- bzw. -sulfonylniederalkyl insbesondere $C_3$-$C_5$-Alkenylsulfinyl- bzw. -sulfonyl-$C_1$-$C_4$-alkyl in Frage kommt.

Niederalkinylthioniederalkyl bedeutet insbesondere $C_3$-$C_5$-Alkinylthio-$C_1$-$C_4$-alkyl, wie 2-Propargylthioethyl oder 3-Propargylpropyl, während als Niederalkinylsulfinyl- bzw. -sulfonylniederalkyl insbesondere $C_3$-$C_5$-Alkinylsulfinyl- bzw. -sulfonyl-$C_1$-$C_4$-alkyl in Frage kommt.

Niederalkyliden bedeutet insbesondere C-C-Alkyliden, wie Ethyliden, 1,1- oder 2,2-Propyliden, ferner 1,1- oder 2,2-Butyliden. Bevorzugt ist $C_2$-$C_3$-Alkyliden.

Halogenalkylsulfamoyl bedeutet insbesondere Halogen-$C_1$-$C_7$-alkansulfamoyl, insbesondere Halogen-$C_1$-$C_4$-alkylsulfamoyl, und ist z.B. Trifluormethan-, Difluormethan- oder 1,1,2-Trifluorethansulfamoyl.

Halogenniederalkyl bedeutet insbesondere Halogen-$C_1$-$C_4$-alkyl, wie Trifluormethyl, 1,1,2-Trifluor-2-chlor-ethyl oder Chlormethyl.

Halogenniederalkenyl bedeutet insbesondere Halogen-$C_3$-$C_5$-alkenyl, wie 3-Chlorallyl.

Halogenniederalkinyl ist insbesondere Halogen-$C_3$-$C_5$-alkinyl, wie 3-Chlorpropargyl.

Hydroxyniederalkyl bedeutet insbesondere Hydroxy-$C_1$-$C_4$-alkyl, wie Hydroxymethyl, 2-Hydroxyethyl oder 3-Hydroxypropyl.

Hydroxyniederalkenyl bedeutet insbesondere Hydroxy-$C_3$-$C_5$-alkenyl, wie 3-Hydroxyallyl.

Hydroxyniederalkinyl bedeutet insbesondere Hydroxy-$C_3$-$C_5$-alkinyl, wie 3-Hydroxypropargyl.

Phenylniederalkoxy bedeutet insbesondere Phenyl-$C_1$-$C_4$-alkoxy, wie Benzyloxy, 1- oder 2- Phenylethoxy, 1-, 2- oder 3- Phenylpropyloxy.

Ausgedehnte pharmakologische Untersuchungen haben ergeben, dass die erfindungsgemässen Verbindungen ausgeprägte Angiotensin-II-antagonisierende Eigenschaften aufweisen.

Bekanntlich hat Angiotensin-II starke vasokonstriktorische Eigenschaften, stimuliert ausserdem die Aldosteronsektretion und bewirkt somit eine deutliche Natrium/Wasser-Retention. Die Folge der Angiotensin-II-Aktivität manifestiert sich in einer Erhöhung des Blutdruckes.

Die Bedeutung von Angiotensin-II-Antagonisten besteht darin, durch kompetitive Hemmung der Bindung von Angiotensin-II an die Rezeptoren die durch Angiotensin-II bewirkten vasokonstriktorischen und Aldosteronsekretion- stimulierenden Effekte zu unterdrücken.

Die Angiotensin-II-antagonisierenden Eigenschaften der erfindungsgemässen Verbindungen können im Angiotensin-II-Bindungstest erfasst werden. Dabei werden glatte Muskelzellen der Ratte aus homogenisierter Rattenaorta verwendet. Das feste Zentrifugat wird in 50 mM- Tris-Puffer, pH 7,4, unter Einsatz von Peptidaseinhibitoren suspendiert. Proben werden 60 Minuten bei 25°C mit $^{125}$I-Angiotensin-II (0,175 nM) und einer variierenden Konzentration Angiotensin-II oder der Testsubstanz inkubiert. Die Inkubation wird dann durch Zugabe von mit eiskaltem Phosphat gepuffertem Kochsalz beendet, es wird durch Whatman GF/F Filter filtriert. Die Filter werden mit einem Gamma-Zähler gezählt. Aus der Dosis-Wirkungs-Kurve werden die $IC_{50}$-Werte bestimmt. Für die erfindungsgemässen Verbindungen wurden $IC_{50}$-Werte ab etwa 10 nM ermittelt.

Zur Bestimmung der Angiotensin-II induzierten Vasokonstriktion können Untersuchungen an dem isolierten Kaninchen-Aortaring herangezogen werden. Hierzu werden von jeder Brustaorta Ringe präpariert und zwischen 2 parallele Klammern bei einer anfänglich bestehenden Spannung von 2 g fixiert. Anschliessend werden die Ringe bei 37°C in 20 ml eines Gewebebades getaucht und mit 95 % $O_2$ und 5 % $CO_2$ begast. Die isometrischen Reaktionen werden gemessen. In 20-minütigen Intervallen werden die Ringe abwechselnd mit 10 nM Angiotensin-II (Hypertensin-CIBA) und 5 nM Noradrenalinchlorid stimuliert. Anschliessend werden die Ringe mit ausgewählten Konzentrationen der Testsubstanzen vor der Behandlung mit den Agonisten inkubiert. Die Daten werden mit einem Buxco Digitalcomputer analysiert. Die Konzentrationen, die eine 50%-ige Hemmung der Anfangskontrollwerte bewirken, werden als $IC_{50}$-Werte angegeben. Für die erfindungsgemässen Verbindungen wurden $IC_{50}$-Werte ab etwa 5 nM bestimmt.

Dass die erfindungsgemässen Verbindungen durch Angiotensin-II induzierten Bluthochdruck reduzieren können, kann im Testmodell an der normotensiven, narkotisierten Ratte verifiziert werden. Nach Kalibration der Präparationen mit jeweils 0,9 % NaCl (1 ml/kg i.v.), Noradrenalin (1 µg/kg i.v.) bzw. Angiotensin-II (0,3 µg/kg i.v.) werden steigende Dosen (3-6) der Testsubstanz durch Bolusinjektion intravenös injiziert, worauf nach jeder Dosis in 5 Minutenintervallen Angiotensin-II bzw. Noradrenalin appliziert werden. Der Blutdruck wird direkt in der Halsschlagader gemessen und mit einem on-line Datenerfassungssystem aufgezeichnet (Buxco). Die Spezifität des Angiotensin-11-Antagonismus wird angezeigt durch die selektive Hemmung des von Angiotensin-II, nicht aber des durch Noradrenalin hervorgerufenen Druckeffektes. Bei diesen Untersuchungen zeigten die erfindungsgemässen Verbindungen ab einer Dosis von etwa 0,3 mg/kg i.v. einen hemmenden Effekt.

Auch an der renalen hypertensiven Ratte kann die antihypertensive Aktivität der erfindungsgemässen Verbindungen manifestiert werden. Bei männlichen Ratten wird durch Verengung einer renalen Arterie gemäss der Goldblatt Methode Bluthochdruck erzeugt. Den Ratten werden mittels einer Magensonde Dosen der Substanz verabreicht. Kontrolltiere erhalten ein äquivalentes Volumen an Lösungsmittel. Blutdruck und Herzschlag werden indirekt bei wachen Tieren nach der Schwanzklemm-Methode von Gerold et al. (Helv. Physiol. Acta 24 , 58, 1966) vor Verabreichung der Substanzen bzw. des Placebos sowie während des Verlaufs der Experimente in Intervallen gemessen. Der ausgeprägte antihypertensive Effekt konnte unterhalb einer Dosierung von etwa 30 mg/kg p.o. nachgewiesen werden.

Dementsprechend können die Verbindungen der Formel I z.B. als Arzneimittelwirkstoffe verwendet werden, wie als Antihypertensiva, z.B. zur Behandlung von Bluthochdruck sowie von Herzinsuffizienz. Ein weiterer Erfindungsgegenstand ist die Verwendung der erfindungsgemässen Verbindungen zur Herstellung

von Arzneimitteln, insbesondere Angiotensin-II-Antagonisten und Antihypertensiva, und die Verwendung dieser Verbindungen zur therapeutischen Behandlung von Bluthochdruck sowie von Herzinsuffizienz. Bei der Herstellung der Arzneimittel ist auch die gewerbsmässige Herrichtung der Wirksubstanzen eingeschlossen.

Die Erfindung betrifft insbesondere Verbindungen der Formel I, ihre Tautomere und Salze, worin Z für O, S oder N(R) und R für Wasserstoff oder einen aliphatischen Kohlenwasserstoffrest steht, $R_1$ einen gegebenenfalls durch Halogen oder Hydroxy substituierten aliphatischen Rest, einen cycloaliphatischen oder araliphatischen Kohlenwasserstoffrest oder einen aromatischen Rest bedeutet, $R_2$ und $R_3$ unabhängig voneinander Halogen, Acyl, einen aromatischen Rest, gegebenenfalls verestertes oder amidiertes Carboxy bedeuten, oder $R_2$ für $-Z_1-R'_2$ und $R_3$ für $-Z_2-R'_3$ stehen, wobei $Z_1$ und $Z_2$ unabhängig voneinander für eine Bindung stehen oder O, $S(O)_n$ oder NH bedeuten, n für 0, 1 oder 2 steht und $R'_2$ und $R'_3$ unabhängig voneinander für Wasserstoff oder einen aliphatischen Kohlenwasserstoffrest stehen, der gegebenenfalls durch Halogen, Hydroxy, gegebenenfalls substituiertes Amino, gegebenenfalls verestertes oder amidiertes Carboxy substituiert ist und der gegebenenfalls durch -O- oder $-S(O)_n-$ unterbrochen ist, wobei n für 0, 1 oder 2 steht, $R_4$ für eine Gruppe der Formel

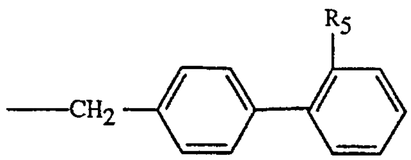

(Ib)

steht, in der $R_5$ COOH, $SO_3H$, Halogenalkansulfamoyl, $PO_2H_2$, $PO_3H_2$ oder 5-Tetrazolyl bedeutet.

Die Erfindung betrifft insbesondere Verbindungen der Formel I, ihre Tautomere und Salze, worin Z für O, S oder N(R) steht und R Wasserstoff, Niederalkyl, Niederalkenyl oder Niederalkinyl bedeutet; $R_1$ Niederalkyl, Niederalkenyl bzw. Niederalkinyl bedeutet, welche jeweils gegebenenfalls substituiert sind durch Substituenten ausgewählt aus Halogen, Hydroxy, Niederalkoxy, Niederalkenyloxy, Phenylniederalkoxy, Phenoxy, Mercapto, Niederalkylthio, Niederalkansulfinyl, -sulfonyl, Niederalkenylthio, Niederalkenylsulfinyl, -sulfonyl, Niederalkinylthio, Niederalkinylsulfinyl, -sulfonyl, Amino, welches gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, Carboxy, Niederalkoxy-, Niederalkoxyniederalkoxy-oder Niederalkenyloxy-carbonyl und Carbamoyl, in welchem die Aminogruppe gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, oder jeweils 3- bis 7-gliedriges Cycloalkyl bzw. Cycloalkenyl, Phenylniederalkyl, Phenylniederalkenyl, Phenylniederalkinyl, Phenyl, Naphthyl, Pyrrolyl, Pyrazolyl, Imidazolyl, Triazolyl, Tetrazolyl, Furyl, Thienyl oder Pyridyl bedeutet; $R_2$ und $R_3$ unabhängig voneinander Halogen, Niederalkanoyl, Phenylniederalkanoyl, Benzoyl, Phenyl, Naphthyl, Pyrrolyl, Pyrazolyl, Imidazolyl, Triazolyl, Tetrazolyl, Furyl, Thienyl, Pyridyl, Amino, welches gegebenenfalls durch Niederalkyl, Niederalkenyl, Niederalkinyl, Phenylniederalkyl, Phenylniederalkinyl unabhängig voneinander mono-oder disubstituiert oder durch Niederalkylen oder Niederalkylenoxyniederalkylen disubstituiert ist, Carboxy, Niederalkoxy-, Niederalkoxyniederalkoxy- oder Niederalkenyloxy-carbonyl, Carbamoyl, in welchem die Aminogruppe gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, bedeuten; oder $R_2$ für $-Z_1-R_2'$ und $R_3$ für $-Z_2-R_3'$ stehen, wobei $Z_1$ und $Z_2$ unabhängig voneinander für eine Bindung, O oder S(O)n stehen und n für 0, 1 oder 2 steht, und $R_2'$ und $R_3'$ unabhängig voneinander Wasserstoff, Phenylniederalkyl, Phenylniederalkenyl oder Phenylniederalkinyl bedeuten oder für Niederalkyl, Niederalkenyl, Niederalkinyl, Niederalkoxyniederalkyl, -niederalkenyl oder -niederalkinyl, Niederalkenyloxyniederalkyl, -niederalkenyl oder -niederalkinyl, Niederalkylthioniederalkyl, -niederalkenyl oder -niederalkinyl, Niederalkansulfinylniederalkyl, -sulfonylniederalkyl, Niederalkenylthioniederalkyl, -sulfinylniederalkyl, -sulfonylniederalkyl, Niederalkinylthioniederalkyl, -sulfinylniederalkyl, -sulfonylniederalkyl stehen, welche jeweils unabhängig voneinander gegebenenfalls substituiert sind durch Substituenten ausgewählt aus Halogen, Hydroxy, Niederalkoxy, Niederalkenyloxy, Phenylniederalkoxy, Phenoxy, Mercapto, Niederalkylthio, Niederalkansulfinyl, -sulfonyl, Niederalkenylthio, Niederalkenylsulfinyl, -sulfonyl, Niederalkinylthio, Niederalkinylsulfinyl, -sulfonyl,

Amino, welches gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, Carboxy, Niederalkoxy-, Niederalkoxyniederalkoxy- oder Niederalkenyloxy-carbonyl, Carbamoyl, in welchem die Aminogruppe gegebenenfalls durch Niederalkyl, Niederalkenyl, Niederalkinyl, Phenylniederalkyl, Phenylniederalkinyl unabhängig voneinander mono-oder disubstituiert oder durch Niederalkylen oder Niederalkylenoxyniederalkylen disubstituiert ist;

oder $R_2$ und $R_3$ gemeinsam für Propylen oder Butylen oder für die Partialstruktur der Formel -CH=CH-CH=CH- stehen, in der gegebenenfalls eine oder zwei der Methingruppen (-CH=) durch -N= ersetzt ist;

$R_4$ für eine Gruppe der Formel (Ia), insbesondere (Ib), steht, in der Alk Niederalkylen oder Niederalkyliden, insbesondere Methylen, bedeutet; $R_5$ COOH, $SO_3H$, Halogenniederalkansulfamoyl, $PO_2H_2$, $PO_3H_2$ oder 5-Tetrazolyl bedeutet;

die Ringe A und B sowie die (hetero-)aromatischen Reste bzw. der durch $R_2$ und $R_3$ gemeinsam gebildete (hetero-)aromatische Ring jeweils unabhängig voneinander gegebenenfalls substituiert sind durch Substituenten ausgewählt aus Halogen, Hydroxy, Niederalkoxy, Niederalkenyloxy, Phenylniederalkoxy, Phenoxy, Mercapto, Niederalkylthio, Niederalkansulfinyl, -sulfonyl, Niederalkenylthio, Niederalkenylsulfinyl, -sulfonyl, Niederalkinylthio, Niederalkinylsulfinyl, -sulfonyl, oder aus Niederalkyl, Niederalkenyl, Niederalkinyl, Niederalkoxyniederalkyl, -niederalkenyl, -niederalkinyl, Niederalkenyloxyniederalkyl, -niederalkenyl bzw. -niederalkinyl, welche jeweils gegebenenfalls substituiert sind durch Substituenten ausgewählt aus Halogen, Hydroxy, Niederalkoxy, Niederalkenyloxy, Phenylniederalkoxy, Phenoxy, Mercapto, Niederalkylthio, Niederalkansulfinyl, -sulfonyl, Niederalkenylthio, Niederalkenylsulfinyl, -sulfonyl, Niederalkinylthio, Niederalkinylsulfinyl, -sulfonyl, Amino, welches gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, Carboxy, Niederalkoxy-, Niederalkoxyniederalkoxy- oder Niederalkenyloxy-carbonyl, Carbamoyl, in welchem die Aminogruppe gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist.

Die Erfindung betrifft insbesondere Verbindungen der Formel I, ihre Tautomere und Salze, worin Z für O, S oder N(R) und R für Wasserstoff, Niederalkyl, Niederalkenyl oder Niederalkinyl steht,

$R_1$ gegebenenfalls durch Halogen oder Hydroxy substituiertes Niederalkyl, Niederalkenyl bzw. Niederalkinyl, jeweils 3- bis 7-gliedriges Cycloalkyl bzw. Cycloalkenyl, Phenylniederalkyl, Phenylniederalkenyl bzw. Phenylniederalkinyl oder Phenyl bedeutet, $R_2$ und $R_3$ unabhängig voneinander Halogen, Niederalkanoyl, Phenyl, Carboxy, welches gegebenenfalls durch einen Alkohol verestert ist, welcher sich von Niederalkyl, Niederalkenyl, Niederalkinyl, Niederalkoxyniederalkyl, -niederalkenyl, -niederalkinyl, Niederalkenyloxyniederalkyl, -niederalkenyl, -niederalkinyl ableitet, Carbamoyl, in welchem die Aminogruppe gegebenenfalls durch Niederalkyl, Niederalkenyl, Niederalkinyl, Phenylniederalkyl, -niederalkenyl oder -niederalkinyl unabhängig voneinander mono- oder disubstituiert oder durch Niederalkylen oder Niederalkylenoxyniederalkylen disubstituiert ist, oder $R_2$ für $-Z_1-R'_2$ und $R_3$ für $-Z_2-R'_3$ stehen, wobei $Z_1$ und $Z_2$ unabhängig voneinander für eine Bindung stehen oder O, $S(O)_n$ oder NH bedeuten, n für 0, 1 oder 2 steht, und $R'_2$ und $R'_3$ unabhängig voneinander Wasserstoff, gegebenenfalls durch Halogen, Hydroxy, Amino, welches gegebenenfalls wie vorstehend angegeben substituiert ist, Carboxy, welches gegebenenfalls wie vorstehend angegeben verestert ist, Carbamoyl, welches gegebenenfalls wie vorstehend angegeben substituiert ist, substituiertes Niederalkyl, Niederalkenyl bzw. Niederalkinyl, Niederalkoxyniederalkyl, -niederalkenyl, -niederalkinyl, Niederalkenyloxyniederalkyl, -niederalkenyl, -niederalkinyl, Niederalkylthioniederalkyl, -niederalkenyl, -niederalkinyl, Niederalkansulfinylniederalkyl, -sulfonylniederalkyl, Niederalkenylthioniederalkyl, -sulfinylniederalkyl, -sulfonylniederalkyl, Niederalkinylthioniederalkyl, -sulfinylniederalkyl bzw. -sulfonylniederalkyl bedeuten, $R_4$ für die Gruppe der Formel Ib steht, in der $R_5$ COOH, $SO_3H$, Halogenniederalkansulfamoyl, $PO_2H_2$, $PO_3H_2$ oder 5-Tetrazolyl bedeutet, wobei Phenyl bzw. Phenyl in Phenyl aufweisenden Resten jeweils unsubstituiert oder durch Substituenten ausgewählt aus der Gruppe bestehend aus Niederalkyl, Niederalkoxy, Halogen, Trifluormethyl und Hydroxy substituiert ist.

Die Erfindung betrifft insbesondere Verbindungen der Formel I, ihre Tautomere und Salze, worin Z für O, S oder N(R) steht und R Wasserstoff oder Niederalkyl bedeutet, $R_1$ jeweils gegebenenfalls durch Halogen oder Hydroxy substituiertes Niederalkyl bzw. Niederalkenyl bedeutet oder $C_3$-$C_7$-Cycloalkyl, $C_3$-$C_7$-Cycloalkenyl, Phenylniederalkyl, Phenyl oder Pyridyl bedeutet;

$R_2$ und $R_3$ unabhängig voneinander Halogen, Niederalkanoyl, Phenylniederalkanoyl, Benzoyl, Phenyl, Tetrazolyl, Pyridyl, Amino, welches gegebenenfalls durch Niederalkyl oder Phenylniederalkyl mono- oder disubstituiert oder durch Niederalkylen oder Niederalkylenoxyniederalkylen disubstuiert ist, Carboxy, Niederalkoxy-, Niederalkoxyniederalkoxy- oder Niederalkenyloxy-carbonyl, Carbamoyl, in welchem die Aminogruppe gegebenenfalls durch Niederalkyl oder Phenylniederalkyl unabhängig voneinander mono- oder

disubstituiert oder durch Niederalkylen oder Niederalkylenoxyniederalkylen disubstituiert ist, bedeuten; oder $R_2$ für $-Z_1-R_2'$ und $R_3$ für $-Z_2-R_3'$ stehen, wobei $Z_1$ und $Z_2$ unabhängig voneinander für eine Bindung, O oder $S(O)_n$ stehen und n für 0, 1 oder 2 steht; und $R_2'$ und $R_3'$ unabhängig voneinander Wasserstoff oder Phenylniederalkyl bedeuten oder für Niederalkyl, Niederalkenyl, Niederalkoxyniederalkyl, Niederalkylthioniederalkyl, Niederalkansulfinylniederalkyl oder -sulfonylniederalkyl stehen, welche jeweils unabhängig voneinander gegebenenfalls substituiert sind durch Substituenten ausgewählt aus Halogen, Hydroxy, Niederalkoxy, Phenylniederalkoxy, Phenoxy, Amino, welches gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, Carboxy, Niederalkoxy-, Niederalkoxyniederalkoxy-carbonyl, Carbamoyl, in welchem die Aminogruppe gegebenenfalls durch Niederalkyl oder Phenylniederalkyl unabhängig voneinander mono- oder disubstituiert oder durch Niederalkylen oder Niederalkylenoxyniederalkylen disubstituiert ist; oder $R_2$ und $R_3$ gemeinsam für Propylen oder Butylen oder für die Partialstruktur der Formel -CH=CH-CH=CH- stehen, in der gegebenenfalls eine oder zwei Methingruppe(n) durch =N- ersetzt ist;

$R_4$ für eine Gruppe der Formel Ia, in erster Linie Ib, steht, in der Alk Niederalkylen oder Niederalkyliden, in erster Linie Methylen, bedeutet;

$R_5$ Carboxy, Halogenniederalkansulfamoyl oder 5-Tetrazolyl bedeutet;

die Ringe A und B sowie die (hetero-)aromatischen Reste bzw. der durch $R_2$ und $R_3$ gemeinsam gebildete (hetero-)aromatische Ring jeweils unabhängig voneinander unsubstituiert oder substituiert sind durch Substituenten ausgewählt aus Halogen, Hydroxy, Niederalkoxy, Phenylniederalkoxy, Mercapto, Niederalkylthio, Niederalkansulfinyl, -sulfonyl, gegebenenfalls durch Halogen, Hydroxy, Niederalkoxy, Phenylniederalkoxy, Phenoxy substituiertes Niederalkyl bzw. Niederalkoxyniederalkoxyniederalkyl.

Die Erfindung betrifft insbesondere Verbindungen der Formel I, ihre Tautomere und Salze, worin Z für O, S oder N(R) und R für Wasserstoff oder Niederalkyl, steht, $R_1$ Niederalkyl, $C_3$-$C_7$-Cycloalkyl, Phenylniederalkyl oder Phenyl bedeutet, $R_2$ und $R_3$ unabhängig voneinander Halogen, Phenyl, Niederalkanoyl, Carboxy, Niederalkoxy-, Niederalkoxyniederalkoxy-carbonyl, Carbamoyl, Niederalkyl-, Diniederalkyl-carbamoyl, Niederalkylen-carbamoyl, Niederalkylenoxyniederalkylen-carbamoyl, bedeutet, oder $R_2$ für $-Z_1-R'_2$ und $R_3$ für $-Z-R'_3$ stehen, wobei $Z_1$ und $Z_2$ unabhängig voneinander für eine Bindung stehen oder O, S oder NH bedeuten und $R'_2$ und $R'_3$ unabhängig voneinander Wasserstoff, Niederalkyl, Halogenniederalkyl, Hydroxyniederalkyl, Niederalkoxyniederalkyl, Aminoniederalkyl, Niederalkylamino-niederalkyl, Diniederalkylaminoniederalkyl, Niederalkylenamino-niederalkyl, Niederalkylenoxyniederalkylenaminoniederalkyl, Carboxyniederalkyl, Niederalkoxy-, Niederalkoxyniederalkoxy-carbonylniederalkyl, bedeuten, $R_4$ für die Gruppe der Formel Ib steht, in der $R_5$ COOH oder 5-Tetrazolyl bedeutet, wobei Phenyl bzw. Phenyl in Phenyl aufweisenden Resten jeweils unsubstituiert oder durch Niederalkyl, Niederalkoxy, Halogen, Trifluormethyl, und/oder Hydroxy substituiert ist.

Die Erfindung betrifft in erster Linie Verbindungen der Formel I, ihre Tautomere und Salze, worin Z für O steht, $R_1$ Niederalkyl, insbesondere mit 3 bis und mit 5 C-Atomen, wie n-Propyl oder n-Butyl, oder Niederalkenyl, insbesondere mit 3 bis und mit 5 C-Atomen, wie 2-Propenyl, bedeutet, $R_2$ und $R_3$ unabhängig voneinander Halogen, insbesondere mit Atomnummer bis und mit 35, wie Chlor, Tetrazolyl, wie 5-Tetrazolyl, Amino, welches gegebenenfalls durch Niederalkyl oder Phenylniederalkyl, insbesondere mit bis und mit 4 C-Atomen je Alkylteil, mono- oder disubstituiert oder durch Niederalkylenoxyniederalkylen, insbesondere mit bis und mit 4 C-Atomen je Niederalkylenteil, wie 4-Morpholinyl, disubstituiert ist, Carboxy, Niederalkoxycarbonyl, insbesondere mit 2 bis und mit 5 C-Atomen, wie Methoxy- oder Ethoxycarbonyl, Niederalkoxyniederalkoxycarbonyl, insbesondere mit bis und mit 4 C-Atomen im Alkoxyteil, wie 2-Methoxyethoxy carbonyl, bedeuten; oder $R_2$ für $-Z_1-R_2'$ und $R_3$ für $-Z_2-R_3'$ stehen, wobei $Z_1$ und $Z_2$ unabhängig voneinander für eine Bindung, O oder $S(O)_n$ stehen und n für 0, 1 oder 2 steht, und $R_2'$ und $R_3'$ unabhängig voneinander Wasserstoff oder Phenylniederalkyl, insbesondere mit bis und mit 4 C-Atomen im Alkylteil, wie Benzyl, bedeuten oder Niederalkyl oder Niederalkoxyniederalkyl, insbesondere jeweils mit bis und mit 4 C-Atomen im Alkylteil, bedeuten, welche jeweils gegebenenfalls durch Halogen, insbesondere mit Atomnummer bis und 35, wie Chlor, Hydroxy, Amino, welches gegebenenfalls durch Niederalkyl oder Phenylniederalkyl, insbesondere mit bis und mit 4 C-Atomen je Alkylteil, mono- oder disubstituiert oder durch Niederalkylenoxyniederalkyen, insbesondere mit bis und mit 4 C-Atomen je Niederalkyteil disubstituiert. ist, wie Methyl-, Dimethyl-, Benzyl- oder Dibenzylamino oder 4-Morpholinyl, Carboxy, Niederalkoxycarbonyl, insbesondere mit 2 bis und mit 5 C-Atomen, wie Methoxy- oder Ethoxycarbonyl, substituert sind; oder $R_2$ und $R_3$ gemeinsam für Propylen oder Butylen oder für die Partialstruktur der Formel -CH=CH-CH=CH- stehen, in der gegebenenfalls eine oder zwei Methingruppe(n) durch =N- ersetzt ist;

$R_4$ für eine Gruppe der Formel Ia, insbesondere Ib, steht, in der Alk Niederalkylen oder Niederalkyliden, insbesondere jeweils mit bis und mit 4 C-Atomen, wie Methylen, Ethylen oder Ethyliden, in erster Linie

Methylen, bedeutet;

$R_5$ Carboxy, Halogenniederalkansulfamoyl, insbesondere mit Atomnummer bis und mit 35 und bis und mit 4 C-Atomen, insbesondere Trifluormethansulfamoyl, oder 5-Tetrazolyl bedeutet;

die Ringe A und B sowie die aromatischen Reste bzw. der durch $R_2$ und $R_3$ gemeinsam gebildete (hetero-)aromatische Ring jeweils unabhängig voneinander unsubstituiert oder durch Hydroxy, Halogen, insbesondere mit Atomnummer bis und mit 35, wie Chlor, Trifluormethyl, Niederalkyl, insbesondere mit bis und mit 4 C-Atomen, wie Methyl, und/oder Niederalkoxy, insbesondere mit bis und mit 4 C-Atomen, wie Methoxy, substituiert sind.

Die Erfindung betrifft in erster Linie Verbindungen der Formel I, ihre Tautomere und Salze, worin Z für O steht, $R_1$ Niederalkyl, insbesondere mit 3 bis und mit 5 C-Atomen, wie n-Butyl, bedeutet, einer der Reste $R_2$ und $R_3$ Halogen, insbesondere mit Atomnummer bis und mit 35, wie Chlor, Phenyl, Carboxy, Niederalkoxycarbonyl, insbesondere mit 2 bis und mit 5 C-Atomen, wie Ethoxycarbonyl, Wasserstoff, Hydroxy, Niederalkylthio, insbesondere mit bis und mit 4 C-Atomen, wie Methylthio, Niederalkansulfonyl, insbesondere mit bis und mit 4 C-Atomen, wie Methansulfonyl, Amino, Diniederalkylamino, insbesondere mit bis und mit 4 C-Atomen je Niederalkylteil, wie Dimethylamino, Morpholino, Niederalkyl, insbesondere mit bis und mit 4 C-Atomen, wie Methyl, Hydro xyniederalkyl, insbesondere mit bis und mit 4° C-Atomen, wie Hydroxymethyl, Halogenniederalkyl, insbesondere mit Atomnummer bis und mit 35 und mit bis und mit 4 C-Atomen, wie Trifluormethyl, Diniederalkylaminoniederalkyl, insbesondere mit bis und mit 4 C-Atomen je Niederalkylteil, wie 2-Dimethylaminoethyl, Carboxyniederalkyl, insbesondere mit bis und mit 5 C-Atomen, wie Carboxymethyl, Niederalkoxycarbonylniederalkyl, insbesondere mit bis und mit 4 C-Atomen je Niederalkoxy- bzw. Niederalkylteil, wie Ethoxycarbonylmethyl, Niederalkoxy, insbesondere mit bis und mit 4 C-Atomen, wie Methoxy, Hydroxyniederalkoxy, insbesondere mit bis und mit 4 C-Atomen, wie 2-Hydroxyethoxy, Niederalkoxyniederalkoxy, insbesondere mit bis und mit 4 C-Atomen je Niederalkoxyteil, wie 2-Methoxyethoxy, Diniederalkylaminoniederalkoxy, insbesondere mit bis und mit 4 C-Atomen je Niederalkyl- bzw. Niederalkoxyteil, wie 2-Dimethylaminoethyl, Carboxyniederalkoxy, insbesondere mit bis und mit 5 C-Atomen, wie 2-Carboxyethoxy, oder Niederalkoxycarbonylniederalkoxy, insbesondere mit bis und mit 4 C-Atomen je Niederalkoxyteil, wie 2-Ethoxycarbonylethoxy, bedeutet und der andere Wasserstoff oder Niederalkyl, insbesondere mit bis und mit 4 C-Atomen, wie Methyl, bedeutet, und $R_4$ für die Gruppe der Formel Ib steht, in der $R_5$ 5-Tetrazolyl bedeutet.

Die Erfindung betrifft in erster Linie Verbindungen der Formel I, ihre Tautomere und Salze, worin Z für O steht, $R_1$ $C_3$-$C_5$-Alkyl, wie n-Propyl oder n-Butyl, bedeutet, $R_2$ und $R_3$ unabhängig voneinander Halogen mit Atomnummer bis und mit 35, wie Chlor, Di-$C_1$-$C_4$-alkylamino, wie Dimethylamino, 4-Morpholinyl, Carboxy, $C_2$-$C_5$-Alkoxycarbonyl, wie Methoxy- oder Ethoxycarbonyl, bedeuten; oder $R_2$ für -$Z_1$-$R_2'$ und $R_3$ für -$Z_2$-$R_3'$ stehen, wobei $Z_1$ und $Z_2$ unabhängig voneinander für eine Bindung, O oder $S(O)_n$ stehen und n für 0, 1 oder 2 steht, und $R_2'$ und $R_3'$ unabhängig voneinander Wasserstoff, Phenyl-$C_2$-$C_5$-alkyl, wie Benzyl, oder $C_1$-$C_4$-Alkyl, wie Methyl, welches gegebenenfalls durch Hydroxy oder Di-$C_1$-$C_4$-alkylamino, wie Dimethylamino, substituiert ist, oder $C_1$-$C_4$-Alkoxy-$C_1$-$C_4$-alkoxy, wie 2-Methoxyethoxy, bedeuten; oder $R_2$ und $R_3$ gemeinsam für Propylen oder Butylen oder für die Partialstruktur der Formel -CH=CH-CH=CH- stehen, in der gegebenenfalls eine oder zwei Methingruppe(n) durch =N- ersetzt ist;

$R_4$ für eine Gruppe der Formel Ib steht, in der $R_5$ Carboxy oder in erster Linie 5-Tetrazolyl bedeutet; und die Ringe A und B bzw. der durch $R_2$ und $R_3$ gemeinsam gebildete (hetero-)aromatische Ring in erster Linie unsubstituiert, ferner durch Hydroxy, Halogen, insbesondere mit Atomnummer bis und mit 35, wie Chlor, Trifluormethyl, Niederalkyl, insbesondere mit bis und mit 4 C-Atomen, wie Methyl, und/oder Niederalkoxy, insbesondere mit bis und mit 4 C-Atomen, wie Methoxy, substituiert sind.

Die Erfindung betrifft in erster Linie Verbindungen der Formel I, ihre Tautomere und Salze, worin Z für O steht, $R_1$ $C_3$-$C_5$-Alkyl, wie n-Propyl oder n-Butyl, bedeutet, $R_2$ Halogen mit Atomnummer bis und mit 35, wie Chlor, Trifluormethyl, Carboxy, $C_2$-$C_5$Alkoxycarbonyl, wie Ethoxycarbonyl, Wasserstoff, Hydroxy, $C_1$-$C_4$-Alkyl, wie Methyl oder n-Butyl, $C_1$-$C_4$-Alkoxy, wie Methoxy, Phenyl-$C_1$-$C_4$-alkoxy, wie Benzyloxy, Hydroxy-$C_1$-$C_4$-alkoxy, wie 2-Hydroxyethoxy, $C_1$-$C_4$-Alkoxy-$C_1$-$C_4$-alkoxy, wie 2-Methoxyethoxy, Di-$C_1$-$C_4$-alkylamino-$C_1$-$C_4$-alkoxy, wie 2-Dimethylaminoethoxy, bedeutet; $R_3$ Wasserstoff, $C_1$-$C_4$-Alkyl, wie Methyl oder n-Butyl, Carboxy, $C_2$-$C_5$-Alkoxycarbonyl, wie Ethoxycarbonyl, oder Hydroxy-$C_1$-$C_4$-alkyl, wie Hydroxymethyl, bedeutet; oder $R_2$ und $R_3$ gemeinsam für Propylen oder Butylen oder für die Partialstruktur der Formel -CH=CH-CH=CH- stehen, in der gegebenenfalls eine Methingruppe durch =N-ersetzt ist;

$R_4$ für eine Gruppe der Formel Ib steht, in der $R_5$ Carboxy oder in erster Linie 5-Tetrazolyl bedeutet; und die Ringe A und B in erster Linie unsubstituiert, ferner durch Hydroxy, Halogen mit Atomnummer bis und mit 35, wie Chlor, Trifluormethyl, $C_1$-$C_4$-Alkyl, wie Methyl, oder $C_1$-$C_4$-Alkoxy, wie Methoxy, substituiert sind.

Die Erfindung betrifft in erster Linie Verbindungen der Formel I, ihre Tautomere und Salze, worin Z für

O steht, $R_1$ $C_3$-$C_5$-Alkyl, wie n-Propyl oder n-Butyl, bedeutet, $R_2$ $C_1$-$C_5$-Alkyl, wie Methyl, n-Propyl oder n-Butyl, oder Hydroxy bedeutet, $R_3$ Wasserstoff oder $C_1$-$C_5$-Alkyl, wie n-Propyl oder n-Butyl, bedeutet; oder $R_2$ und $R_3$ gemeinsam für Propylen oder Butylen oder für die Partialstruktur der Formel -CH = CH-CH = CH-stehen, in der gegebenenfalls eine Methingruppe durch = N- ersetzt ist;
$R_4$ für eine Gruppe der Formel Ib steht, in der $R_5$ Carboxy oder in erster Linie 5-Tetrazolyl bedeutet; und die Ringe A und B in erster Linie unsubstituiert oder ferner durch Hydroxy, Halogen mit Atomnummer bis und mit 35, wie Chlor, Trifluormethyl, $C_1$-$C_4$-Alkyl, wie Methyl, oder $C_1$-$C_4$-Alkoxy, wie Methoxy, substituiert sind.

Die Erfindung betrifft in erster Linie Verbindungen der Formel I und ihre Salze, worin Z für O steht, $R_1$ $C_3$-$C_5$-Alkyl, wie n-Propyl oder n-Butyl, bedeutet, $R_2$ und $R_3$ gemeinsam für Propylen oder Butylen oder für die Partialstruktur der Formel -CH = CH-CH = CH-stehen, in der gegebenenfalls eine Methingruppe durch = N- ersetzt ist;
$R_4$ für eine Gruppe der Formel Ib steht, in der $R_5$ 5-Tetrazolyl bedeutet; und die Ringe A und B unsubstituiert sind.

Die Erfindung betrifft in erster Linie Verbindungen der Formel I und ihre Salze, worin Z für O steht, $R_1$ $C_3$-$C_5$-Alkyl, wie n-Butyl, bedeutet, $R_2$ Wasserstoff, Halogen mit Atomnummer bis und mit 35, wie Chlor, $C_1$-$C_4$-Alkyl, wie Methyl, $C_1$-$C_4$-Alkoxy, wie Methoxy, bedeutet und $R_3$ Wasserstoff bedeutet, und $R_4$ für die Gruppe der Formel Ib steht, in der $R_5$ 5-Tetrazolyl bedeutet.

Die Erfindung betrifft in erster Linie Verbindungen der Formel I und ihre Salze, worin Z für O steht, $R_1$ $C_3$-$C_5$-Alkyl, wie n-Butyl, bedeutet, $R_2$ Wasserstoff, Halogen mit Atomnummer bis und mit 35, wie Chlor, oder $C_1$-$C_4$-Alkyl, wie Methyl, bedeutet, $R_3$ Wasserstoff ist, $R_4$ für die Gruppe der Formel Ib steht, in der $R_5$ 5-Tetrazolyl bedeutet.

Die Erfindung betrifft in erster Linie Verbindungen der Formel I und ihre Salze, worin Z für O steht, $R_1$ $C_3$-$C_5$-Alkyl, wie n-Butyl, bedeutet, $R_2$ $C_1$-$C_4$-Alkyl, wie Methyl oder n-Butyl, und $R_3$ $C_1$-$C_4$-Alkyl, wie n-Butyl, bedeuten, oder $R_2$ Hydroxy und $R_3$ Wasserstoff oder Methyl bedeuten, $R_4$ für die Gruppe der Formel Ib steht, in der $R_5$ 5-Tetrazolyl bedeutet.

Die Erfindung betrifft insbesondere die in den Beispielen genannten neuen Verbindungen sowie die dort beschriebenen Herstellungsweisen.

Ein weiterer Gegenstand der Erfindung sind Verfahren zur Herstellung der erfindungsgemässen Verbindungen. Die Herstellung von Verbindungen der Formel I erfolgt in an sich bekannter Weise und ist z.B. dadurch gekennzeichnet, dass man

a) eine Verbindung der Formel

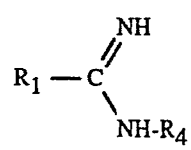

(IIa)

oder ein Salz davon mit einer Verbindung der Formel

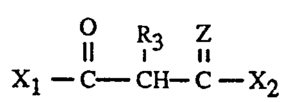

(IIb)

einem Salz, einem Tautomeren oder einem funktionell abgewandelten Derivat des Tautomeren davon umsetzt, worin $X_1$ für die Variable $R_2$ oder für verethertes Hydroxy steht und $X_2$ verethertes Hydroxy bedeutet, oder

b) in einer Verbindung der Formel

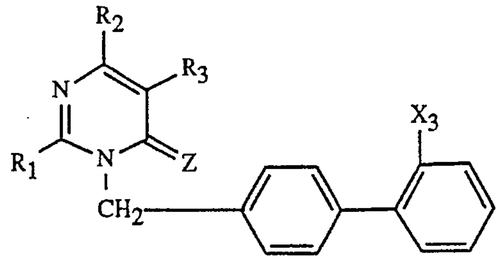

(III)

oder einem Salz davon, worin $X_3$ einen in die Variable $R_5$ überführbaren Rest bedeutet, $X_3$ in die Variable $R_5$ überführt, oder

c) eine Verbindung der Formel

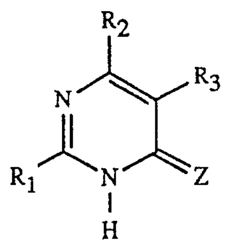

(IVa),

ein Tautomeres oder Salz davon mit einer Verbindung der Formel

$X_4$-$R_4$ (IVb)

oder einem Salz davon, worin $X_4$ für reaktionsfähiges verestertes Hydroxy steht, umsetzt, und, wenn erwünscht, eine verfahrensgemäss oder auf andere Weise erhältliche Verbindung der Formel I oder ein Salz davon in eine andere erfindungsgemässe Verbindung oder ein Salz davon überführt, eine verfahrensgemäss erhältliche freie Verbindung der Formel I in ein Salz überführt, ein verfahrensgemäss erhältliches Salz in die freie Verbindung der Formel I oder in ein anderes Salz überführt, ein verfahrensgemäss erhältliches Gemisch von Isomeren auftrennt und die gewünschte Verbindung isoliert.

Salze von Ausgangsmaterialien, die mindestens ein basisches Zentrum aufweisen, beispielsweise der Formel IIa, sind entsprechende Säureadditionssalze, während Salze von Ausgangsstoffen, die eine acide Gruppe aufweisen, als Salze mit Basen vorliegen, jeweils wie in Zusammenhang mit entsprechenden Salzen der Formel I vorstehend aufgeführt.

Als Tautomeres einer Verbindung der Formel IIb kommt insbesondere eine Verbindung der Formel

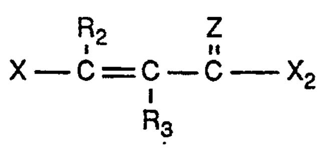

(IIb')

in Frage, worin X Hydroxy bedeutet. Die enolische Hydroxygruppe ist vorteilhaft funktionell abgewandelt; X bedeutet dementsprechend beispielsweise reaktionsfähiges verestertes Hydroxy, wie Halogen, verethertes Hydroxy, wie Niederalkoxy, gegebenenfalls verethertes Mercapto, wie Niederalkylthio, gegebenenfalls substituiertes Amino, wie Niederalkyl- oder Diniederalkylamino.

Verethertes Hydroxy ($X_1$ bzw. $X_2$) bedeutet insbesondere Alkoxy, wie Niederalkoxy, z.B. Methoxy oder Ethoxy.

In die Variable $R_5$ überführbare Reste $X_3$ stellen beispielsweise Cyano, Mercapto, Halogen, die Gruppe $-N_2^+ A^-$, in der $A^-$ ein von einer Säure abgeleitetes Anion bedeutet, Amino sowie von COOH, $SO_3H$, $PO_3H_2$ oder $PO_2H_2$ verschiedene funktionell abgewandelte Formen sowie N-geschütztes 5-Tetrazolyl.

Reaktionsfähiges verestertes Hydroxy, z.B. $X_4$, ist insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes ($C_1$-$C_7$-)Alkansulfonyloxy, z.B. Methan- oder Trifluormethansulfonyloxy, ($C_5$-$C_7$-)Cycloalkansulfonyloxy, z.B. Cyclohexansulfonyloxy, oder gegebenenfalls, z.B. durch ($C_1$-$C_7$-)Alkyl oder

Halogen, substituiertes Benzolsulfonyloxy, z.B. p-Brombenzol-oder p-Toluolsulfonyloxy.

Die vor- und nachstehend in den Varianten beschriebenen Umsetzungen werden in an sich bekannter Weise durchgeführt, z.B. in Ab- oder üblicher Weise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -80° C bis zur Siedetemperatur des Reaktionsmediums, vorzugsweise von etwa -10° bis etwa +180° C, und, falls erforderlich, in einem geschlossenen Gefäss, unter Druck, in einer Inertgasatmosphäre und/oder unter wasserfreien Bedingungen arbeitet.

Variante a):

Die Umsetzung wird erforderlichenfalls in Gegenwart einer Base durchgeführt.

Als Basen kommen beispielsweise Alkalimetall-hydroxide, -hydride, -amide, -alkanolate, -carbonate, -triphenylmethylide, -diniederalkylamide, -aminoalkylamide oder -niederalkylsilylamide, Naphthalinamine, Niederalkylamine, basische Heterocyclen, Ammoniumhydroxide, sowie carbocyclische Amine in Frage. Beispielhaft seien Natriumhydroxid, -hydrid, -amid, Natrium(m)ethylat, Kalium-tert-butylat, -carbonat, Lithiumtriphenylmethylid, -diisopropylamid, Kalium-3-(aminopropyl)-amid, -bis-(trimethylsilyl)-amid, Dimethylaminonaphthalin, Di- oder Triethylamin, oder Ethyl-diisopropylamin, N-Methyl-piperidin, Pyridin, Benzyltrimethyl-ammoniumhydroxid, 1,5-Diazabicyclo[4.3.0]non-5-en (DBN) sowie 1,8-Diaza-bicyclo[5.4.0]-undec-7-en (DBU) genannt.

Setzt man solche Verbindungen der Formel IIb ein, worin sowohl $X_1$ als auch $X_2$ verethertes Hydroxy bedeuten, gelangt man zu Verbindungen der Formel I, worin $R_2$ Hydroxy bedeutet.

Vorzugsweise setzt man solche Verbindungen der Formel IIb ein, worin Z für O steht.

Zur Herstellung von Verbindungen der Formel I, worin Z für NH steht, geht man zweckmässig von Nitrilen der Formel $X_1$-CO-CH($R_3$)-CN (IIC) aus.

Zur Herstellung des Ausgangsmaterials der Formel IIa geht man beispielsweise von einer Verbindung der Formel

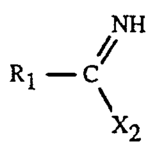

(IId)

oder einem Salz davon aus, worin $X_2$ verethertes Hydroxy, wie Niederalkoxy, z.B. Methoxy oder Ethoxy, bedeutet, und setzt diese mit einer Verbindung der Formel $R_4$-$NH_2$ (IIe) oder einem Salz davon um. Verbindungen der Formel IId lassen sich z.B. aus den entsprechenden Nitrilen herstellen, während Verbindungen der Formel IIb und IIe zum Teil bekannt sind oder in an sich bekannter Weise hergestellt werden können. So sind Verbindungen der Formel IIe beispielsweise in EP 253310 beschrieben bzw. in analoger Weise herstellbar.

Funktionell abgewandelte Derivate von Tautomeren der Formel IIb, worin X Halogen bedeutet, sind beispielsweise zugänglich, indem man von Verbindungen der Formel IIb ausgeht, worin $X_1$ für $R_2$ steht, und setzt diese in an sich bekannter Weise z.B. mit einem Oxalylhalogenid, wie Oxalylchlorid, um. Das resultierende Derivat der Formel IIb′ (X = Halogen) kann weiter in andere entsprechende Derivate der Formel IIb′ umgewandelt werden, beispielsweise durch Umsetzung mit einem Niederalkanolat, wie Natriumethanolat, mit einem Niederalkylthiolat, wie Natriummethylthiolat, mit Ammoniak oder einem entsprechenden Amin, wie Niederalkyl- oder Diniederalkylamin, z.B.Ethylamin oder Diethylamin.

Variante b):

In 5-Tetrazolyl $R_5$ überführbare Reste $X_3$ sind beispielsweise Cyano oder geschütztes 5-Tetrazolyl.

Zur Herstellung von Verbindungen der Formel I, worin $R_5$ 5-Tetrazolyl bedeutet, geht man beispielsweise von Ausgangsmaterial der Formel III aus, worin $X_3$ Cyano bedeutet, und setzt dieses mit einem Azid, wie $HN_3$ oder insbesondere einem Salz, wie Alkalimetallsalz, davonoder mit einem Organozinnazid, wie Tri-(nieder)alkyl- oder Triarylzinnazid, um. Bevorzugte Azide sind beispielsweise Natrium- und Kaliumazid

sowie Tri-$C_1$-$C_4$-alkyl-, z.B. Triethyl- oder Tributylzinnazid, und Triphenylazid.

Als Schutzgruppen von geschütztem 5-Tetrazolyl kommen die üblicherweise in der Tetrazolchemie verwendeten Schutzgruppen in Frage, insbesondere Triphenylmethyl, gegebenenfalls, z.B. durch Nitro, substituiertes Benzyl, wie 4-Nitrobenzyl, Niederalkoxymethyl, wie Methoxy- und Ethoxyethyl, Niederalkylthiomethyl, wie Methylthiomethyl, sowie 2-Cyanoethyl, ferner Niederalkoxyniederalkoxymethyl, wie 2-Methoxyethoxymethyl, Benzyloxymethyl sowie Phenacyl.

Die Abspaltung der Schutzgruppen erfolgt in Anlehnung an bekannte Methoden, beispielsweise wie in J. Green, Protective Groups in Organic Synthesis, Wiley-Interscience (1980) beschrieben. So wird z.B. die Triphenylmethylgruppe üblicherweise durch Hydrolyse, insbesondere in Gegenwart einer Säure, oder Hydrogenolyse in Gegenwart eines Hydrierungskatalysators, 4-Nitrobenzyl z.B. durch Hydrogenolyse in Gegenwart eines Hydrierungskatalysators, Methoxy- oder Ethoxyethoxy z.B. durch Behandeln mit einem Triniederalkyl-, wie Triethyl- oder Tributyl-zinn-bromid, Methylthiomethyl z.B. durch Behandeln mit Trifluoressigsäure, 2-Cyanoethyl z.B. durch Hydrolyse, beispielsweise mit Natronlauge, 2-Methoxyethoxymethyl z.B. durch Hydrolyse, z.B. mit Salzsäure, Benzyloxymethyl und Phenacyl z.B. durch Hydrogenolyse in Gegenwart eines Hydrierungskatalysators abgespalten.

Ein in $SO_3H$ $R_5$ überführbarer Rest ist beispielsweise die Mercaptogruppe. Eine solche Gruppe aufweisende Ausgangsverbindungen der Formel III werden beispielsweise durch an sich bekannte Oxidationsverfahren zu solchen Verbindungen der Formel I oxidiert, worin $R_5SO_3H$ ist. Als Oxidationsmittel kommen beispielsweise anorganische Persäuren, wie Persäuren von Mineralsäuren, z.B. Periodsäure oder Perschwefelsäure, organische Persäuren, wie entsprechende Percarbon- oder Persulfonsäuren, z.B. Perameisen-, Peressig-, Trifluorperessig- bzw. Perbenzoesäure oder p-Toluolpersulfonsäure, oder Gemische aus Wasserstoffperoxid und Säuren, z.B. Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VII. Nebengruppe, z.B. Vanadium-, Molybdän- oder Wolframoxid, zu nennen sind. Die Oxidation wird unter milden Bedingungen, z.B. bei Temperaturen von etwa -50° bis etwa +100° C, durchgeführt.

Unter einer in $PO_3H_2R_5$ überführbare Gruppe ist beispielsweise eine Gruppe $N_2^+A^-$ zu verstehen, wobei $A^-$ für ein Anion einer Säure, wie Mineralsäure, steht. Derartige Diazoniumverbindungen werden beispielsweise in an sich bekannter Weise mit einem P(III)-Halogenid, wie $PCl_3$ oder $PBr_3$, umgesetzt und hydrolytisch aufgearbeitet, wobei solche Verbindungen der Formel I erhältlich sind, worin $R_5PO_3H_2$ ist.

Als in Halogenalkylsulphamoyl $R_5$ überführbarer Rest $X_3$ kommt beispielsweise primäres Amino in Frage.

Zur Herstellung von Verbindungen der Formel I, worin $R_5$ Halogenalkylsulphamoyl bedeutet, setzt man beispielsweise entsprechende Aniline mit einer üblicherweise reaktionsfähig veresterten Halogenalkylsulfonsäure um, wobei gegebenenfalls in Gegenwart einer Base gearbeitet wird. Als bevorzugte reaktionsfähig veresterte Halogensulfonsäure kommt das entsprechende Halogenid, wie Chlorid oder Bromid, in Frage.

Ein in COOH $R_5$ überführbarer Rest $X_3$ steht beispielsweise für funktionell abgewandeltes Carboxy, wie Cyano, verestertes oder amidiertes Carboxy, Hydroxymethyl oder Formyl.

Verestertes Carboxy ist beispielsweise mit einem gegebenenfalls substituierten aliphatischen, cycloaliphatischen oder aromatischen Alkohol verestertes Carboxy. Ein aliphatischer Alkohol ist beispielsweise ein Niederalkanol, wie Methanol, Ethanol, Propanol, Isopropanol, n-Butanol, sec- oder tert.-Butanol, während als cycloaliphatischer Alkohol beispielsweise ein 3- bis 8-gliedriges Cycloalkanol, wie Cyclopentanol, -hexanol oder -heptanol, in Frage kommt. Ein aromatischer Alkohol ist beispielsweise ein Phenol oder heterocyclischer Alkohol, welche jeweils gegebenenfalls substituiert sein können, insbesondere Hydroxypyridin, z.B. 2-, 3- oder 4-Hydroxypyridin.

Amidiertes Carboxy ist beispielsweise Carbamoyl, durch Hydroxy, Amino oder gegebenenfalls substituiertes Phenyl monosubstituiertes, durch Niederalkyl mono- oder disubstituiertes oder durch 4- bis 7-gliedriges Alkylen bzw. 3-Aza-, 3-Niederalkylaza-, 3-Oxo- oder 3-Thiaalkylen disubstituiertes Carbamoyl. Als Beispiele sind Carbamoyl, N-Mono- oder N,N-Diniederalkylcarbamoyl, wie N-Methyl-, N-Ethyl-, N,N-Dimethyl-, N,N-Diethyl- oder N,N-Dipropylcarbamoyl, Pyrrolidino- oder Piperidinocarbonyl, Morpholino-, Piperazino- bzw. 4-Methylpiperazino- sowie Thiomorpholinocarbonyl, Anilinocarbonyl oder durch Niederalkyl, Niederalkoxy und/oder Halogen substituiertes Anilinocarbonyl zu nennen.

Bevorzugtes funktionell abgewandeltes Carboxy ist beispielsweise Niederalkoxycarbonyl, wie Methoxy- oder Ethoxycarbonyl, oder Cyano. Verbindungen der Formel I, worin $R_5$ Carboxy ist, können beispielsweise ausgehend von Verbindungen der Formel III, worin $X_3$ funktionell abgewandeltes Carboxy bedeutet, durch Hydrolyse, insbesondere in Gegenwart einer Base, bzw. ausgehend von solchen Verbindungen der Formel III, worin $X_3$ Hydroxymethyl oder Formyl bedeutet, unter Verwendung üblicher Oxidationsmittel, durch

Oxidation hergestellt werden.

Die Oxidation erfolgt beispielsweise in einem inerten Lösungsmittel, wie einer Niederalkancarbonsäure z.B. Essigsäure, einem Keton, z.B. Aceton, einem Ether, z.B. Tetrahydrofuran, einem heterocyclischen Aromaten, z.B. Pyridin, oder Wasser oder einem Gemisch davon, erforderlichenfalls unter Kühlen oder Erwärmen, z.B. von etwa 0° bis etwa 150° C. Als Oxidationsmittel kommen beispielsweise oxidierende Übergangsmetallverbindungen, insbesondere solche mit Elementen der I., VI., oder VIII. Nebengruppe, in Frage. Als Beispiele seien genannt: Silberverbindungen, wie Silbernitrat, -oxid oder -picolinat, Chromverbindungen, wie Chromtrioxid oder Kaliumdichromat, Manganverbindungen, wie Kaliumferrat, Tetrabutylammonium- oder Benzyl(triethyl)ammoniumper manganat. Weitere Oxidationsmittel sind beispielsweise geeignete Verbindungen mit Elementen der IV. Hauptgruppe, wie Bleidioxid, oder Halogen-Sauerstoff-Verbindungen, wie Natriumdiodat oder Kaliumperiodat.

So wird beispielsweise Hydroxymethyl und Formyl zu Carboxy $R_5$ oxidiert.

Vorzugsweise eignet sich diese Variante zur Herstellung solcher Verbindungen der Formel I, worin die Variablen Bedeutungen haben, die von ungesättigten Resten verschieden sind.

Das Ausgangsmaterial der Formel III ist beispielsweise zugänglich, indem man von Verbindungen der Formel IVa ausgeht und diese in Analogie zu Variante c) mit einer Verbindung der Formel

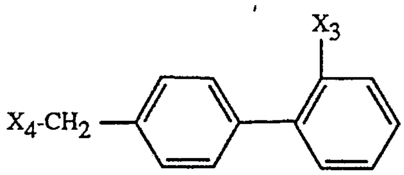

(III),

worin $X_3$ und $X_4$ die vorstehend aufgeführten Bedeutungen haben, umsetzt.

Verbindungen der Formel III sind zum Teil bekannt bzw. können nach bekannten Methoden hergestellt werden, beispielsweise wie in EP 253310 (Seite 52 ff.) dargestellt.

Variante c:

Die Umsetzung von Verbindungen der Formel IVa mit Verbindungen der Formel IVb erfolgt in an sich bekannter Weise vorteilhaft in Gegenwart einer der vorstehend genannten Basen.

$X_4$ bedeutet vorzugsweise Halogen, wie Chlor oder Brom, oder Sulfonyloxy, wie Methan-oder p-Toluolsulfonyloxy.

Die Ausgangsverbindungen der Formel IVa und IVb sind zum Teil bekannt bzw. können in an sich bekannter Weise hergestellt werden. So gelangt man z.B. zu Verbindungen der Formel IVa, indem man in Analogie zu Variante a) von Verbindungen der Formel IIa ausgeht, worin $R_4$ Wasserstoff bedeutet, und setzt diese mit einer Verbindung der Formel IIb um. Verbindungen der Formel IVa, worin $R_2$ und $R_3$ gemeinsam für die Partialstruktur der Formel $-CH=CH-CH=CH-$ stehen, in der gegebenenfalls eine oder zwei der Methingruppen durch $=N-$ ersetzt sind, können beispielsweise hergestellt werden, indem man von einer entsprechenden Verbindung der Formel

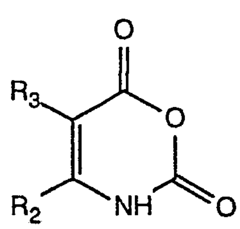

(IVc)

ausgeht und diese, gegebenenfalls in Gegenwart einer Base, mit einem Amidin der Formel IIa umsetzt.

Das Ausgangsmaterial der Formel IVb ist zum Teil aus EP 253310 bekannt bzw. kann unter Verwendung üblicher Methoden hergestellt werden.

Eine verfahrensgemäss erhältliche erfindungsgemässe Verbindung kann in an sich bekannter Weise in eine andere erfindungsgemässe Verbindung übergeführt werden.

Eine Hydroxy aufweisende erfindungsgemässe Verbindung kann nach an sich bekannten Methoden verethert werden. Die Veretherung kann z.B. mit einem Alkohol, wie gegebenenfalls substituiertem Niederalkanol, oder einem reaktionsfähigen Ester desselben erfolgen. Als reaktionsfähige Ester der gewünschten Alkohole kommen beispielsweise solche mit starken anorganischen oder organischen Säuren, wie entsprechende Halogenide, Sulfate, Niederalkansulfonate oder gegebenenfalls substituierte Benzolsulfonate, z.B. Chloride, Bromide, lodide, Methan-, Benzol- oder p-Toluol-sulfonate, in Betracht. Die Veretherung kann z.B. in Gegenwart einer Base, eines Alkalimetallhydrids, -hydroxids, -carbonats oder eines basischen Amins, erfolgen. Umgekehrt können entsprechende Ether, wie Niederalkoxyverbindungen, z.B. mittels starker Säuren, wie Mineralsäuren, z.B. den Halogenwasserstoffsäuren Brom- oder lodwasserstoffsäure, die vorteilhaft in Form von Pyridiniumhalogeniden vorliegen können, oder mittels Lewissäuren, z.B. Halogeniden von Elementen der III. Hauptgruppe oder der entsprechenden Nebengruppen, gespalten werden. Diese Umsetzungen können, falls erforderlich, unter Kühlen oder Erwärmen, z.B. in einem Temperaturbereich von etwa -20° bis etwa 100°C, in An- oder Abwesenheit eines Lösungs- oder Verdünnungsmittels, unter Inertgas und/oder unter Druck und gegebenenfalls in einem geschlossenen Gefäss, durchgeführt werden.

Hydroxymethylgruppen aufweisende erfindungsgemässe Verbindungen können beispielsweise ausgehend von entsprechenden Carboxy oder verestertes Carboxy aufweisenden Verbindungen hergestellt werden, wobei entsprechende Verbindungen in an sich bekannter Weise reduziert werden, z.B. durch Reduktion mit einem gegebenenfalls komplexen Hydrid, wie einem Hydrid gebildet aus einem Element der I. und III. Hauptgruppe des Periodensystems der Elemente, z.B. Boranat oder Alanat, beispielsweise Lithiumborhydrid, Lithium-, Diisobutylaluminiumhydrid (gegebenenfalls ist ein nachgelagerter Reduktionsschritt unter Verwendung von Alkalmetall-, wie Natriumcyanoborhydrid, erforderlich), ferner Diboran.

Falls ein aromatischer Strukturbestandteil durch (Nieder-)Alkylthio substituiert ist, kann man dieses auf übliche Weise zu entsprechendem (Nieder-)Alkansulfinyl bzw. -sulfonyl oxidieren. Als geeignetes Oxidationsmittel für die Oxidation zur Sulfoxidstufe kommen beispielsweise anorganische Persäuren, wie Persäuren von Mineralsäuren, z.B. Periodsäure oder Perschwefelsäure, organische Persäuren, wie entsprechende Percarbon- oder Persulfonsäuren, z.B. Perameisen-, Peressig-, Trifluorperessig- bzw. Perbenzoesäure oder p-Toluolpersulfonsäure, oder Gemische aus Wasserstoffperoxid und Säuren, z.B. Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Übergangsmetalloxide, wie Oxide von Elementen der VII. Nebengruppe, z.B. Vanadium-, Molybdän- oder Wolframoxid, zu nennen sind. Die Oxidation wird unter milden Bedingungen, z.B. bei Temperaturen von etwa -50° bis etwa +100°C, durchgeführt.

Die Oxidation zur Sulfonstufe kann man auch mit Distickstofftetroxid als Katalysator in Gegenwart von Sauerstoff bei tiefen Temperaturen entsprechend durchführen, ebenso wie die direkte Oxidation des (Nieder-)Alkylthio zum (Nieder-)Alkansulfonyl. Jedoch setzt man hierbei üblicherweise das Oxidationsmittel im Überschuss ein.

Weist einer der Variablen, (z.B. $R_2$ und $R_3$) Amino auf, können entsprechende Verbindungen der Formel I, ihre Tautomeren oder Salze in an sich bekannter Weise N-alkyliert werden; ebenso können Carbamoyl bzw. Carbamoyl aufweisende Reste (z.B. $R_2$) N-alkyliert werden. Die (Aryl-)Alkylierung erfolgt z.B. mit einem reaktiven Ester eines (Aryl-)$C_1$-$C_7$-Alkylhalogenids, z.B. -bromid oder -iodid, (Aryl-)$C_1$-$C_7$-Alkylsulfonat, z.B. methansulfonat oder -p-toluolsulfonat, oder einem Di-$C_1$-$C_7$-alkylsulfat, z.B. Dimethylsulfat, vorzugsweise unter basischen Bedingungen, wie in Gegenwart von Natronlauge oder Kalilauge, und vorteilhaft eines Phasentransfer-Katalysators, wie Tetrabutylammoniumbromid oder Benzyltrimethylammoniumchlorid, wobei indes stärker basische Kondensationsmittel, wie Alkalimetallamide, -hydride oder -alkoholate, z.B. Natriumamid, Natriumhydrid oder Natriumethanolat, erforderlich sein können.

In Verbindungen der Formel (I), die als Substituenten eine veresterte oder amidierte Carboxygruppe (z.B. $R_2$ bzw. $R_3$) aufweisen, kann man eine solche Gruppe z.B. mittels Hydrolyse, z.B. in Gegenwart eines basischen Mittels, oder eines sauren Mittels, wie einer Mineralsäure, in eine freie Carboxygruppe überführen. Tert-Butyloxycarbonyl beispielsweise kann weiterhin z.B. in an sich bekannter Weise, wie durch Behandeln mit Trihalogen-, wie Trifluoressigsäure, und Benzyloxycarbonyl z.B. durch katalytische Hydrierung in Gegenwart eines Hydrierungskatakysators, z.B. in der nachstehend beschriebenen Weise, in Carboxy überführt werden.

Ferner kann man in Verbindungen der Formel (I),die als Substituenten eine Carboxygruppe (z.B. $R_3$) aufweisen, insbesondere sofern $R_5$ von Carboxy verschieden ist, diese z.B. durch Behandeln mit einem Alkohol, wie einem Niederalkanol, in Gegenwart eines geeigneten Veresterungsmittels, wie eines sauren Reagens, z.B. einer anorganischen oder organischen Säure oder einer Lewissäure, z.B. Zinkchlorid, oder eines wasserbindenden Kondensationsmittels, z.B. eines Carbodiimids, wie N,N'-Dicyclohexyl-carbodiimid,

oder durch Behandeln mit einem Diazoreagens, wie mit einem Diazoniederalkan, z.B. Diazomethan, in eine veresterte Carboxygruppe (z.B. $R_3$) überführen. Diese kann man auch erhalten, wenn man Verbindungen der Formel I, worin die Carboxygruppe (z.B. $R_2$) in freier Form oder in Salz-, wie Ammonium- oder Metall-, z.B. Alkalimetall-, wie Natrium-oder Kaliumsalzform vorliegt, mit einem reaktionsfähigen Ester eines ($C_1$ -$C_7$-)Alkylhalogenid, z.B. Methyl- oder Ethyl-bromid oder -iodid, oder einem organischen Sulfonsäureester, wie einem entsprechenden ($C_1$-$C_7$-)Alkylester, z.B. Methansulfonsäure- oder p-Toluolsulfonsäuremethylester oder -ethylester, behandelt.

Verbindungen der Formel (I), die als Substituenten eine veresterte Carboxygruppe (z.B. $R_3$) aufweisen, kann man durch Umesterung, z.B. durch Behandeln mit einem Alkohol, üblicherwiese einem höheren als dem der veresterten Carboxygruppe im Ausgangsmaterial entsprechenden Alkohol, in Gegenwart eines geeigneten Umesterungsmittels, wie eines basischen Mittels, z.B. eines Alkalimetall-($C_1$-$C_7$-)alkanoats, -($C_1$-$C_7$-)alkanolats oder -cyanids, wie Natriumacetat, -methanolat, -ethylat, -tert-butanolat oder -cyanid, oder eines geeigneten sauren Mittels, gegebenenfalls unter Entfernung des entstehenden Alkohols, z.B. durch Destillation, in andere Esterverbindungen der Formel (I) umestern. Man kann auch von entsprechenden, sogenannten aktivierten Estern der Formel (I) ausgehen, die als Substituenten eine aktivierte veresterte Carboxygruppe aufweisen (siehe unten), und diese durch Behandeln mit einem ($C_1$-$C_7$-)Alkanol, in einen anderen Ester umwandeln.

Man kann in Verbindungen der Formel (I), die als Substituenten die Carboxylgruppe (z.B. $R_2$) enthalten, diese auch zuerst in ein reaktionsfähiges Derivat, wie ein Anhydrid, inkl. ein gemischtes Anhydrid, wie ein Säurehalogenid, z.B. -chlorid (z.B. durch Behandeln mit einem Thionylhalogenid, z.B. -chlorid), oder ein Anyhdrid mit einem Ameisensäureester, z.B. -($C_1$-$C_7$-)alkylester (z.B. durch Behandeln eines Salzes, wie eines Ammonium- oder Alkalimetallsalzes, mit einem Halogen-, wie Chlorameisensäureester, wie ($C_1$-$C_7$-)Alkylester), oder in einen aktivierten Ester, wie Cyanmethyl-, Nitrophenyl-, z.B. 4-Nitrophenyl-, oder Polyhalogenphenyl-, z.B. Pentachlorphenylester (z.B. durch Behandeln mit einer entsprechenden Hydroxyverbindung in Gegenwart eines geeigneten Kondensationsmittels, wie N,N′-Dicyclohexyl-carbodiimid) überführen, und ein solches reaktionsfähiges Derivat dann mit einem Amin umsetzen und so zu Amidverbindungen der Formel (I) gelangen, die als Substituenten eine amidierte Carboxygruppe aufweisen. Dabei kann man diese direkt oder über Zwischenverbindungen erhalten; so kann man z.B. einen aktivierten Ester, wie einen 4-Nitrophenylester, einer Verbindung der Formel I mit einer Carboxygruppe zuerst mit einem 1-unsubstituierten Imidazol umsetzen und die so entstandene 1-Imidazolylcarbonylverbindung mit einem Amin in Reaktion bringen. Man kann aber auch andere, nicht-aktivierte Ester, wie ($C_1$-$C_7$-)Alkylester von Verbindungen der Formel (I), die als Substituenten z.B. ($C_2$-$C_8$-)Alkoxycarbonyl (z.B. $R_2$) aufweisen, mit Aminen zur Reaktion bringen.

Weist ein aromatischer Ring als Substituenten ein Wasserstoffatom auf, so kann dieses mit Hilfe eines Halogenierungsmittels in üblicher Weise durch ein Halogenatom ersetzt, z.B. mit Brom, Hypobromsäure, Acylhypobromite oder andere organische Bromverbindungen, z.B. N-Bromsuccinimid, N-Bromacetamid, N-Bromphthalimid, Pyridiniumperbromid, Dioxandibromid, 1,3-Dibrom-5,5-dimethylhydantoin, 2,4,4,6-Tetrabrom-2,5-cyclohexandien-1-on, bromiert bzw. mit elementarem Chlor, z.B. in einem halogenierten Kohlenwasserstoff, wie Chloroform, und unter Kühlen, z.B. bis auf etwa -10° bis etwa +100° C, chloriert werden.

Enthält ein aromatischer Ring in den erfindungsgemässen Verbindungen eine Aminogruppe, so kann diese in üblicher Weise diazotiert werden, z.B. durch Behandeln mit einem Nitrit, z.B. Natriumnitrit, in Gegenwart einer geeigneten Protonsäure, z.B. Mineralsäure, wobei die Reaktionstemperatur vorteilhaft unter etwa 5° C gehalten wird. Die so erhältliche, in Salzform vorliegende Diazoniumgruppe kann man nach analogen Verfahren beispielsweise wie folgt substituieren: durch die Hydroxygruppe analog der Phenolverkochung in Gegenwart von Wasser; durch eine Alkoxygruppe durch Behandeln mit einem entsprechenden Alkohol, wobei Energie zugeführt werden muss; durch das Fluoratom analog der Schiemann-Reaktion bei der Thermolyse von entsprechenden Diazoniumtetrafluorboraten; durch die Halogenatome Chlor, Brom oder Iod sowie die Cyanogruppe analog der Sandmeyer-Reaktion bei der Umsetzung mit entsprechenden Cu(I)-Salzen, zunächst unter Kühlen, z.B. auf etwa unter 5° C, und anschliessendem Erhitzen, z.B. auf etwa 60° bis etwa 150° C.

Enthalten die Verbindungen der Formel (I) ungesättigte Reste, wie (Nieder-)Alkenyl oder (Nieder-)Alkinylgruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Nickel, wie Raney-Nickel, sowie Edelmetalle bzw. deren Derivate, z.B. Oxide, geeignet sind, wie Palladium, Platinoxid, die gegebenenfalls auf Trägermaterialien, z.B. auf Kohle oder Calciumcarbonat, aufgezogen sein können. Die Hydrierung kann vorzugsweise bei Drucken zwischen 1 und etwa 100 at und bei Raumtemperatur zwischen etwa -80° bis etwa 200° C, vor allem

zwischen Raumtemperatur und etwa 100 °C, durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Weiterhin kann in Verbindungen der Formel I, worin z.B. einer der Reste $R_1$ und/oder Alk Halogen, wie Chlor, bedeutet, Halogen durch Umsetzung mit einem entsprechenden gegebenenfalls substituierten Amin, Alkohol, insbesondere einem Salz davon, oder Mercaptan, insbesondere einem Salz davon, ausgetauscht werden.

Die Erfindung betrifft insbesondere die in den Beispielen beschriebenen Verfahren.

Salze von Verbindungen der Formel (I) oder deren Tautomeren können in an sich bekann ter Weise hergestellt werden. So erhält man beispielsweise Säureadditionssalze von Verbindungen der Formel (I) oder ein Tautomeres davon durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedingungen können die erfindungsgemässen Verbindungen mit salzbildenden, insbesondere basischen Eigenschaften, in freier Form oder bevorzugt in Form von Salzen erhalten werden.

Infolge der engen Beziehung zwischen der neuen Verbindung in freier Form und in Form ihrer Salze sind im Vorausgegangenen und nachfolgend unter der freien Verbindung oder ihren Salzen sinn- und zweckgemäss gegebenenfalls auch die entsprechenden Salze bzw. die freie Verbindung zu verstehen.

Die neuen Verbindungen einschliesslich ihrer Salze von salzbildenden Verbindungen können auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Racematgemische können auf Grund der physikalisch-chemischen Unterschiede der Bestandteile in bekannter Weise in die reinen Isomeren oder Racemate getrennt aufgetrennt werden, beispielsweise durch fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenether, wobei nur ein Enantiomeres komplexiert wird, oder durch Überführung in diastereomere Salze, z.B. durch Umsetzung eines basischen Endstoffracemats mit einer optisch aktiven Säure, wie Carbonsäure, z.B. Wein- oder Äpfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes und/oder seiner Racemate bzw. Antipoden verwendet oder insbesondere unter den Reaktionsgedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, die speziell für die Herstellung der erfindungsgemässen Verbindungen entwickelt wurden, ihre Verwendung und Verfahren zu ihrer Herstellung bilden ebenfalls einen Gegenstand der Erfindung, wobei die Variablen Z, $R_1$, $R_2$, $R_3$, $R_4$ und $R_5$ die für die jeweils bevorzugten Verbindungsgruppen der Formel I bzw. Tautomere davon angegebenen Bedeutungen haben. Insbesondere sind Verbindungen der Formel III, ihre Tautomeren und Salze, worin $X_3$ Cyano bedeutet, als Ausgangsmaterial bevorzugt.

Die Erfindung betrifft ebenfalls die Verwendung der Verbindungen der Formel (I) bzw. Tautomere davon oder von pharmazeutisch verwendbaren Salzen von solchen Verbindungen mit salzbildenden Eigenschaften, insbesondere als pharmakologische, in erster Linie Angiotensin-II-antagonisierende Wirksubstanzen. Dabei kann man sie, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Angiotensin-II-Antagonisten, verwenden.

Die Erfindung betrifft gleichfalls pharmazeutische Präparate, die die erfindungsgemässen Verbindungen oder pharmazeutisch verwendbare Salze derselben als Wirkstoffe enthalten, sowie Verfahren zu ihrer Herstellung.

Bei den erfindungsgemässen pharmazeutischen Präparaten, welche die erfindungsgemässe Verbindung

oder pharmazeutisch verwendbare Salze davon enthalten, handelt es sich um solche zur enteralen, wie oralen, ferner rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffes hängt von dem Alter und dem individuellen Zustand sowie von der Applikationsweise ab.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und"bder Polyethylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B natürliche oder synthetisch Triglyceride, Paraffinkohlenwasserstoffe, Polyethylenglyckole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Dosierung des Wirkstoffes hängt von der Warmblüter-Spezies, dem Alter und dem individuellen Zustand sowie der Applikationsweise ab. Im Normalfall ist für einen etwa 75 kg schweren Patienten bei oraler Applikation eine ungefähre Tagesdosis von etwa 10 mg bis etwa 250 mg zu veranschlagen.

Die nachfolgenden Beispiele illustrieren die oben beschriebene Erfindung; sie sollen jedoch diese in ihrem Umfang in keiner Weise einschränken.

Beispiel 1:

2-n-Butyl-1,6-dihydro- 1- [(2′-cyanobiphenyl-4-yl)methyl]-6-oxopyrimidin (2,0 g, 5,82 mmol) und Tributylzinnazid [Synthesis 1976 ,329] (3,86 g, 11,64 mmol) in o-Xylol (50 ml) werden während 24 Stunden unter Rückfluss gerührt. Das Reaktionsgemisch wird im Vakuum eingedampft und in einem Gemisch von $CH_2Cl_2/CH_3OH/NH_3$ (5:3:1,100 ml) während 30 Minuten gerührt. Nach erneutem Eindampfen im Vakuum wird der Rückstand mittels Flash-Chromatographie (Kieselgel 60, 40-63 µm, $CH_2Cl_2/CH_3OH/NH_3$ = 80:10:1) aufgetrennt und das Produkt aus Ethanol-$H_2O$ umkristallisiert. Man erhält so weisse Kristalle von 2-n-Butyl-1,6-dihydro-1-[(2′-(1H-tetrazolyl-5-yl)biphenyl-4-yl)-methyl]-6-oxopyrimidin. Smp.: 180-182° C.

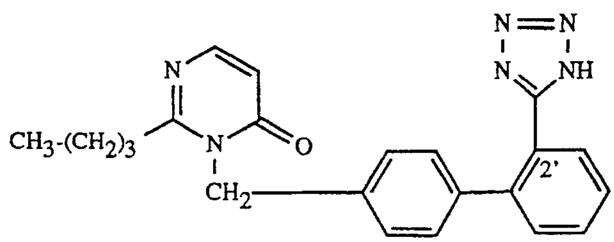

Das Ausgangsmaterial kann wie folgt hergestellt werden:

a) Zu einer Lösung von 5,7 g (3,05 mmol) 2-n-Butyl-4-chlor-6-hydroxypyrimidin [J. Chem. Soc. 1964 ,3204] in 60 ml DMF wird NaH (80% in Weissöl, 0,92 g, 30,5 mmol) portionenweise bei Raumtemperatur zugegeben. Nach beendeter Zugabe wird noch 30 Minuten bei Raumtemperatur gerührt und anschliessend eine Lösung von 8,3 g (30,5 mmol) 4-Brommethyl-2′-cyanobiphenyl [EP 253310] in 50 ml DMF zugetropft. Das Reaktionsgemisch wird 12 Stunden bei Raumtemperatur gerührt und anschliessend im Vakuum eingedampft. Der Rückstand wird mit Essigester versetzt und zweimal mit $H_2O$ gewaschen, getrocknet ($Na_2SO_4$) und im Vakuum eingedampft. Flash-Chromatographie (Kieselgel 60,40-63 µm, Hexan-Essigester = 2:1) ergibt 2-n-Butyl-4-chlor-1,6-dihydro-[(2′-cyanobiphenyl-4-yl)-methyl]-6-oxopyrimidin, welches aus Diethylether umkristallisiert wird. Es verbleiben weisse Kristalle. Smp. 96-98° C.

b) Ein Gemisch aus 5,5 g (14,5 mmol) 2-n-Butyl-4-chlor-1,6-dihydro-[(2′-cyanobiphenyl-4-yl)-methyl]-6-oxopyrimidin in 80 ml Methanol und 1,62 g (16 mmol) Triethylamin wird in Gegenwart von 5 % Pd-C (0,5 g) bei Raumtemperatur und 1 atm $H_2$ während 1 Stunde hydriert. Der Katalysator wird abfiltriert und das Filtrat im Vakuum eingedampft. Der Rückstand wird mit Essigester versetzt und zweimal mit $H_2O$ gewaschen, getrocknet ($Na_2SO_4$) und im Vakuum eingedampft. Man erhält so 2-n-Butyl-1,6-dihydro-1-[-(2′-cyanobiphenyl-4-yl)methyl]-6-oxopyrimidin, welches ohne weitere Reinigung umgesetzt wird.

## Beispiel 2:

Ausgehend von 2-n-Butyl-4-chlor-1,6-dihydro-1-[(2′-cyanobiphenyl-4-yl)methyl]-6-oxopyrimidin (Beispiel 1a) und Tributylzinnazid erhält man beispielsweise in der in Beispiel 1 beschriebenen Weise 2-n-Butyl-4-chlor-1,6-dihydro-1-[(2′-(1H-tetrazol-5-yl)biphenyl-4-yl)-methyl]-6-oxopyrimidin. Smp.: 127° C (Zers.)

## Beispiel 3:

Ausgehend von 2-n-Butyl-1,6-dihydro-4-dimethylamino-1-[(2′-cyanobiphenyl-4-yl)methyl] -6-oxopyrimidin und Tributylzinnazid erhält man beispielsweise in der in Beispiel 1 beschriebenen Weise 2-n-Butyl-1,6-dihydro-4-dimethyl-amino-1-[(2′-(1H-tetrazol-5-yl)biphenyl-4-yl)methyl]-6-oxopyrimidin. Smp.: 246° C (Zersetzung).

Das Ausgangsmaterial kann beispielsweise wie folgt hergestellt werden:

2,0 g (5,36 mmol) 2-n-Butyl-4-chlor-1,6-dihydro-1-[(2′-cyanobiphenyl-4-yl)-methyl]-6oxo-pyrimidin und 33 % in Ethanol (3,8 ml, 21,4 mmol) Dimethylamin in 10 ml Ethanol werden während 12 Stunden unter Rückfluss gerührt. Nach dem Eindampfen im Vakuum wird der Rückstand mit Essigester versetzt und zweimal mit $H_2O$ gewaschen, getrocknet ($Na_2SO_4$) und im Vakuum eingedampft. Flash-Chromatographie (Kieselgel 60, 40-63 µm, Hexan/Essigester 1:2) ergibt 2-n-Butyl-1,6-dihydro-4-dimethylamino-1-[(2′-cyanobiphenyl-4-yl)methyl]-6-oxopyrimidin als weissen Schaum, der direkt weiterverarbeitet wird.

## Beispiel 4:

Ausgehend von 2-n-Butyl-1,6-dihydro-4-methyl-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-methyl-1-[(2′-tetrazol-5-yl)biphenyl-4-yl)methyl]-6-oxo-pyrimidin. Smp. 213-216 °C.

Das Ausgangsmaterial kann beispielsweise wie folgt hergestellt werden:

Durch Alkylierung von 2-n-Butyl-4-hydroxy-6-methylpyrimidin [J. Chem. Soc. 1963, 5642] mit 4-Brommethyl-2′-cyanobiphenyl in der in Beispiel 1a beschriebenen Weise erhält man 2-n-Butyl-1,6-dihydro-4-methyl-1-[(2′-cyanobiphenyl-4-yl)methyl]-6-oxopyrimidin. Das durch Flash-Chromatographie (Kieselgel 60, 40-63 µm, $CH_2Cl_2/CH_3OH$ = 98:2) erhaltene Produkt wird direkt weiterverarbeitet.

Beispiel 5:

Ausgehend von 2-n-Butyl-1,6-dihydro-4-methoxy-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-methoxy-1-[(2′-(1H-tetrazol-5-yl)biphenyl-4-yl)methyl]-6-oxopyrimidin. Smp. 223-225 °C.

Das Ausgangsmaterial kann beispielsweise wie folgt hergestellt werden:

Zu einer Lösung von 343 mg (6,35 mmol) Natriummethylat in 15 ml Methanol werden 1,0 g (2,65 mmol) 2-n-Butyl-4-chlor-1,6-dihydro-[(2′-cyanobiphenyl-4-yl)-methyl]-6-oxo-pyrimidin zugegeben. Das Reaktionsgemisch wird 12 Stunden bei Raumtemperatur gerührt und anschliessend im Vakuum eingedampft. Der Rückstand wird mit Essigester versetzt und zweimal mit $H_2O$ gewaschen, getrocknet ($Na_2SO_4$) und im Vakuum eingedampft. Flash-Chromatographie (Kieselgel 60, 40-63 µm, Hexan/Essigester = 1:1) ergibt 2-n-Butyl-1,6-dihydro-4-methoxy-1-[(2′ -cyanobiphenyl-4-yl)methyl]-6-oxopyrimidin, welches direkt weiterverarbeitet wird.

Beispiel 6:

Ausgehend von 2-n-Butyl-1,6-dihydro-4-morpholino-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-morpholino-1-[2′-(1H-tetrazol-5-yl)biphenyl-4-yl)methyl]-6-oxopyrimidin. Smp. 278 °C (aus Essigester).

Das Ausgangsmaterial kann beispielsweise wie folgt hergestellt werden:

1,0 g (2,65 mmol) 2-n-Butyl-4-chlor-1,6-dihydro-1-[(2′-cyanobiphenyl-4-yl)-methyl]-6-oxopyrimidin und 0,92 ml(10,6 mmol) Morpholin in 20 ml n-Butanol werden während 12 Stunden unter Rückfluss gerührt. Nach dem Eindampfen im Vakuum wird der Rückstand mit Essigester versetzt und zweimal mit $H_2O$ gewaschen, getrocknet ($Na_2SO_4$) und im Vakuum eingedampft. Der Rückstand wird aus Essigester/Hexan umkristallisiert. Man erhält so 2-n-Butyl-1,6-dihydro-4-morpholino-1-[(2′-cyanobiphenyl-4-yl)-methyl]-6-oxo-pyrimidin. Smp. 124-126 °C.

Beispiel 7:

Ausgehend von 2-n-Butyl-1,6-dihydro-4-methylthio-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-methylthio-1-[2′-(1H-tetrazol-5-yl)biphenyl-4-yl)methyl]-6-oxopyrimidin. Smp. 205 °C (aus Essigester).

Das Ausgangsmaterial kann beispielsweise wie folgt hergestellt werden:

870 mg (2,30 mmol) 2-n-Butyl-4-chlor-1,6-dihydro-1-[(2′-cyanobiphenyl-4-yl)methyl]-6-oxopyrimidin und 180 mg (2,53 mmol) Natrium-methanthiolat in 10 ml 1,3-Dimethyl-3,4,5,6-tetrahyro-2(1H)-pyrimidinon werden 30 Minuten bei Raumtemperatur gerührt. Nach Zugabe von $H_2O$ wird mit Essigester extrahiert, die organische Phase mit $H_2O$ gewaschen, getrocknet ($Na_2SO_4$) und im Vakuum eingedampft. Flash-Chromatographie (Kieselgel 60, 40-63 µm, Hexan/Essigester = 1:1) ergibt 2-n-Butyl-1,6-dihydro-4-methylthio-1-[(2′-cyanobiphenyl-4-yl)methyl]-6-oxopyrimidin als Harz, welches direkt weiterverarbeitet wird.

Beispiel 8:

240 mg (0,55 mmol) 2-n-Butyl-1,6-dihydro-4-methylthio-1-[2′-(1H-tetrazol-5-yl)biphenyl-4-yl)methyl]-6-oxopyrimidin und 500 mg (1,45 mmol) 50 % 3-Chlorperbenzoesäure in 10 ml $CH_2Cl_2$ werden während 12

Stunden bei Raumtemperatur gerührt. Nach dem Eindampfen im Vakuum wird der Rückstand mit Essigester versetzt und zweimal mit $H_2O$ gewaschen, getrocknet ($Na_2SO_4$) und im Vakuum eingedampft. Flash-Chromatographie (Kieselgel 60, 40-63 µm, $CH_2Cl_2/CH_3OH/NH_3$ = 80:10:1) ergibt 2-n-Butyl-1,6-dihydro-4-methansulfonyl- 1-[2′-(1H-tetrazol-5-yl)biphenyl-4-yl)methyl]-6-oxopyrimidin als amorphen Festkörper.

Beispiel 9:

Ausgehend von 2-n-Butyl-1,6-dihydro-4-(2-hydroxyethyoxy)-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-(2-hydroxyethoxy)-1-[2\-(1H-tetrazol-5-yl)biphenyl-4-yl)methyl]-6-oxopyrimidin. Smp. 150° C (aus Isopropanol/Essigester).

Das Ausgangsmaterial kann beispielsweise wie folgt hergestellt werden:

Zu einer Lösung von 122 mg (5,3 mmol) Natrium in 20 ml Ethan-1,2-diol wird 1 g (2,65 mmol) 2-n-Butyl-4-chlor-1,6-dihydro-1-[2′-(1H-tetrazol-5-yl)biphenyl-4-yl)methyl]-6-oxopyrimidin zugegeben und das Reaktionsgemisch während 1 Stunde bei 70° gerührt. Nach dem Eindampfen im Vakuum wird der Rückstand mit Essigester gewaschen, getrocknet ($Na_2SO_4$) und im Vakuum eingedampft. Man erhält so 2-n-Butyl-1,6-dihydro-4-(2-hydroxyethoxy)-1-[2′-(1H-tetrazol-5-yl)biphenyl-4-yl)methyl]-6-oxopyrimidin vom Smp. 119-121° (aus Essigester/Hexan).

Beispiel 10:

Ausgehend von 2-n-Butyl-1,6-dihydro-4-(2-methoxyethoxy)-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-(2-methoxyethoxy)-1-[2′-(1H-tetrazol-5-yl)biphenyl-4-yl)methyl]-6-oxopyrimidin. Smp. 195-197° C (aus Essigester).

Das Ausgangsmaterial kann beispielsweise wie folgt hergestellt werden:

Zu einer Lösung von 122 mg (5,2 mmol) Natrium in 20 ml 2-Methoxyethanol wird 1,0 g (2,65 mmol) 2-n-Butyl-4-chlor-1,6-dihydro-1-[(2′-cyanobiphenyl-4-yl)-methyl]-6-oxopyrimidin zug egeben und das Reaktionsgemisch während 12 Stunden bei Raumtemperatur gerührt. Nach dem Eindampfen im Vakuum wird der Rückstand mit Essigester versetzt und mit $H_2O$ gewaschen, getrocknet ($Na_2SO_4$) und im Vakuum eingedampft. Flash-Chromatogrpahie (Kieselgel 60, 40-63 µm, Essigester/Hexan = 1:1) ergibt 2-n-Butyl-1,6-dihydro-4-(2-methoxyethoxy)-1-[(2′-cyanobiphenyl-4-yl)methyl]-6-oxopyrimidin als zähes Oel, welches direkt weiterverarbeitet wird.

Beispiel 11:

Ausgehend von 2-n-Butyl-1,6-dihydro-4-(2-dimethylaminoethoxy)-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-(2-dimethylaminoethoxy)-1-[2′-( 1H-tetrazol-5-yl)biphenyl-4-yl)methyl]-6-oxopyrimidin als amorphen Festkörper.

Das Ausgangsmaterial kann beispielsweise wie folgt hergestellt werden:

Zu einer Lösung von 142 mg (5,92 mmol) Natrium in 20 ml 2-Dimethylamino-ethanol wird 1,0 g (2,65 mmol) 2-n-Butyl-4-chlor-1,6-dihydro-1-[(2′-cyanobiphenyl-4-yl)methyl]-6-oxopyrimidin zugegeben und das Reaktionsgemisch während 12 Stunden bei Raumtemperatur gerührt. Nach dem Eindampfen im Vakuum wird der Rückstand mit Essigester versetzt und mit $H_2O$ gewaschen, getrocknet ($Na_2SO_4$) und im Vakuum eingedampft. Flash-Chromatographie (Kieselgel 60, 40-63 µm, $CH_2Cl_2/CH_3OH$ = 95:5) ergibt 2-n-Butyl-1,6-dihydro-4-(2-dimethylaminoethoxy)-1-[(2′-cyanobiphenyl-4-yl)methyl]-6-oxopyrimidin als Oel, welches direkt weiterverarbeitet wird.

Beispiel 12:

Ausgehend von 4-Benzyloxy-2-n-butyl-1,6-dihydro-1-[(2′-cyanobiphenyl-4-yl)methyl]-6-oxopyrimidin und Triburylzinnazid erhält man beispielsweise in der in Beispiel 1 beschriebenen Weise 4-Benzyloxy-2-n-butyl-1,6-dihydro-1-[2′-(1H-tetrazol-5-yl)biphenyl-4-yl)methyl]-6-oxopyrimidin. Smp. 240° C (aus N,N-

Dimethylformamid/$H_2O$).

Das Ausgangsmaterial kann beispielsweise wie folgt hergestellt werden:

Zu einer Lösung von 122 mg (5,3 mmol) Natrium in 10 ml Benzylalkohol wird 1,0 g (2,65 mmol) 2-n-Butyl-4-chlor-1,6-dihydro-1-[(2′-cyanobiphenyl-4-yl)methyl]-6-oxopyrimidin zugegeben und das Reaktionsgemisch während 3 Stunden bei 60 °C gerührt. Nach dem Eindampfen im Vakuum wird der Rückstand mit Essigester versetzt und mit $H_2O$ gewaschen, getrocknet ($Na_2SO_4$) und im Vakuum eingedampft. Flash-Chromatogrpahie (Kieselgel 60, 40-63 µm, Hexan/Essigester = 3:1) ergibt 4-Benzyloxy-2-n-butyl-1,6-dihydro-1-[(2′-cyanobiphenyl-4-yl)methyl]-6-oxopyrimidin als Oel, welches direkt weiterverarbeitet wird.

Beispiel 13:

Ausgehend von 2,4-Di-n-butyl-1,6-dihydro-1-[(2′-cyanobiphenyl-4-yl)methyl]-6-oxopyrimidin und Tributylzinnazid erhält man beispielsweise in der in Beispiel 1 beschriebenen Weise 2,4-Di-n-butyl-1,6-dihydro-1-[2′-(1H-tetrazol-5-yl)biphenyl-4-yl)methyl]-6-oxopyrimidin. Smp. 167-169 °C (aus Essigsäure/$H_2O$).

Das Ausgangsmaterial kann beispielsweise wie folgt hergestellt werden:

a) Zu einer Lösung von 920 ml (40 mmol) Natrium in 40 ml absolutem Ethanol werden unter Eiskühlung 2,73 g (20 mmol) n-Valeroylamidin.HCl zugegeben. Nach beendeter Zugabe wird 30 Minuten gerührt und anschliessend eine Lösung von 3,44 g (20 mmol) 3-Oxo-hexansäure-ethylester in 5 ml absolutem Ethanol zugetropft. Das Reaktionsgemisch wird während 12 Stunden bei Raumtemperatur gerührt und anschliessend im Vakuum eingedampft. Der Rückstand wird in $H_2O$ gelöst und durch Zugabe von Essigsäure angesäuert. Man extrahiert mit Essigester, wäscht mit $H_2O$, trocknet ($Na_2SO_4$) und dampft im Vakuum ein. Flash-Chromatographie (Kieselgel 60, 40-63 µm, Hexan/Essigester = 1:1) ergibt 2,4-Di-n-butyl-6-hydroxypyridmidin, welches direkt weiterverarbeitet wird.

b) Durch Alkylierung von 2,4-Di-n-butyl-6-hydroxypyrimidin mit 4-Brommethyl-2\-cyanobiphenyl in der in Beispiel 1a) beschriebenen Weise und Flash-Chromatographie (Kieselgel 60, 40-63 µm, Hexan/Essigester = 2:1) erhält man 2,4-Di-n-butyl-1,6-dihydro-1-[(2′-cyanobiphenyl-4-yl)-methyl]-6-oxo-pyrimidin als Oel, welches direkt weiterverarbeitet wird.

Beispiel 14:

Ausgehend von 2-n-Propyl-1,6-dihydro-4-methyl-1-[(2′-cyanobiphenyl-4-yl)methyl]-6-oxopyrimidin und Tributylzinnazid erhält man beispielsweise in der in Beispiel 1 beschriebenen Weise 2-n-Propyl-1,6-dihydro-4-methyl-1-[2′-(1H-tetrazol-5-yl)biphenyl-4-yl)methyl]-6-oxopyrimidin. Smp. 203-205 °C (aus Essigester).

Das Ausgangsmaterial kann beispielsweise wie folgt hergestellt werden:

a) Durch Umsetzung von 3-Oxo-pentansäure-ethylester mit Acetamidin.HCl und Natrium in absolutem Ethanol in der in Beispiel 13a) beschriebenen Weise erhält man 2-n-Propyl-4-methyl-6-hydroxypyrimidin, welches direkt weiterverarbeitet wird.

b) Durch Alkylierung von 2-n-Propyl-4-methyl-6-hydroxypyrimidin mit 4-Brommethyl-2′-cyanobiphenyl in der in Beispiel 1a) beschriebenen Weise und Flash-Chromatographie (Kieselgel 60, 40-63 µm, Hexan/Essigester 3:1) erhält man 2-n-Propyl-1,6-dihydro-4-methyl-1-[(2′-cyanobiphenyl-4-yl)methyl]-6-oxopyrimidin. Smp. 101-103 °C (aus Diethylether/Hexan).

Beispiel 15:

Ausgehend von 2-n-Butyl-1,6-dihydro-4,5-dimethyl-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4,5-dimethyl-1-[2′-(1H-tetrazol-5-yl)biphenyl-4-yl)methyl]-6-oxopyrimidin. Smp. 198-200 °C (aus Essigsäure/$H_2O$).

Das Ausgangsmaterial kann beispielsweise wie folgt hergestellt werden:

a) Durch Umsetzung von 2-Methyl-acetessigsäure-ethylester mit n-Valeroylamidin.HCl und Natrium in absolutem Ethanol in der in Beispiel 1 3a) beschriebenen Weise erhält man 2-n-Butyl-4,5-dimethyl-6-hydroxypyrimidin, welches direkt weiterverarbeitet wird.

b) Durch Alkylierung von 2-n-Butyl-4,5-dimethyl-6-hydroxypyrimidin mit 4-Brommethyl-2′-cyanobiphenyl in der in Beispiel 1a) beschriebenen Weise und Flash-Chromatographie (Kieselgel 60, 40-63 µm, Hexan/Essigester = 2:1) erhält man 2-n-Butyl-1,6-dihydro-4,5-dimethyl-1-[(2′-cyanobiphenyl-4-yl)-

methyl]-6-oxopyrimidin, welches direkt weiterverarbeitet wird.

Beispiel 16:

Ausgehend von 2-n-Butyl-1,6-dihydro-4-trifluormethyl-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-trifluormethyl-1-[2′-(1H-tetrazol-5-yl)biphenyl-4-yl)methyl]-6-oxopyrimidin. Smp. 138-140° C (aus Diethylether).

Das Ausgangsmaterial kann beispielsweise wie folgt hergestellt werden:

a) Durch Umsetzung von Trifluoracetessigsäure-ethylester mit n-Valeroylamidin.HCl und Natrium in absolutem Ethanol in der in Beispiel 13a) beschriebenen Weise erhält man 2-n-Butyl-4-trifluormethyl-6-hyroxypyrimidin, welches direkt weiterverarbeitet wird.

b) Durch Alkylierung von 2-n-Butyl-4-trifluormethyl-6-hydroxypyrimidin mit 4-Brommethyl-2′-cyanobiphenyl in der in Beispiel 1a) beschriebenen Weise und Flash-Chromatographie (Kieselgel 60, 40-63 µm, Hexan/Essigester = 4:1) erhält man 2-n-Butyl-1,6-dihydro-4-trifluormethyl-1-[(2′ -cyanobiphenyl-4-yl)-methyl]-6-oxopyrimidin, welches direkt weiterverarbeitet wird.

Beispiel 17:

Ausgehend von 2-n-Butyl-1,6-dihydro-5-ethyl-4-methyl-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-5-ethyl-4-methyl-1-[(2′-(1H-tetrazol-5-yl)biphenyl-4-yl)methyl]-6-oxopyrimidin. Smp. 162-163° C (aus Acetonitril).

Das Ausgangsmaterial kann beispielsweise wie folgt hergestellt werden:

a) Durch Umsetzung von 2-Ethyl-acetessigsäure-ethylester mit n-Valeroylamidin.HCl und Natrium in absolutem Ethanol in der in Beispiel 13a) beschriebenen Weise erhält man 2-n-Butyl-5-ethyl-4-methyl-5-hydroxypyrimidin, welches direkt weiterverarbeitet wird.

b) Durch Alkylierung von 2-n-Butyl-5-ethyl-4-methyl-6-hydroxypyrimidin mit 4-Brommethyl-2′-cyanobiphenyl in der in Beispiel 1a) beschriebenen Weise und Flash-Chromatographie (Kieselgel 60, 40-63 µm, Hexan/Essigester = 4:1) erhält man 2-n-Butyl-1,6-dihydro-5-ethyl-4-methyl-1- [(2′-cyanobiphenyl-4-yl)-methyl]-6-oxopyrimidin, welches direkt weiterverarbeitet wird.

Beispiel 18:

Ausgehend von 2-n-Butyl-1,6-dihydro-5-isopropyl-4-methyl-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-5-isopropyl-4-methyl-1-[2′-(1H-tetrazol-5-yl)biphenyl-4-yl)methyl]-6-oxopyrimidin. Smp. 182° C (aus Diethylether).

Das Ausgangsmaterial kann beispielsweise wie folgt hergestellt werden:

a) Durch Umsetzung von 2-Isopropyl-acetessigsäure-ethylester mit n-Valeroylamidin.HCl und Natrium in absolutem Ethanol in der in Beispiel 13a) beschriebenen Weise erhält man 2-n-Butyl-5-isopropyl-4-methyl-6-hydroxy-pyrimidin, welches direkt weiterverarbeitet wird.

b) Durch Alkylierung von 2-n-Butyl-5-isopropyl-4-methyl-6-hydroxypyrimidin mit 4-Brommethyl-2′-cyanobiphenyl in der in Beispiel 1a) beschriebenen Weise und Flash-Chromatographie (Kieselgel 60, 40-63 µm, Hexan/Essigester = 4:1) erhält man 2-n-Butyl-1,6-dihydro-5-isopropyl-4-methyl-1-[(2′-cyanobiphenyl-4-yl)methyl]-6-oxopyrimidin, welches direkt weiterverarbeitet wird.

Beispiel 19:

Ausgehend von 2-n-Butyl-1,6-dihydro-4-ethyl-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-ethyl-1-[2′-(1H-tetrazol-5-yl)biphenyl-4-yl)methyl]-6-oxopyrimidin. Smp. 178-180° C (aus Acetonitril).

Das Ausgangsmaterial kann beispielsweise wie folgt hergestellt werden:

a) Durch Umsetzung von 3-Oxo-valeriansäure-ethylester mit n-Valeroylamidin.HCl und Natrium in absolutem Ethanol in der in Beispiel 13a) beschriebenen Weise erhält man 2-n-Butyl-4-ethyl-6-hydroxypyridimdin, welches direkt weiterverarbeitet wird.

b) Durch Alkylierung von 2-n-Butyl-4-ethyl-6-hydroxypyrimidin mit 4-Brommethyl-2′-cyanobiphenyl in der in Beispiel 1a) beschriebenen Weise und Flash-Chromatographie (Kieselgel 60, 40-63 µm, Hexan/Essigester = 2:1) erhält man 2-n-Butyl-1,6-dihydro-4-ethyl-1-[(2′-cyanobiphenyl-4-yl)-methyl]-6-oxopyrimidin, welches direkt weiterverarbeitet wird.

Beispiel 20:

Ausgehend von 2-n-Butyl-1,6-dihydro-4-n-propyl-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-n-propyl-1-[2′-(1H-tetrazol-5-yl)biphenyl-4-yl)methyl]-6-oxopyrimidin. Smp. 177-178 °C (aus Essigester/Hexan).

Das Ausgangsmaterial kann beispielsweise wie folgt hergestellt werden:

a) Durch Umsetzung von Butylrylessigsäure-ethylester mit n-Valeroylamidin.HCl und Natrium in absolutem Ethanol in der in Beispiel 13a) beschriebenen Weise erhält man 2-n-Butyl-4-n-propyl-6-hydroxypyrimidin, welches direkt weiterverarbeitet wird.

b) Durch Alkylierung von 2-n-Butyl-4-n-propyl-6-hydroxypyrimidin mit 4-Brommethyl-2\-cyanobiphenyl in der in Beispiel 1a) beschriebenen Weise und Flash-Chromatographie (Kieselgel 60, 40-63 µm, Hexan/Essigester = 4:1) erhält man 2-n-Butyl-1,6-dihydro-4-isopropyl-1-[(2′-cyanobiphenyl-4-yl)-methyl]-6-oxopyrimidin, welches direkt weiterverarbeitet wird.

Beispiel 21:

Ausgehend von 2-n-Butyl-1,6-dihydro-4-methyl-5-n-propyl-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-methyl-5-n-propyl-1-[2′-(1H-tetrazol-5-yl)biphenyl-4-yl)methyl]-6-oxopyrimidin als amorphen Festkörper.

Das Ausgangsmaterial kann beispielsweise wie folgt hergestellt werden:

a) Durch Umsetzung von 2-n-Propyl-acetessigsäure-ethylester mit n-Valeroylamidin.HCl und Natrium in absolutem Ethanol in der in Beispiel 13a) beschriebenen Weise erhält man 2-n-Butyl-4-methyl-5-n-propyl-6-hydroxypyrimidin, welches direkt weiterverarbeitet wird.

b) Durch Alkylierung von 2-n-Butyl-4-methyl-5-n-propyl-6-hydroxypyrimidin mit 4-Brommethyl-2′-cyanobiphenyl in der in Beispiel 1a) beschriebenen Weise und Flash-Chromatographie (Kieselgel 60, 40-63 µm, Hexan/Essigester = 4:1) erhält man 2-n-Butyl-1,6-dihydro-4-methyl-5-n-propyl-1-[(2′-cyanobiphenyl-4-yl)-methyl]-6-oxopyrimidin, welches direkt weiterverarbeitet wird.

Beispiel 22:

Ausgehend von 2-n-Butyl-1,6-dihydro-5-ethoxycarbonyl-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-5-ethoxycarbonyl-1-[2′-(1H-tetrazol-5-yl)biphenyl-4-yl)methyl]-6-oxopyrimidin. Smp. 175-177 °C (aus Essigester).

Das Ausgangsmaterial kann beispielsweise wie folgt hergestellt werden:

a) Durch Umsetzung von Ethoxymethylen-malonsäure-diethylester mit n-Valeroylamidin.HCl und Natrium in absolutem Ethanol in der in Beispiel 13a) beschriebenen Weise erhält man 2-n-Butyl-5-ethoxycarbonyl-6-hydroxypyrimidin, welches direkt weiterverarbeitet wird.

b) Durch Alkylierung von 2-n-Butyl-5-ethoxycarbonyl-6-hydroxypyrimidin mit 4-Brommethyl-2′-cyanobiphenyl in der in Beispiel 1a) beschriebenen Weise und Flash-Chromatographie (Kieselgel 60, 40-63 um, Hexan/Essigester = 3:1) erhält man 2-n-Butyl-1,6-dihydro-5-ethoxycarbonyl-1-[(2′-cyanobiphenyl-4-yl)-methyl]-6-oxopyrimidin, welches direkt weiterverarbeitet wird.

Beispiel 23:

Zu einer Lösung von 245 mg (0,534 mmol) 2-n-Butyl-1,6-dihydro-5-ethoxycarbonyl-1-[2'-(1H-tetrazol-5-yl)biphenyl-4-yl)methyl]-6-oxopyrimidin in 8 ml Ethanol werden 1,1 ml 1H NaOH zugegeben. Das Reaktionsgemisch wird während 2 Stunden bei Raumtemperatur gerührt und anschliessend im Vakuum eingedampft. Nach Zugabe von $H_2O$ wird mit 1N HCl angesäuert (pH = 3) und mit $CH_2Cl_2$ exträhiert. Die organischen Phasen werden getrocknet ($Na_2SO_4$) und im Vakuum eingedampft. Flash-Chromatographie (Kieselgel 60, 40-63 µm, $CH_2Cl_2/CH_3OH/NH_3$ = 5:3:1) ergibt 2-n-Butyl-5-carboxy-1,6-dihydro-1-[2'-(1H-tetrazol-5-yl)-biphenyl-4-yl)methyl]-6-oxopyrimidin als amorphen Festkörper.

Beispiel 24:

Ausgehend von 2-n-Butyl-1,6-dihydro-4-methoxymethyl-1-[(2'-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-methoxymethyl-1-[2'-(1H-tetrazol-5-yl)biphenyl-4-yl)methyl]-6-oxopyrimidin. Smp. 193°C (aus Essigester)

Das Ausgangsmaterial kann beispielsweise wie folgt hergestellt werden:

a) Durch Umsetzung von 2-Methoxy-acetessigsäure-methylester mit n-Valeroylamidin.HCl und Natrium in absolutem Ethanol in der in Beispiel 13a) beschriebenen Weise erhält man 2-n-Butyl-4-methoxymethyl-6-hydroxypyrimidin, welches direkt weiterverarbeitet wird.

b) Durch Alkylierung von 2-n-Butyl-4-methoxymethyl-6-hydroxypyrimidin mit 4-Brommethyl-2'-cyanobiphenyl in der in Beispiel 1a) beschriebenen Weise und Flash-Chromatographie (Kieselgel 60, 40-63 µm, Hexan/Essigester = 1:1) erhält man 2-n-Butyl-1,6-dihydro-4-methoxymethyl-1-[(2'-cyanobiphenyl-4-yl)-methyl]-6-oxopyrimidin, welches direkt weiterverarbeitet wird.

Beispiel 25:

Ausgehend von 2-n-Butyl-1,6-dihydro-5-hydroxymethyl-1-[(2'-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-5-hydroxymethyl-1-[2'-(1H-tetrazol-5-yl)biphenyl-4-yl)methyl]-6-oxopyrimidin. Smp. 147°C (aus Essigester).

Das Ausgangsmaterial kann beispielsweise wie folgt hergestellt werden:

a) Zu einer Suspension von 440 mg (11,6 mmol) Lithiumaluminiumhydrid in 20 ml absolutem Tetrahydrofuran wird bei Raumtemperatur eine Lösung von 2,6 g (11,6 mmol) 2-n-Butyl-5-ethoxycarbonyl-6-hydroxypyrimidin in 50 ml absolutem Tetrahydrofuran zugetropft. Nach beendeter Zugabe wird 1 Stunden bei Raumtemperatur gerührt und anschliessend durch Zudosieren von 50 ml $H_2O$ hydrolysiert. Nach Ansäuern mit Essigsäure (pH = 4) wird mit Essigester extrahiert. Die organischen Phasen werden getrocknet ($Na_2SO_4$) und im Vakuum eingedampft. Nach Zugabe von Diethylether werden die Kristalle abfiltriert und im Vakuum getrocknet. Man erhält so 2-n-Butyl-5-hydroxymethyl-6-hydroxypyrimidin. Smp. 107-109°C.

b) Zu einer Lösung von 9,0 g (49,4 mmol) 2-n-Butyl-5-hydroxymethyl-6-hydroxypyrimidin in 200 ml N,N-Dimethylformamid werden 13,6 g (49,4 mmol) Kaliumcarbonat zugegeben. Man rührt während 20 Minuten bei Raumtemperatur und gibt anschliessend portionenweise 13,4 g (49,4 mmol) 4-Brommethyl-2'-cyanobiphenyl zu. Nach beendeter Zugabe wird während 12 Stunden bei Raumtemperatur gerührt und anschliessend im Vakuum eingedampft. Der Rückstand wird mit Essigester versetzt, mit $H_2O$ gewaschen, getrocknet ($Na_2SO_4$) und im Vakuum eingedampft. Flash-Chromatographie (Kieselgel 60, 40-63 um, Hexan/Essigester = 1:1) ergibt 2-n-Butyl-5-hydroxymethyl-1-[(2'-cyanobiphenyl-4-yl)-methyl]-6-oxopyrimidin. Smp. 96-98°C.

Beispiel 26:

Durch Umsetzung von 2-n-Butyl-1,6-dihydro-4-ethoxycarbonyl-1-[(2'-cyano biphenyl-4-yl)methyl]-6-oxopyrimidin und Tributylzinnazid erhält man beispielsweise in der in Beispiel 1 beschriebenen Weise 2-n-Butyl-1,6-dihydro-4-ethoxycarbonyl-1-[2'-(1H-tetrazol-5-yl)biphenyl-4-yl)methyl]-6-oxopyrimidin als amorphen Festkörper.

Das Ausgangsmaterial kann beispielsweise wie folgt hergestellt werden:

a) Durch Umsetzung des Natriumsalzes von Oxalessigsäure-diethylester mit n-Valeroylamidin.HCl und

Natrium in absolutem Ethanol in der in Beispiel 13a) beschriebenen Weise erhält man 2-n-Butyl-4-ethoxycarbonyl-6-hydroxypyrimidin. Smp. 127- 128 °C (aus Diethylether/Hexan).

b) Durch Alkylierung von 2-n-Butyl-4-ethoxycarbonyl-6-hydroxypyrimidin mit 4-Brommethyl-2′-cyanobiphenyl in der in Beispiel 1a) beschriebenen Weise und Flash-Chromatographie (Kieselgel 60, 40-63 µm, Hexan/Essigester = 1:1) erhält man 2-n-Butyl-1,6-dihydro-4-ethoxycarbonyl-1-[(2′-cyanobiphenyl-4-yl)-methyl]-6-oxopyrimidin, welches direkt weiterverarbeitet wird.

Beispiel 27:

Durch Hydrolyse von 2-n-Butyl-1,6-dihydro-4-ethoxycarbonyl-1-[2′-(1H-tetrazol-5-yl)biphenyl-4-yl)-methyl]-6-oxopyrimidin in der in Beispiel 23 beschriebenen Weise erhält man 2-n-Butyl-1,6-dihydro-4-carboxy-1-[2′-(1H-tetrazol-5-yl)biphenyl-4-yl)methyl]-6-oxopyrimidin. Smp. 160-162 °C (aus Essigsäure/$H_2O$).

Beispiel 28:

Eine Lösung von 400 mg (0,81 mmol) 2-n-Butyl-4-benzyloxy-1,6-dihyro-1-[2′-(1H-tetrazol-5-yl)biphenyl-4-yl)methyl]-6-oxopyrimidin in 75 ml Methanol/$CH_2Cl_2$ (1:1) wird in Gegenwart von 100 mg 5 % Pd-C bei Raumtemperatur und 1 atm. $H_2$ geschüttelt. Nach beendigter Wasserstoffaufnahme wird der Katalysator abfiltriert, mit Methanol gewaschen und das Filtrat im Vakuum eingedampft. Man erhält so 2-n-Butyl-1,6-dihydro-4-hydroxy-1-[2′-(1H-tetrazol-5-yl)biphenyl-4-yl)methyl]-6-oxopyrimidin. Smp. 153 °C (aus Isopropanol/Essigester).

Beispiel 29:

Durch Umsetzung von 2-n-Butyl-1,6-dihydro-4-hydroxy-5-methyl-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-4-hydroxy-5-methyl-1-[2′-(1H-tetrazol-5-yl)biphenyl-4-yl)methyl]-6-oxopyrimidin. Smp. 221-223 °C (aus Acetonitril).

Das Ausgangsmaterial kann beispielsweise wie folgt hergestellt werden.

a) Eine Mischung von 28,1 g (0,2 mol) n-Valeroylamidin.HCl und 42,8 g (0,2 mol) 4-Aminomethyl-2′-cyanobiphenyl (EP 253310) in 50 ml EtOH wird während 12 Stunden unter Rückfluss gerührt. Nach dem Eindampfen im Vakuum und Flash-Chromatographie (Kieselgel 60, 40-63 µm, $CH_2Cl_2/CH_3OH$ = 4:1) erhält man N-[(2′-Cyanobiphenyl-4-yl)methyl]-n-valeroylamidin.HCl als amorphen Festkörper, welcher direkt weiterverarbeitet wird.

b) Zu einer Lösung von 280 mg (12 mmol) Natrium in 5 ml absolutem Ethanol wird bei Raumtemperatur eine Lösung von 1,97 g (6 mmol) N-[(2′-Cyanobiphenyl-4-yl)methyl]-n-valeroylamidin.HCl in 5 ml absolutem Ethanol zugetropft. Nach 10 Minuten werden 1,05 ml (6 mmol) Methylmalonsäure-diethylester zugegeben und die Suspension während 24 Stunden unter Rückfluss gerührt. Das Reaktionsgemisch wird im Vakuum eingedampft, mit $H_2O$ versetzt und mit 2N HCl angesäuert (pH = 3). Man extrahiert mit Essigester, wäscht mit $H_2O$, trocknet ($Na_2SO_4$) und dampft im Vakuum ein. Flash-Chromatographie (Kieselgel 60, 40-63 µm, $CH_2Cl_2/CH_3OH$ = 95:5) ergibt 2-n-Butyl-1,6-dihydro-4-hydroxy-5-methyl-1-[(2′-cyanobiphenyl-4-yl)methyl]-6-oxopyrimidin. Smp. 204-206 °C (aus Diethylether).

Beispiel 30:

Ausgehend von 2-n-Butyl-1,6-dihydro-5-ethyl-4-hydroxy-1-[(2′-cyanobiphenyl-4-yl)methyl]-6-oxopyrimidin und Tributylzinnazid erhält man beispielsweise in der in Beispiel 1 beschriebenen Weise 2-n-Butyl-1,6-dihydro-5-ethyl-4-hydroxy-1-[2′-(1H-tetrazol-5-yl)biphenyl-4-yl)methyl]-6-oxopyrimidin. Smp. 248-250 °C (aus Ethanol/$H_2O$).

Das Ausgangsmaterial kann beispielsweise wie folgt hergestellt werden:

a) Durch Umsetzung von Ethylmalonsäure-diethylester mit N-[(2′-Cyanobiphenyl-4-yl)methyl]-n-valeroylamidin.HCl und Natrium in absolutem EtOH in der in Beispiel 29b) beschriebenen Weise erhält man 2-n-Butyl-1,6-dihydro-5-ethyl-4-hydroxy-1-[(2′-cyanobiphenyl-4-yl)-methyl]-6-oxopyrimidin. Smp. 157-158 °C

(aus Isopropanol).

Beispiel 31:

Ausgehend von 2,5-Di-n-butyl-1,6-dihydro-4-hydroxy-1-[(2′-cyanobiphenyl-4-yl)methyl]-6-oxopyrimidin ued Tributylzinnazid erhält man beispielsweise in der in Beispiel 1 beschriebenen Weise 2,5-Di-n-butyl-1,6-dihydro-4-hydroxy-1-[2′-(1H-tetrazol-5-yl)biphenyl-4-yl)methyl]-6-oxopyrimidin. Smp. 245-247° C (aus Essigsäure/$H_2O$).

Das Ausgangsmaterial kann beispielsweise wie folgt hergestellt werden:

a) Durch Umsetzung von n-Butylmalonsäure-diethylester mit N-[(2′Cyanobiphenyl-4-yl)methyl]-n-valeroylamidin.HCl und Natrium in absolutem Ethanol in der in Beispiel 29b) beschriebenen Weise erhält man 2,5-Di-n-butyl-1,6-dihydro-4-hydroxy-1-[(2′-cyanobiphenyl-4-yl)methyl]-6-oxopyrimidin, welches direkt weiterverarbeitet wird.

Beispiel 32:

Eine Lösung von 1,96 g (5,02 mmol) 2-n-Butyl-1,6-dihydro-4-methyl-1-[(2′-methoxycarbonylbiphenyl-4-yl)methyl]-6-oxopyrimidin in 15 ml Methanol und 5 ml 2N NaOH wird während 12 Stunden bei 50° C gerührt. Nach dem Eindampfen des Methanols im Vakuum wird mit 2N HCl angesäuert (pH = 1) und mit $CH_2Cl_2$ exträhiert. Die organische Phase wird getrocknet ($Na_2SO_4$) und eingedampft. Man erhält so 2-n-Butyl-1,6-dihydro-4-methyl-1-[(2′-carboxybiphenyl-4-yl)-methyl]-6-oxo-pyrimidin. Smp. 190° - 192° C (aus $CH_2Cl_2$/Hexan).

Das Ausgangsmaterial kann beispielsweise wie folgt hergestellt werden:

Durch Alkylierung von 2-n-Butyl-4-hydroxy-6-methylpyrimidin-biphenyl mit 4-Brommethyl-2′-methoxycarbonylbiphenyl (EP 253310) in der in Beispiel 1a) beschriebenen Weise und Flash-Chromatographie (Kieselgel 60, 40-63 µm, Hexan/Essigester = 1:1) erhält man 2-n-Butyl-1,6-dihydro-4-methyl-1-[(2′-methoxycarbonylbiphenyl-4-yl)-methyl]-6-oxopyrimidin, welches direkt weiterverarbeitet wird.

Beispiel 33:

Ausgehend von 2-n-Butyl-3-[(2′-cyanobiphenyl-4yl)methyl]-3,5,6,7-tetrahydro-4H-cyclopentapyrimidin-4-on und Tributylzinnazid erhält man beispielsweise in der in Beispiel 1 beschriebenen Weise 2-n-Butyl-3-[-(2′-(1H-tetrazol-5-yl)biphenyl-4-yl)methyl]-3,5,6,7-tetrahydro-4H-cyclopentapyrimidin-4-on. Smp. 214-216°C (Aus Acetonitril)

Das Ausgangsmaterial kann beispielsweise wie folgt hergestellt werden:

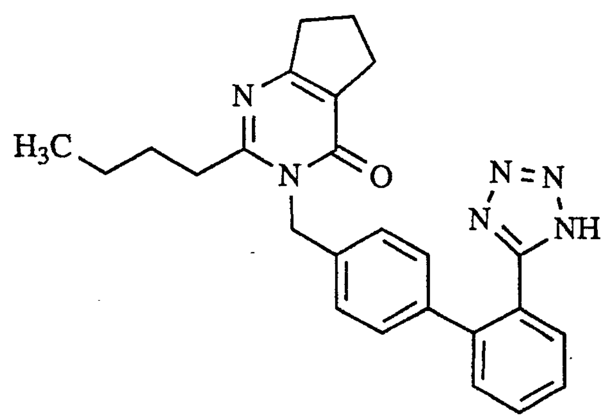

a) Durch Umsetzung von Cyclopentanon-2-carbonsäure-ethylester mit n-Valeroylamidin.HCl und Natrium in absolutem Ethanol in der in Beispiel 13a) beschriebenen Weise erhält man 2-n-Butyl-3,5,6,7-tetrahydro-4H-cyclopentapyrimidin-4-on, welches direkt weiterverarbeitet wird.

b) Durch Alkylierung von 2-n-Butyl-3,5,6,7-tetrahydro-4H-cyclopentapyrimidin-4-on mit 4-Brommethyl-2′-

cyanobiphenyl in der in Beispiel 1a) beschriebenen Weise und Flash-Chromatographie (Kieselgel 60, 40-63 µm,Hexan/Essigester = 4:1) erhält man 2-n-Butyl-3-[(2′-cyanobiphenyl-4yl)methyl]-3,5,6,7-tetrahydro-4H-cyclopentapyrimidin-4-on, welches direkt weiterverarbeitet wird.

Beispiel 34:

Ausgehend von 2-n-Butyl-3-[(2′-cyanobiphenyl-4yl)methyl]-5,6,7,8-tetrahydro-3H-quinazolin-4-on und Triburylzinnazid erhält man beispielsweise in der in Beispiel 1 beschriebenen Weise 2-n-Butyl-3-[(2′-(1H-tetrazol-5-yl)biphenyl-4-yl)methyl]-5,6,7,8-tetrahydro-3H-quinazolin-4-on. Smp. 240 ° C (Aus Acetonitril).

Das Ausgangsmaterial kann beispielsweise wie folgt hergestellt werden:

a) Durch Umsetzung von Cyclohexanon-2-carbonsäure-ethylester mit n-Valeroylamidin.HCl und Natrium in absolutem Ethanol in der in Beispiel 13a) beschriebenen Weise erhält man 2-n-Butyl-5,6,7,8-tetrahydro-3H-quinazolin-4-on, welches direkt weiterverarbeitet wird.

b) Durch Alkylierung von 2-n-Butyl-5,6,7,8-tetrahydro-3H-quinazolin-4-on mit 4-Brommethyl-2′-cyanobiphenyl in der in Beispiel 1a) beschriebenen Weise und Flash-Chromatographie (Kieselgel 60,40-63 µm, Hexan/Essigester = 2:1) erhält man 2-n-Butyl-3-[(2′-cyanobiphenyl-4-yl)methyl]-5,6,7,8-tetrahydro-3H-quinazolin-4-on, welches direkt weiterverarbeitet wird.

Beispiel 35:

Ausgehend von 2-n-Butyl-3-[(2′-cyanobiphenyl-4-yl)methyl]-3H-quinazolin-4-on und Tributylzinnazid erhält man beispielsweise in der in Beispiel 1 beschriebenen Weise 2-n-Butyl-3-[(2′-(1H-tetrazol-5-yl)biphenyl-4-yl)methyl]-3H-quinazolin-4-on. Smp. 179-180 ° C (Aus Essigester).

Das Ausgangsmaterial kann beispielsweise wie folgt herge stellt werden:

a) Eine Lösung von 5.0 g (36.6 mmol) n-Valeroylamidin.HCl und 5.97 g (36.6 mmol) Isatosäureanhydrid in 200 ml Pyridin wird während 24 Stunden bei 100 °C gerührt. Nach dem Eindampfen im Vakuum wird der Rückstand in $H_2O$ suspendiert, abfiltriert und im Vakuum bei 50 °C getrocknet. Man erhält auf diese Weise 2-n-Butyl-3H-quinazolin-4-on,welches direkt weiterverarbeitet wird.

b) Durch Alkylierung von 2-n-Butyl-3H-quinazolin-4-on mit 4-Brommethyl-2′-cyanobiphenyl in der in Beispiel 1a) beschriebenen Weise und Flash-Chromatographie (Kieselgel 60, 40-63 µm, Hexan/Essigester = 2:1) erhält man 2-n-Butyl-3-[(2′-cyanobiphenyl-4-yl)methyl]-3H-quinazolin-4-on, welches direkt weiterverarbeitet wird.

Beispiel 36:

Ausgehend von 2-n-Butyl-3-[(2′-cyanobiphenyl-4yl)methyl]-3H-pyrido-[2,3-d]pyrimidin-4-on und Tributylzinnazid erhält man beispielsweise in der in Beispiel 1 beschriebenen Weise 2-n-Butyl-3-[(2′-(1H-tetrazol-5-yl)biphenyl-4-yl)methyl]-3H-pyrido[2,3-d]pyrimidin-4-on. Smp.135-137 ° C (Aus Methanol/Essigester).

Das Ausgangsmaterial kann beispielsweise wie folgt hergestellt werden:

a) Eine Lösung von 5.0 g (36.6 mmol) n-Valeroylamidin.HCl und 6.0 g (36.6 mmol) 2H-Pyrido[2,3-d][1,3]-oxazin-2,4(1H)-dion in 200 ml Pyridin wird während 24 Stunden bei 100°C gerührt. Nach dem Eindampfen im Vakuum wird der Rückstand mit Essigester versetzt, mit $H_2O$ gewaschen, getrocknet ($Na_2SO_4$) und im Vakuum eingedampft. Flash-Chromatagraphie (Kieselgel 60, 40-63µm, $CH_2Cl_2/CH_3OH$ = 98:2) ergibt 2-n-Butyl-3H-pyrido[2,3-d]pyrimidin-4-on, welches direkt weiterverarbeitet wird.

b) Durch Alkylierung von 2-n-Butyl-3H-pyrido[2,3-d]pyrimidin4-on mit 4-Brommethyl- 2′-cyanobiphenyl in der in Beispiel 1a) beschriebenen Weise und Flash-Chromatographie (Kieselgel 60, 40-63 µm, $CH_2Cl_2/CH_3OH$ = 98:2) erhält man 2-n-Butyl-3-[(2′cyanobiphenyl-4-yl)methyl]-3H-pyrido[2,3-d]pyrimidin-4-on, welches direkt weiterverarbeitet wird.

Beispiel 37:

Ausgehend von 2-n-Butyl-3-[(2′-cyanobiphenyl-4yl)methyl]-3H-pyrido-[3,2-d]pyrimidin-4-on und Tributylzinnazid erhält man beispielsweise in der in Beispiel 1 beschriebenen Weise 2-n-Butyl-3-[(2′-(1H-tetrazol-5-yl)biphenyl-4-yl)methyl]-3H-pyrido[3,2-d]pyrimidin-4-on. Smp.245 ° C (Aus Essigester)

Das Ausgangsmaterial kann beispielsweise wie folgt hergestellt werden:

a) Eine Lösung von 5.0 g (36.6 mmol) n-Valeroylamidin.HCl und 6.0 g (36.6 mmol) 2H-Pyrido[3,2-d][1,3]-oxazin-2,4(1H)-dion in 200 ml Pyridin wird während 24 Stunden bei 100 °C gerührt. Nach dem Eindampfen im Vakuum wird der Rückstand mit Essigester versetzt, mit $H_2O$ gewaschen, getrocknet ($Na_2SO_4$) und im Vakuum eingedampft. Flash-Chromatographie (Kieselgel 60, 40-63 µm, $CH_2Cl_2/CH_3OH$ = 98:2) ergibt 2-n-Butyl-3H-pyrido[3,2-d]pyrimidin-4-on, welches direkt weiterverarbeitet wird.

b) Durch Alkylierung von 2-n-Butyl-3H-pyrido[3,2-d]pyrimidin-4-on mit 4-Brommethyl-2′-cyanobiphenyl in der in Beispiel 1a) beschriebenen Weise und Flash-Chromatographie (Kieselgel 60, 40-63 µm, $CH_2Cl_2/CH_3OH$ = 98:2) erhält man 2-n-Butyl-3-[(2′cyanobiphenyl-4-yl)methyl]-3H-pyrido[3,2-d]pyrimidin-4-on, welches direkt weiterverarbeitet wird.

Beispiel 38:

In analoger Weise, z.B. wie einem der vorstehenden Beispiele 1 bis 5 beschrieben, kann man herstellen:

2-n-Butyl-1,6-dihydro-4-ethyl-1-[(2′-(1H-tetrazol-5-yl)biphenyl-4-yl)methyl]-6-oxopyrimidin,
2-n-Butyl-1,6-dihydro-4-propyl- 1-[(2′-(1H-tetrazol-5-yl)biphenyl-4-yl)methyl]-6-oxopyrimidin,
2-n-Butyl-1,6-dihydro-4-isopropyl-1-[(2′-(1H-tetrazol-5-yl)biphenyl-4-yl)methyl]-6-oxopyrimidin,
2,4-Di-n-butyl-1,6-dihydro-1-[(2′-(1H-tetrazol-5-yl)biphenyl-4-yl)methyl]-6-oxopyrimidin,
2-n-Butyl-4-t-butyl-1,6-dihydro-1-[(2′-(1H-tetrazol-5-yl)biphenyl-4-yl)methyl]-6-oxopyrimidin,
2-n-Butyl-1,6-dihydro-4-trifluormethyl- 1-[(2′-(1H-tetrazol-5-yl)biphenyl-4-yl)methyl]-6-oxopyrimidin,
2-n-Butyl-1,6-dihydro-4-phenyl-1-[(2′-(1H-tetrazol-5-yl)biphenyl-4-yl)methyl]-6-oxopyrimidin,
2-n-Butyl-1,6-dihydro-4-ethoxycarbonyl-1- [(2′-(1H-tetrazol-5-yl)biphenyl-4-yl)methyl]-6-oxopyrimidin,
2-n-Butyl-4-carboxy-1,6-dihydro-1-[(2′-(1H-tetrazol-5-yl)biphenyl-4-yl)methyl]-6-oxopyrimidin,
2-n-Butyl-1,6-dihydro-4-hydroxymethyl-1- [(2′-(1H-tetrazol-5-yl)biphenyl-4-yl)methyl]-6-oxopyrimidin,
2-n-Butyl-1,6-dihydro-4-methoxymethyl-1-[(2′-(1H-tetrazol-5-yl)biphenyl-4-yl)methyl]-6-oxopyrimidin,
2-n-Butyl-1,6-dihydro-4-dimethylaminomethyl-1-[(2′-(1H-tetrazol-5-yl)biphenyl-4-yl)methyl] -6-oxopyrimidin,
2-n-Butyl-1,6-dihydro-3-ethoxycarbonylmethyl-1-[(2′-(1H-tetrazol-5-yl)biphenyl-4-yl)methyl]-6-oxopyrimidin,
2-n-Butyl-4-carboxymethyl-1,6-dihydro-1-[(2′-(1H-tetrazol-5-yl)biphenyl-4-yl)methyl]-6-oxopyrimidin,
2-n-Butyl-1,6-dihydro-4-(2-hydroxyethyl)-1-[(2′-(1H-tetrazol-5-yl)biphenyl-4-yl)methyl]-6-oxopyrimidin,
2-n-Butyl-1,6-dihydro-4-(2-methoxyethyl)-1- [(2′-(1H-tetrazol-5-yl)biphenyl-4-yl)methyl] -6-oxopyrimidin,
2-n-Butyl-1,6-dihydro-4-(2-dimethylaminoethyl)-1-[(2′-(1H-tetrazol-5-yl)biphenyl-4-yl)methyl]-6-oxopyrimidin,
2-n-Butyl-1,6-dihydro-4-hydroxy-1-[(2′-(1H-tetrazol-5-yl)biphenyl-4-yl)methyl]-6-oxopyrimidin,
2-n-Butyl-1,6-dihydro-4-methylthio- 1 · -[(2′-(1H-tetrazol-5-yl)biphenyl-4-yl)methyl]-6-oxopyrimidin, 2-n-Butyl-1,6-dihydro-4-methylsulfonyl-1-[(2′-(1H-tetrazol-5-yl)biphenyl-4-yl)methyl]-6-oxopyrimidin,
2-n-Butyl-1,6-dihydro-4-morpholino-1-[(2′-(1H-tetrazol-5-yl)biphenyl-4-yl)methyl]-6-oxopyrimidin,
2-n-Butyl-1,6-dihydro-4-(2-methoxyethoxy)-1-[(2′-(1H-tetrazol-5-yl)biphenyl-4-yl)methyl]-6-oxopyrimidin,
2-n-Butyl-1,6-dihydro-4-(2-dimethylaminoethoxy)-1-[(2′-(1H-tetrazol-5-yl)biphenyl-4-yl)methyl]-6-oxopyrimidin,
2-n-Butyl-1,6-dihydro-5-methoxy-1-[(2′-(1H-tetrazol-5-yl)biphenyl-4-yl)methyl]-6-oxopyrimidin,
2-n-Butyl-1,6-dihydro-5-ethoxycarbonyl-1-[(2′-(1H-tetrazol-5-yl)biphenyl-4-yl)methyl]-6-oxopyrimidin,
2-n-Butyl-5-carboxy-1,6-dihydro-1-[(2′-(1H-tetrazol-5-yl)biphenyl-4-yl)methyl]-6-oxopyrimidin,
2-n-Butyl-1,6-dihydro-5-hydroxymethyl-1-[(2′-(1H-tetrazol-5-yl)biphenyl-4-yl)methyl]-6-oxopyrimidin,
2-n-Butyl-1,6-dihydro-5-methoxymethyl-1-[(2′-(1H-tetrazol-5-yl)biphenyl-4-yl)methyl]-6-oxopyrimidin,
2-n-Butyl-1,6-dihydro-5-dimethylaminomethyl-1-[(2′-(1H-tetrazol-5-yl)biphenyl-4-yl)methyl]-6-oxopyrimidin,
2-n-Butyl-1,6-dihydro-5-methyl-1-[(2′-(1H-tetrazol-5-yl)biphenyl-4-yl)methyl]-6-oxopyrimidin,
2-n-Butyl-1,6-dihydro-4,5-dimethyl-1-[(2′-(1H-tetrazol-5-yl)biphenyl-4-yl)methyl]-6-oxopyrimidin,
2-n-Butyl-1,6-dihydro-5-ethyl-4-methyl-1-[(2′-(1H-tetrazol-5-yl)biphenyl-4-yl)methyl]-6-oxopyrimidin,
2-n-Butyl-1,6-dihydro-4-methyl-5-propyl-1-[(2′-(1H-tetrazol-5-yl)biphenyl-4-yl)methyl]-6-oxopyrimidin,
2-n-Butyl-1,6-dihydro-5-isopropyl-4-methyl-1-[(2′-(1H-tetrazol-5-yl)biphenyl-4-yl)methyl]-6-oxopyrimidin,
2,5-Di-n-butyl-1,6-dihydro-4-methyl-1-[(2′-(1H-tetrazol-5-yl)biphenyl-4-yl)methyl]-6-oxopyrimidin,
2-n-Butyl-1,6-dihydro-5-methyl-4-phenyl-1-[(2′-(1H-tetrazol-5-yl)biphenyl-4-yl)methyl]-6-oxopyrimidin,
2-n-Butyl-1,6-dihydro-5-hydroxyethyl-4-methyl-1-[(2′-(1H-tetrazol-5-yl)biphenyl-4-yl)methyl]-6-oxopyrimidin,
2-n-Butyl-1,6-dihydro-5-methoxyethyl-4-methyl-1-[(2′-(1H-tetrazol-5-yl)biphenyl-4-yl)methyl]-6-oxopyrimidin,
2-n-Butyl-1,6-dihydro-5-dimethylaminoethyl-4-methyl-1-[(2′-(1H-tetrazol-5-yl)biphenyl-4-yl)methyl]-6-oxopyrimidin,
2-n-Butyl-1,6-dihydro-5-ethoxycarbonylmethyl-4-methyl-1-[(2′-(1H-tetrazol-5-yl)biphenyl-4-yl)methyl]-6-

oxopyrimidin,
2-n-Butyl-5-carboxymethyl-1,6-dihydro-4-methyl-1-[(2'-(1H-tetrazol-5-yl)biphenyl-4-yl)methyl]-6-oxopyrimidin,
2-n-Propyl-1,6-dihydro-4-methyl-1-[(2'-(1H-tetrazol-5-yl)biphenyl-4-yl)methyl]-6-oxopyrimidin,
2-n-Pentyl-1,6-dihydro-4-methyl-1-[(2'-(1H-tetrazol-5-yl)biphenyl-4-yl)methyl]-6-oxopyrimidin.

Beispiel 39:

Tabletten, enthaltend je 50 mg des Wirkstoffs, z.B. 2-n-Butyl-4-chlor-1,6-dihydro-1-[(2'-(1H-tetrazol-5-yl)-biphenyl-4-yl)-methyl]-6-oxopyrimidin, können wie folgt hergestellt werden.

| Zusammensetzung (10000 Tabletten) | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdisper.) | 20,0 g |
| Ethanol | q.s. |

Der Wirkstoff wird mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 40:

Lacktabletten, enthaltend je 100 mg des Wirkstoff, z.B. 2-n-Butyl-1,6-dihydro-1-[(2'-(1H-tetrazol-5-yl)-biphenyl-4-yl)-methyl]-6-oxopyrimidin, können wie folgt hergestellt werden:

| Zusammensetzung (10000 Tabletten) | |
|---|---|
| Wirkstoff | 100,00 g |
| Lactose | 100,00 g |
| Maisstärke | 70,00 g |
| Talk | 8,50 g |
| Calciumstearat | 1,50 g |
| Hydroxypropyl-methylcellulose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen) befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 mg.

Beispiel 41:

In analoger Weise wie in den Beispielen 39 und 40 beschrieben können auch Tabletten bzw. Lacktabletten, enthaltend eine erfindungsgemässe Verbindung, z.B. gemäss der Beispiele 1-38, hergestellt werden.

**Ansprüche**

1. Verbindungen der Formel

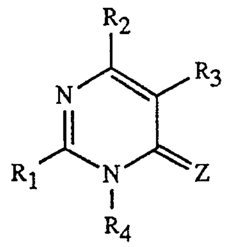

(I),

ihre Tautomere und Salze, worin
Z für O, S oder N(R) und R für Wasserstoff oder einen aliphatischen Kohlenwasserstoffrest steht;
$R_1$ einen gegebenenfalls substituierten aliphatischen Kohlenwasserstoffrest, einen cycloaliphatischen oder araliphatischen Kohlenwasserstoffrest oder einen aromatischen Rest bedeutet;
$R_2$ und $R_3$ unabhängig voneinander Halogen, Acyl, einen aromatischen Rest, gegebenenfalls substituiertes Amino, gegebenenfalls verestertes oder amidiertes Carboxy bedeuten; oder
$R_2$ für $-Z_1-R'_2$ und $R_3$ für $-Z_2-R'_3$ stehen, wobei $Z_1$ und $Z_2$ unabhängig voneinander für eine Bindung, O oder $S(O)_n$ stehen und n für 0, 1 oder 2 steht, und $R'_2$ und $R'_3$ unabhängig voneinander für Wasserstoff, einen araliphatischen oder aliphatischen Kohlenwasserstoffrest stehen, wobei letzterer gegebenenfalls substituiert und gegebenenfalls durch -O-oder $-S(O)_n-$ unterbrochen ist, wobei n für 0, 1 oder 2 steht; oder
$R_2$ und $R_3$ gemeinsam für Propylen oder Butylen oder für die Partialstruktur der Formel -CH=CH-CH=CH-, in der gegebenenfalls eine oder zwei der Methingruppen durch -N= ersetzt ist;
$R_4$ für eine Gruppe der Formel

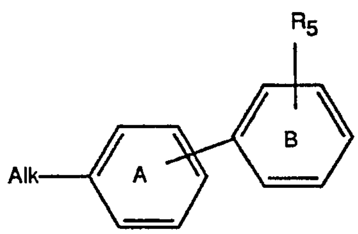

(Ia)

steht, in der
Alk einen zweiwertigen aliphatischen Kohlenwasserstoff bedeutet;
$R_5$ COOH, $SO_3H$, Halogenalkansulfamoyl, $PO_2H_2$, $PO_3H_2$ oder 5-Tetrazolyl bedeutet;
die Ringe A und B bzw. der durch $R_2$ und $R_3$ gemeinsam gebildete (hetero-)aromatische Ring unabhängig voneinander gegebenenfalls substituiert sind.

2. Verbindungen gemäss Anspruch 1 der Formel I, ihre Tautomere und Salze, worin Z für O, S oder N(R) und R für Wasserstoff oder einen aliphatischen Kohlenwasserstoffrest steht,
$R_1$ einen gegebenenfalls durch Halogen oder Hydroxy substituierten aliphatischen Rest, einen cycloaliphatischen oder araliphatischen Kohlenwasserstoffrest oder einen aromatischen Rest bedeutet,
$R_2$ und $R_3$ unabhängig voneinander Halogen, Acyl, einen aromatischen Rest, gegebenenfalls verestertes oder amidiertes Carboxy bedeuten, oder
$R_2$ für $-Z_1-R'_2$ und $R_3$ für $-Z_2-R'_3$ stehen, wobei $Z_1$ und $Z_2$ unabhängig voneinander für eine Bindung stehen oder O, $S(O)_n$ oder NH bedeuten, n für 0, 1 oder 2 steht und $R'_2$ und $R'_3$ unabhängig voneinander für Wasserstoff oder einen aliphatischen Kohlenwasserstoffrest stehen, der gegebenenfalls durch Halogen,

Hydroxy, gegebenenfalls substituiertes Amino, gegebenenfalls verestertes oder amidiertes Carboxy substituiert ist und der gegebenenfalls durch -O- oder $S(O)_n$- unterbrochen ist, wobei n für 0, 1 oder 2 steht, $R_4$ für eine Gruppe der Formel

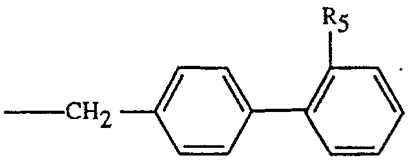

(Ib)

steht, in der

$R_5$ COOH, $SO_3H$, Halogenalkansulfamoyl, $PO_2H_2$, $PO_3H_2$ oder 5-Tetrazolyl bedeutet.

3. Verbindung gemäss Anspruch 1 der Formel I, ihre Tautomere und Salze, worin Z für O, S oder N(R) steht und R Wasserstoff, Niederalkyl, Niederalkenyl oder Niederalkinyl bedeutet;

$R_1$ Niederalkyl, Niederalkenyl bzw. Niederalkinyl bedeutet, welche jeweils gegebenenfalls substituiert sind durch Substituenten ausgewählt aus Halogen, Hydroxy, Niederalkoxy, Niederalkenyloxy, Phenylniederalkoxy, Phenoxy, Mercapto, Niederalkylthio, Niederalkansulfinyl, -sulfonyl, Niederalkenylthio, Niederalkenylsulfinyl, -sulfonyl, Niederalkinylthio, Niederalkinylsulfinyl, -sulfonyl, Amino, welches gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylen-oxyniederalkylen disubstituiert ist, Carboxy, Niederalkoxy-, Niederalkoxyniederalkoxy- oder Niederalkenyloxy-carbonyl und Carbamoyl, in welchem die Aminogruppe gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, oder jeweils 3- bis 7-gliedriges Cycloalkyl bzw. Cycloalkenyl, Phenylniederalkyl, Phenylniederalkenyl, Phenylniederalkinyl, Phenyl, Naphthyl, Pyrrolyl, Pyrazolyl, Imidazolyl, Triazolyl, Tetrazolyl, Furyl, Thienyl oder Pyridyl bedeutet;

$R_2$ und $R_3$ unabhängig voneinander Halogen, Niederalkanoyl, Phenylniederalkanoyl, Benzoyl, Phenyl, Naphthyl, Pyrrolyl, Pyrazolyl, Imidazolyl, Triazolyl, Tetrazolyl, Furyl, Thienyl, Pyridyl, Amino, welches gegebenenfalls durch Niederalkyl, Niederalkenyl, Niederalkinyl, Phenylniederalkyl, Phenylniederalkinyl unabhängig voneinander mono-oder disubstituiert oder durch Niederalkylen oder Niederalkylenoxyniederalkylen disubstituiert ist, Carboxy, Niederalkoxy-, Niederalkoxyniederalkoxy- oder Niederalkenyloxy-carbonyl, Carbamoyl, in welchem die Aminogruppe gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, bedeuten; oder $R_2$ für $-Z_1-R_2'$ und $R_3$ für $-Z_2-R_3'$ stehen, wobei $Z_1$ und $Z_2$ unabhängig voneinander für eine Bindung, O oder $S(O)_n$ stehen und n für 0, 1 oder 2 steht, und $R_2'$ und $R_3'$ unabhängig voneinander Wasserstoff, Phenylniederalkyl, Phenylniederalkenyl oder Phenylniederalkinyl bedeuten oder für Niederalkyl, Niederalkenyl, Niederalkinyl, Niederalkoxyniederalkyl, -niederalkenyl oder -niederalkinyl, Niederalkenyloxyniederalkyl, -niederalkenyl oder -niederalkinyl, Niederalkylthioniederalkyl, -niederalkenyl oder -niederalkinyl, Niederalkansulfinylniederallkyl, -sulfonylniederalkyl, Niederalkenylthioniederalkyl, -sulfinylniederalkyl, -sulfonylniederalkyl, Niederalkinylthioniederalkyl, -sulfinylniederalkyl, -sulfonylniederalkyl stehen, welche jeweils unabhängig voneinander gegebenenfalls substituiert sind durch Substituenten ausgewählt aus Halogen, Hydroxy, Niederalkoxy, Niederalkenyloxy, Phenylniederalkoxy, Phenoxy, Mercapto, Niederalkylthio, Niederalkansulfinyl, -sulfonyl, Niederalkenylthio, Niederalkenylsulfinyl, -sulfonyl, Niederalkinylthio, Niederalkinylsulfinyl, -sulfonyl, Amino, welches gegebenenfalls durch Niederalkyl, Niederalkenyl, Niederalkinyl, Phenylniederalkyl, Phenylniederalkinyl unabhängig voneinander monooder disubstituiert oder durch Niederalkylen oder Niederalkylenoxyniederalkylen disubstituiert ist, Carboxy, Niederalkoxy-,. Niederalkoxyniederalkoxy- oder Niederalkenyloxy-carbonyl, Carbamoyl, in welchem die Aminogruppe gegebenenfalls durch Niederalkyl, Niederalkenyl, Niederalkyl, Phenylniederalkyl, Phenylniederalkinyl unabhängig voneinander mono- oder disubstituiert oder durch Niederalkylen oder Niederalkylenoxy niederalkylen disubstituiert ist;

oder $R_2$ und $R_3$ gemeinsam für Propylen oder Butylen oder für die Partialstruktur der Formel -CH=CH-CH=CH- stehen, in der gegebenenfalls eine oder zwei der Methingruppen (-CH=) durch -N= ersetzt ist;

$R_4$ für eine Gruppe der Formel (Ia), insbesondere (Ib), steht, in der

Alk Niederalkylen oder Niederalkyliden, insbesondere Methylen, bedeutet;

$R_5$ COOH, $SO_3H$, Halogenniederalkansulfamoyl, $PO_2H_2$, $PO_3H_2$ oder 5-Tetrazolyl bedeutet;

die Ringe A und B sowie die (hetero-)aromatischen Reste bzw. der durch $R_2$ und $R_3$ gemeinsam gebildete (hetero-)aromatische Ring jeweils unabhängig voneinander gegebenenfalls substituiert sind durch Substitu-

enten ausgewählt aus Halogen, Hydroxy, Niederalkoxy, Niederalkenyloxy, Phenylniederalkoxy, Phenoxy, Mercapto, Niederalkylthio, Niederalkansulfinyl, -sulfonyl, Niederalkenylthio, Niederalkenylsulfinyl, -sulfonyl, Niederalkinylthio, Niederalkinylsulfinyl, -sulfonyl, oder aus Niederalkyl, Niederalkenyl, Niederalkinyl, Niederalkoxyniederalkyl, -niederalkenyl, -niederalkinyl, Niederalkenyloxyniederalkyl, -niederalkenyl bzw. -niederalkinyl, welche jeweils gegebenenfalls substituiert sind durch Substituenten ausgewählt aus Halogen, Hydroxy, Niederalkoxy, Niederalkenyloxy, Phenylniederalkoxy, Phenoxy, Mercapto, Niederalkylthio, Niederalkansulfinyl, -sulfonyl, Niederalkenylthio, Niederalkenylsulfinyl, -sulfonyl, Niederalkinylthio, Niederalkinylsulfinyl, -sulfonyl, Amino, welches gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, Carboxy, Niederalkoxy-, Niederalkoxyniederalkoxy- oder Niederalkenyloxy-carbonyl, Carbamoyl, in welchem die Aminogruppe gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist.

4. Verbindungen gemäss Anspruch 2 der Formel I, ihre Tautomere und Salze, worin Z für O, S oder N(R) und R für Wasserstoff, Niederalkyl, Niederalkenyl oder Niederalkinyl steht,
$R_1$ gegebenenfalls durch Halogen oder Hydroxy substituiertes Niederalkyl, Niederalkenyl bzw. Niederalkinyl, jeweils 3- bis 7-gliedriges Cycloalkyl bzw. Cycloalkenyl, Phenylniederalkyl, Phenylniederalkenyl bzw. Phenylniederalkinyl oder Phenyl bedeutet, $R_2$ und $R_3$ unabhängig voneinander Halogen, Niederalkanoyl, Phenyl, Carboxy, welches gegebenenfalls durch einen Alkohol verestert ist, welcher sich von Niederalkyl, Niederalkenyl, Niederalkinyl, Niederalkoxyniederalkyl, -niederalkenyl, -niederalkinyl, Niederalkenyloxyniederalkyl, -niederalkenyl, -niederalkinyl ableitet, Carbamoyl, in welchem die Aminogruppe gegebenenfalls durch Niederalkyl, Niederalkenyl, Niederalkinyl, Phenylniederalkyl, -niederalkenyl oder -niederalkinyl unabhängig voneinander mono- oder disubstituiert oder durch Niederalkylen oder Niederalkylenoxyniederalkylen disubstituiert ist, oder $R_2$ für $-Z_1-R'_2$ und $R_3$ für $-Z_2-R'_3$ stehen, wobei $Z_1$ und $Z_2$ unabhängig voneinander für eine Bindung stehen oder O, $S(O)_n$ oder NH bedeuten, n für 0, 1 oder 2 steht, und $R'_2$ und $R'_3$ unabhängig voneinander Wasserstoff, gegebenenfalls durch Halogen, Hydroxy, Amino, welches gegebenenfalls wie vorstehend angegeben substituiert ist, Carboxy, welches gegebenenfalls wie vorstehend angegeben verestert ist, Carbamoyl, welches gegebenenfalls wie vorstehend angegeben substituiert ist, substituiertes Niederalkyl, Niederalkenyl bzw. Niederalkinyl, Niederalkoxyniederalkyl, -niederalkenyl, -niederalkinyl, Niederalkenyloxyniederalkyl, -niederalkenyl, -niederalkinyl, Niederalkylthioniederalkyl, -niederalkenyl, -niederalkinyl, Niederalkansulfinylniederalkyl, -sulfonylniederalkyl, Niederalkenylthioniederalkyl, -sulfinylniederalkyl, -sulfonylniederalkyl, Niederalkinylthioniederalkyl, -sulfinylniederalkyl bzw. -sulfonylniederalkyl bedeuten, $R_4$ für die Gruppe der Formel Ib steht, in der $R_5$ COOH, $SO_3H$, Halogenniederalkansulfamoyl, $PO_2H_2$, $PO_3H_2$ oder 5-Tetrazolyl bedeutet, wobei Phenyl bzw. Phenyl in Phenyl aufweisenden Resten jeweils unsubstituiert oder durch Substituenten ausgewählt aus der Gruppe bestehend aus Niederalkyl, Niederalkoxy, Halogen, Trifluormethyl und Hydroxy substituiert ist.

5. Verbindung gemäss Anspruch 1 der Formel I, ihre Tautomere und Salze, worin Z für O, S oder N(R) steht und R Wasserstoff oder Niederalkyl bedeutet, $R_1$ jeweils gegebenenfalls durch Halogen oder Hydroxy substituiertes Niederalkyl bzw. Niederalkenyl bedeutet oder $C_3$-$C_7$-Cycloalkyl, $C_3$-$C_7$-Cycloalkenyl, Phenylniederalkyl, Phenyl oder Pyridyl bedeutet;
$R_2$ und $R_3$ unabhängig voneinander Halogen, Niederalkanoyl, Phenylniederalkanoyl, Benzoyl, Phenyl, Tetrazolyl, Pyridyl, Amino, welches gegebenenfalls durch Niederalkyl oder Phenylniederalkyl mono- oder disubstituiert oder durch Niederalkylen oder Niederalkylenoxyniederalkylen disubstuiert ist, Carboxy, Niederalkoxy-, Niederalkoxyniederalkoxy- oder Niederalkenyloxy-carbonyl, Carbamoyl, in welchem die Aminogruppe gegebenenfalls durch Niederalkyl oder Phenylniederalkyl unabhängig voneinander mono- oder disubstituiert oder durch Niederalkylen oder Niederalkylenoxyniederalkylen disubstituiert ist, bedeuten; oder $R_2$ für $-Z_1-R_2'$ und $R_3$ für $-Z_2-R_3'$ stehen, wobei $Z_1$ und $Z_2$ unabhängig voneinander für eine Bindung, O oder $S(O)_n$ stehen und n für 0, 1 oder 2 steht; und
$R_2'$ und $R_3'$ unabhängig voneinander Wasserstoff oder Phenylniederalkyl bedeuten oder für Niederalkyl, Niederalkenyl, Niederalkoxyniederalkyl, Niederalkylthioniederalkyl, Niederalkansulfinylniederalkyl oder -sulfonylniederalkyl stehen, welche jeweils unabhängig voneinander gegebenenfalls substituiert sind durch Substituenten ausgewählt aus Halogen, Hydroxy, Niederalkoxy, Phenylniederalkoxy, Phenoxy, Amino, welches gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, Carboxy, Niederalkoxy-, Niederalkoxyniederalkoxy-carbonyl, Carbamoyl, in welchem die Aminogruppe gegebenenfalls durch Niederalkyl oder Phenylniederalkyl unabhängig voneinander mono- oder disubstituiert oder durch Niederalkylen oder Niederalkylenoxyniederalkylen disubstituiert ist; oder $R_2$ und $R_3$ gemeinsam für Propylen oder Butylen oder für die Partialstruktur der Formel -CH=CH-

CH=CH- stehen, in der gegebenenfalls eine oder zwei Methingruppe(n) durch =N- ersetzt ist;
$R_4$ für eine Gruppe der Formel Ia, in erster Linie Ib, steht, in der Alk Niederalkylen oder Niederalkyliden, in erster Linie Methylen, bedeutet;
$R_5$ Carboxy, Halogenniederalkansulfamoyl oder 5-Tetrazolyl bedeutet;
die Ringe A und B sowie die (hetero-)aromatischen Reste bzw. der durch $R_2$ und $R_3$ gemeinsam gebildete (hetero-)aromatische Ring jeweils unabhängig voneinander unsubstituiert oder substituiert sind durch Substituenten ausgewählt aus Halogen, Hydroxy, Niederalkoxy, Phenylniederalkoxy, Mercapto, Niederalkylthio, Niederalkansulfinyl, -sulfonyl, gegebenenfalls durch Halogen, Hydroxy, Niederalkoxy, Phenylniederalkoxy, Phenoxy substituiertes Niederalkyl bzw. Niederalkoxyniederalkoxyniederalkyl.

6. Verbindung gemäss Anspruch 1 der Formel I, ihre Tautomere und Salze,
worin Z für O, S oder N(R) und R für Wasserstoff oder Niederalkyl, steht, $R_1$ Niederalkyl, $C_3$-$C_7$-Cycloalkyl, Phenylniederalkyl oder Phenyl bedeutet, $R_2$ und $R_3$ unabhängig voneinander Halogen, Phenyl, Niederalkanoyl, Carboxy, Niederalkoxy-, Niederalkoxyniederalkoxy-carbonyl, Carbamoyl, Niederalkyl-, Diniederalkylcarbamoyl, Niederalkylen-carbamoyl, Niederalkylenoxyniederalkylen-carbamoyl, bedeutet, oder $R_2$ für -$Z_1$-$R'_2$ und $R_3$ für Z-$R'_3$ stehen, wobei $Z_1$ und $Z_2$ unabhängig voneinander für eine Bindung stehen oder O, S oder NH bedeuten und $R'_2$ und $R'_3$ unabhängig voneinander Wasserstoff, Niederalkyl, Halogenniederalkyl, Hydroxyniederalkyl, Niederalkoxyniederalkyl, Aminoniederalkyl, Niederalkylamino-niederalkyl, Diniederalkylaminoniederalkyl, Niederalkylenamino-niederalkyl, Niederalkylenoxyniederalkylenaminoniederalkyl, Carboxyniederalkyl, Niederalkoxy-, Niederalkoxyniederalkoxy-carbonyl niederalkyl, bedeuten, $R_4$ für die Gruppe der Formel Ib steht, in der $R_5$ COOH oder 5-Tetrazolyl bedeutet, wobei Phenyl bzw. Phenyl in Phenyl aufweisenden Resten jeweils unsubstituiert oder durch Niederalkyl, Niederalkoxy, Halogen, Trifluormethyl, und/oder Hydroxy substituiert ist.

7. Verbindung gemäss Anspruch 2 der Formel I, ihre Tautomere und Salze, worin Z für O steht, $R_1$ Niederalkyl, insbesondere mit 3 bis und mit 5 C-Atomen, wie n-Butyl, bedeutet, einer der Reste $R_2$ und $R_3$ Halogen, insbesondere mit Atomnummer bis und mit 35, wie Chlor, Phenyl, Carboxy, Niederalkoxycarbonyl, insbesondere mit 2 bis und mit 5 C-Atomen, wie Ethoxycarbonyl, Wasserstoff, Hydroxy, Niederalkylthio, insbesondere mit bis und mit 4 C-Atomen, wie Methylthio, Niederalkansulfonyl, insbesondere mit bis und mit 4 C-Atomen, wie Methansulfonyl, Amino, Diniederalkylamino, insbesondere mit bis und mit 4 C-Atomen je Niederalkylteil, wie Dimethylamino, Morpholino, Niederalkyl, insbesondere mit bis und mit 4 C-Atomen, wie Methyl, Hydroxyniederalkyl, insbesondere mit bis und mit 4 C-Atomen, wie Hydroxymethyl, Halogenniederalkyl, insbesondere mit Atomnummer bis und mit 35 und mit bis und mit 4 C-Atomen, wie Trifluormethyl, Diniederalkylaminoniederalkyl, insbesondere mit bis und mit 4 C-Atomen je Niederalkylteil, wie 2-Dimethylaminoethyl, Carboxyniederalkyl, insbesondere mit bis und mit 5 C-Atomen, wie Carboxymethyl, Niederalkoxycarbonylniederalkyl, insbesondere mit bis und mit 4 C-Atomen je Niederalkoxy- bzw. Niederalkylteil, wie Ethoxycarbonylmethyl, Niederalkoxy, insbesondere mit bis und mit 4 C-Atomen, wie Methoxy, Hydroxyniederalkoxy, insbesondere mit bis und mit 4 C-Atomen, wie 2-Hydroxyethoxy, Niederalkoxyniederalkoxy, insbesondere mit bis und mit 4 C-Atomen je Niederalkoxyteil, wie 2-Methoxyethoxy, Diniederalkylaminoniederalkoxy, insbesondere mit bis und mit 4 C-Atomen je Niederalkyl- bzw. Niederalkoxyteil, wie 2-Dimethylaminoethyl, Carboxyniederalkoxy, insbesondere mit bis und mit 5 C-Atomen, wie 2-Carboxyethoxy, oder Niederalkoxycarbonylniederalkoxy, insbesondere mit bis und mit 4 C-Atomen je Niederalkoxyteil, wie 2-Ethoxycarbonylethoxy, bedeutet und der andere Wasserstoff oder Niederalkyl, insbesondere mit bis und mit 4 C-Atomen, wie Methyl, bedeutet, und $R_4$ für die Gruppe der Formel Ib steht, in der $R_5$ 5-Tetrazolyl bedeutet.

8. Verbindung gemäss Anspruch 1 der Formel I, ihre Tautomere und Salze,
worin Z für O steht, $R_1$ Niederalkyl, insbesondere mit 3 bis und mit 5 C-Atomen, wie n-Propyl oder n-Butyl, oder Niederalkenyl, insbesondere mit 3 bis und mit 5 C-Atomen, wie 2-Propenyl, bedeutet, $R_2$ und $R_3$ unabhängig voneinander Halogen, insbesondere mit Atomnummer bis und mit 35, wie Chlor, Tetrazolyl, wie 5-Tetrazolyl, Amino, welches gegebenenfalls durch Niederalkyl oder Phenylniederalkyl, insbesondere mit bis und mit 4 C-Atomen je Alkylteil, mono- oder disubstituiert oder durch Niederalkylenoxyniederalkylen, insbesondere mit bis und mit 4 C-Atomen je Niederalkylenteil, wie 4-Morpholinyl, disubstituiert ist, Carboxy, Niederalkoxycarbonyl, insbesondere mit 2 bis und mit 5 C-Atomen, wie Methoxy- oder Ethoxycarbonyl, Niederalkoxyniederalkoxycarbonyl, insbesondere mit bis und mit 4 C-Atomen im Alkoxyteil, wie 2-Methoxyethoxycarbonyl, bedeuten; oder $R_2$ für -$Z_1$-$R_2'$ und $R_3$ für -$Z_2$-$R_3'$ stehen, wobei $Z_1$ und $Z_2$ unabhängig voneinander für eine Bindung, O oder $S(O)_n$ stehen und n für 0, 1 oder 2 steht, und $R_2'$ und $R_3'$ unabhängig voneinander Wasserstoff oder Phenylniederalkyl, insbesondere mit bis und mit 4 C-Atomen im Alkylteil, wie Benzyl, bedeuten oder Niederalkyl oder Niederalkoxyniederalkyl, insbesondere jeweils mit bis und mit 4 C-Atomen im Alkylteil, bedeuten, welche jeweils gegebenenfalls durch Halogen, insbesondere mit Atomnummer bis und 35, wie Chlor, Hydroxy, Amino, welches gegebenenfalls durch Niederalkyl oder

Phenylniederalkyl, insbesondere mit bis und mit 4 C-Atomen je Alkylteil, mono- oder disubstituiert oder durch Niederalkylenoxyniederalkyen, insbesondere mit bis und mit 4 C-Atomen je Niederalkyteil disubstituiert ist, wie Methyl-, Dimethyl-, Benzyl oder Dibenzylamino oder 4-Morpholinyl, Carboxy, Niederalkoxycarbonyl, insbesondere mit 2 bis und mit 5 C-Atomen, wie Methoxy- oder Ethoxycarbonyl, substituiert sind; oder $R_2$ und $R_3$ gemeinsam für Propylen oder Butylen oder für die Partialstruktur der Formel -CH = CH-CH = CH- stehen, in der gegebenenfalls eine oder zwei Methingruppe(n) durch = N- ersetzt ist;

$R_4$ für eine Gruppe der Formel Ia, insbesondere Ib, steht, in der Alk Niederalkylen oder Niederalkyliden, insbesondere jeweils mit bis und mit 4 C-Atomen, wie Methylen, Ethylen oder Ethyliden, in erster Linie Methylen, bedeutet;

$R_5$ Carboxy, Halogenniederalkansulfamoyl, insbesondere mit Atomnummer bis und mit 35 und bis und mit 4 C-Atomen, insbesondere Trifluormethansulfamoyl, oder 5-Tetrazolyl bedeutet;

die Ringe A und B sowie die aromatischen Reste bzw. der durch $R_2$ und $R_3$ gemeinsam gebildete (hetero-)aromatische Ring jeweils unabhängig voneinander unsubstituiert oder durch Hydroxy, Halogen, insbesondere mit Atomnummer bis und mit 35, wie Chlor, Trifluormethyl, Niederalkyl, insbesondere mit bis und mit 4 C-Atomen, wie Methyl, und/oder Niederalkoxy, insbesondere mit bis und mit 4 C-Atomen, wie Methoxy, substituiert sind.

9. Verbindungen gemäss Anspruch 1 der Formel I, ihre Tautomere und Salze, worin Z für O steht, $R_1$ $C_3$-$C_5$-Alkyl, wie n-Propyl oder n-Butyl, bedeutet, $R_2$ und $R_3$ unabhängig voneinander Halogen mit Atomnummer bis und mit 35, wie Chlor, Di-$C_1$-$C_4$-alkylamino, wie Dimethylamino, 4-Morpholinyl, Carboxy, $C_2$-$C_5$-Alkoxycarbonyl, wie Methoxy- oder Ethoxycarbonyl, bedeuten; oder $R_2$ für -$Z_1$-$R_2'$ und $R_3$ für -$Z_2$-$R_3'$ stehen, wobei $Z_1$ und $Z_2$ unabhängig voneinander für eine Bindung, O oder $S(O)_n$ stehen und n für 0, 1 oder 2 steht, und $R_2'$ und $R_3'$ unabhängig voneinander Wasserstoff, Phenyl-$C_2$-$C_5$-alkyl, wie Benzyl, oder $C_1$-$C_4$-Alkyl, wie Methyl, welches gegebenenfalls durch Hydroxy oder Di-$C_1$-$C_4$-alkylamino, wie Dimethylamino, substituiert ist, oder $C_1$-$C_4$-Alkoxy-$C_1$-$C_4$-alkoxy, wie 2-Methoxyethoxy, bedeuten; oder $R_2$ und $R_3$ gemeinsam für Propylen oder Butylen oder für die Partialstruktur der Formel -CH = CH-CH = CH- stehen, in der gegebenenfalls eine oder zwei Methingruppe(n) durch = N- ersetzt ist;

$R_4$ für eine Gruppe der Formel Ib steht, in der $R_5$ Carboxy oder in erster Linie 5-Tetrazolyl bedeutet; und die Ringe A und B bzw. der durch $R_2$ und $R_3$ gemeinsam gebildete (hetero-)aromatische Ring in erster Linie unsubstituiert, ferner durch Hydroxy, Halogen, insbesondere mit Atomnummer bis und mit 35, wie Chlor, Trifluormethyl, Niederalkyl, insbesondere mit bis und mit 4 C-Atomen, wie Methyl, und/oder Niederalkoxy, insbesondere mit bis und mit 4 C-Atomen, wie Methoxy, substituiert sind.

10. Verbindungen gemäss Anspruch 1 der Formel I, ihre Tautomere und Salze,

worin Z für O steht, $R_1$ $C_3$-$C_5$-Alkyl, wie n-Propyl oder n-Butyl, bedeutet, $R_2$ Halogen mit Atomnummer bis und mit 35, wie Chlor, Trifluormethyl, Carboxy, $C_2$-$C_5$-Alkoxycarbonyl, wie Ethoxycarbonyl, Wasserstoff, Hydroxy, $C_1$-$C_4$-Alkyl, wie Methyl oder n-Butyl, $C_1$-$C_4$-Alkoxy, wie Methoxy, Phenyl-$C_1$-$C_4$-alkoxy, wie Benzyloxy, Hydroxy-$C_1$-$C_4$-alkoxy, wie 2-Hydroxyethoxy, $C_1$-$C_4$-Alkoxy-$C_1$-$C_4$-alkoxy, wie 2-Methoxyethoxy, Di-$C_1$-$C_4$-alkylamino-$C_1$-$C_4$-alkoxy, wie 2-Dimethylaminoethoxy, bedeutet; $R_3$ Wasserstoff, $C_1$-$C_4$-Alkyl, wie Methyl oder n-Butyl, Carboxy, $C_2$-$C_5$-Alkoxycarbonyl, wie Ethoxycarbonyl, oder Hydroxy-$C_1$-$C_4$-alkyl, wie Hydroxymethyl, bedeutet; oder $R_2$ und $R_3$ gemeinsam für Propylen oder Butylen oder für die Partialstruktur der Formel -CH = CH-CH = CH- stehen, in der gegebenenfalls eine Methingruppe durch = N- ersetzt ist;

$R_4$ für eine Gruppe der Formel Ib steht, in der $R_5$ Carboxy oder in erster Linie 5-Tetrazolyl bedeutet; und die Ringe A und B in erster Linie unsubstituiert, ferner durch Hydroxy, Halogen mit Atomnummer bis und mit 35, wie Chlor, Trifluormethyl, $C_1$-$C_4$-Alkyl, wie Methyl, oder $C_1$-$C_4$-Alkoxy, wie Methoxy, substituiert sind.

11. Verbindungen gemäss Anspruch 1 der Formel I, ihre Tautomere und Salze,

worin Z für O steht, $R_1$ $C_3$-$C_5$-Alkyl, wie n-Propyl oder n-Butyl, bedeutet, $R_2$ $C_1$-$C_5$-Alkyl, wie Methyl, n-Propyl oder n-Butyl, oder Hydroxy bedeutet, $R_3$ Wasserstoff oder $C_1$-$C_5$-Alkyl, wie n-Propyl oder n-Butyl, bedeutet; oder $R_2$ und $R_3$ gemeinsam für Propylen oder Butylen oder für die Partialstruktur der Formel -CH = CH-CH = CH- stehen, in der gegebenenfalls eine Methingruppe durch = N- ersetzt ist;

$R_4$ für eine Gruppe der Formel Ib steht, in der $R_5$ Carboxy oder in erster Linie 5-Tetrazolyl bedeutet; und die Ringe A und B in erster Linie unsubstituiert oder ferner durch Hydroxy, Halogen mit Atomnummer bis und mit 35, wie Chlor, Trifluormethyl, $C_1$-$C_4$-Alkyl, wie Methyl, oder $C_1$-$C_4$-Alkoxy, wie Methoxy, substituiert sind.

12. Verbindungen gemäss Anspruch 1 der Formel I und ihre Salze,

worin Z für O steht, $R_1$ $C_3$-$C_5$-Alkyl, wie n-Propyl oder n-Butyl, bedeutet, $R_2$ und $R_3$ gemeinsam für Propylen oder Butylen oder für die Partialstruktur der Formel -CH = CH-CH = CH- stehen, in der gegebenenfalls eine Methingruppe durch = N- ersetzt ist; $R_4$ für eine Gruppe der Formel Ib steht, in der $R_5$ 5-Tetrazolyl bedeutet; und die Ringe A und B unsubstituiert sind.

13. Verbindung gemäss Anspruch 2 der Formel I und ihre Salze, worin Z für O steht, $R_1$ Alkyl mit 3 bis und mit 5 C-Atomen, wie n-Butyl, bedeutet, $R_2$ Wasserstoff, Halogen mit Atomnummer bis und mit 35, wie Chlor, Alkyl mit bis und mit 4 C-Atomen, wie Methyl, Alkoxy mit bis und mit 4 C-Atomen, wie Methoxy, bedeutet und $R_3$ Wasserstoff bedeutet, und $R_4$ für die Gruppe der Formel Ia steht, in der $R_5$ 5-Tetrazolyl bedeutet.

14. Verbindung gemäss Anspruch 2 der Formel I und ihre Salze, worin Z für O steht, $R_1$ Alkyl mit 3 bis und mit 5 C-Atomen, wie n-Butyl, bedeutet, $R_2$ Wasserstoff, Halogen mit Atomnummer bis und mit 35, wie Chlor, oder Alkyl mit bis und mit 4 C-Atomen, wie Methyl, bedeutet, $R_3$ Wasserstoff ist, $R_4$ für die Gruppe der Formel Ia steht und $R_5$ 5-Tetrazolyl bedeutet.

15. Verbindungen gemäss Anspruch 1 der Formel I und ihre Salze,
worin Z für O steht, $R_1$ $C_3$-$C_5$-Alkyl, wie n-Butyl, bedeutet, $R_2$ $C_1$-$C_4$-Alkyl, wie Methyl oder n-Butyl, und $R_3$ $C_1$-$C_4$-Alkyl, wie n-Butyl bedeuten, oder $R_2$ Hydroxy und $R_3$ Wasserstoff oder Methyl bedeuten, $R_4$ für die Gruppe der Formel Ib steht, in der $R_5$ 5-Tetrazolyl bedeutet.

16. 2-n-Butyl-1,6-dihydro-1-[(2'-(1H-tetrazolyl-5-yl)biphenyl-4-yl)-methyl]-6-oxopyrimidin oder ein Salz davon.

17. 2-n-Butyl-1,6-dihydro-4-methyl-1-[(2'-tetrazol-5-yl)biphenyl-4-yl)methyl]-6-oxo-pyrimidin oder ein Salz davon.

18. 2-n-Butyl-1,6-dihydro-4-butyl-1-[(2'-tetrazol-5-yl)biphenyl-4-yl)methyl]-6-oxo-pyrimidin oder ein Salz davon.

19. 2-n-Butyl-1,6-dihydro-4,5-dimethyl-1-[(2'-tetrazol-5-yl)biphenyl-4-yl)methyl]-6-oxo-pyrimidin oder ein Salz davon.

20. 2-n-Butyl-1,6-dihydro-4-methyl-5-propyl-1-[(2'-tetrazol-5-yl)biphenyl-4-yl)methyl]-6-oxo-pyrimidin oder ein Salz davon.

21. 2-n-Butyl-1,6-dihydro-4-hydroxy-1-[2'-(1H-tetrazol-5-yl)biphenyl-4-yl)methyl]-6-oxopyrimidin oder ein Salz davon.

22. 2-n-Butyl-1,6-dihydro-4-hydroxy-5-methyl-1-[2'-(1H-tetrazol-5-yl)biphenyl-4-yl)methyl]-6-oxopyrimidin oder ein Salz davon.

23. 2-n-Butyl-3-[(2'-(1H-tetrazol-5-yl)biphenyl-4-yl)methyl]- 3,5,6,7-tetrahydro-4H-cyclopentapyrimidin-4-on oder ein Salz davon.

24. 2-n-Butyl-3-[(2'-(1H-tetrazol-5-yl)biphenyl-4-yl)methyl]-5,6,7,8-tetrahydro-3H-quinazolin-4-on oder ein Salz davon.

25. Verbindung gemäss einem der Ansprüche 1-24 in Form eines pharmazeutisch verwendbaren Salzes.

26. Verbindung gemäss einem der Ansprüche 1-25 zur therapeutischen Behandlung des menschlichen oder tierischen Körpers.

27. Pharmazeutische Präparate enthaltend als Wirkstoff eine Verbindung gemäss einem der Ansprüche 1-24 in freier Form oder in Form eines pharmazeutisch verwendbaren Salzes.

28. Verwendung einer Verbindung gemäss einem der Ansprüche 1-24 in freier Form oder in Form eines pharmazeutisch verwendbaren Salzes zur Herstellung von Angiotensin-II- antagonisierend wirkenden pharmazeutischen Präparaten und von Antihypertensiva.

29. Verfahren zur Herstellung einer Verbindung gemäss einem der Ansprüche 1-25,
dadurch gekennzeichnet, dass man
a) eine Verbindung der Formel

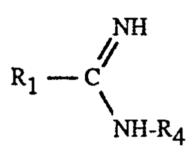

(IIa)

oder ein Salz davon mit einer Verbindung der Formel

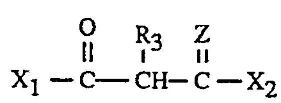

(IIb)

einem Salz, einem Tautomeren oder einem funktionell abgewandelten Derivat des Tautomeren davon

umsetzt, worin $X_1$ für die Variable $R_2$ oder für verethertes Hydroxy steht und $X_2$ verethertes Hydroxy bedeutet, oder

b) in einer Verbindung der Formel

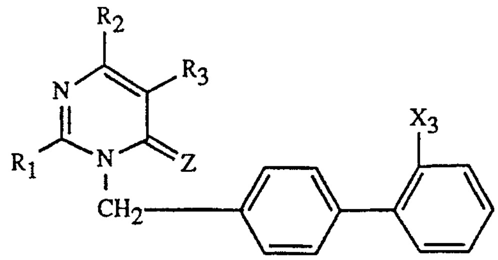

(III)

oder einem Salz davon, worin $X_3$ einen in die Variable $R_5$ überführbaren Rest bedeutet, $X_3$ in die Variable $R_5$ überführt, oder

c) eine Verbindung der Formel

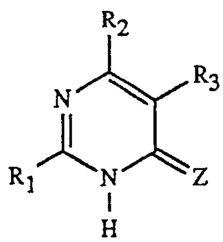

(IVa),

ein Tautomeres oder Salz davon mit einer Verbindung der Formel

$X_4$-$R_4$ (IVb)

oder einem Salz davon, worin $X_4$ für reaktionsfähiges verestertes Hydroxy steht, umsetzt,

und, wenn erwünscht, eine verfahrensgemäss oder auf andere Weise erhältliche Verbindung der Formel I oder ein Salz davon in eine andere erfindungsgemässe Verbindung oder ein Salz davon überführt, eine verfahrensgemäss erhältliche freie Verbindung der Formel in ein Salz überführt, ein verfahrensgemäss erhältliches Salz in die freie Verbindung der Formel I oder in ein anderes Salz überführt, ein verfahrensgemäss erhältliches Gemisch von Isomeren auftrennt und die gewünschte Verbindung isoliert.

30. Die nach dem Verfahren gemäss Anspruch 29 erhältlichen Verbindungen.

Patentansprüche für folgende Vertragssataaten ES und GR

1. Verfahren zur Herstellung einer Verbindung

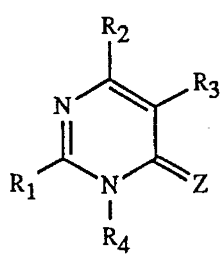

(I),

ihrer Tautomere und Salze, worin

Z für O, S oder N(R) und R für Wasserstoff oder einen aliphatischen Kohlenwasserstoffrest steht;

$R_1$ einen gegebenenfalls substituierten aliphatischen Kohlenwasserstoffrest, einen cycloaliphatischen oder araliphatischen Kohlenwasserstoffrest oder einen aromatischen Rest bedeutet;

$R_2$ und $R_3$ unabhängig voneinander Halogen, Acyl, einen aromatischen Rest, gegebenenfalls substituiertes Amino, gegebenenfalls verestertes oder amidiertes Carboxy bedeuten; oder

$R_2$ für $-Z_1-R'_2$ und $R_3$ für $-Z_2-R'_3$ stehen, wobei $Z_1$ und $Z_2$ unabhängig voneinander für eine Bindung, O oder $S(O)_n$ stehen und n für 0, 1 oder 2 steht, und $R'_2$ und $R'_3$ unabhängig voneinander für Wasserstoff, einen araliphatischen oder aliphatischen Kohlenwasserstoffrest stehen, wobei letzterer gegebenenfalls substituiert und gegebenenfalls durch -O-oder $-S(O)_n-$ unterbrochen ist, wobei n für 0,1 oder 2 steht; oder $R_2$ und $R_3$ gemeinsam für Propylen oder Butylen oder für die Partialstruktur der Formel -CH=CH-CH=CH-, in der gegebenenfalls eine oder zwei der Methingruppen durch -N= ersetzt ist;
$R_4$ für eine Gruppe der Formel

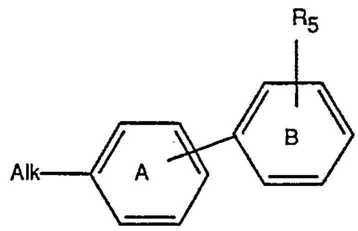

(Ia)

steht, in der
Alk einen zweiwertigen aliphatischen Kohlenwasserstoff bedeutet;
$R_5$ COOH, $SO_3H$, Halogenalkansulfamoyl, $PO_2H_2$, $PO_3H_2$ oder 5-Tetrazolyl bedeutet;
die Ringe A und B bzw. der durch $R_2$ und $R_3$ gemeinsam gebildete (hetero-)aromatische Ring unabhängig voneinander gegebenenfalls substituiert sind,
dadurch gekennzeichnet, dass man
a) eine Verbindung der Formel

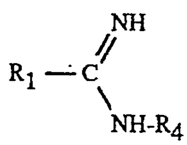

(IIa)

oder ein Salz davon mit einer Verbindung der Formel

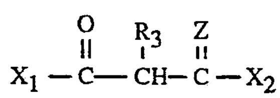

(IIb)

einem Salz, einem Tautomeren oder einem funktionell abgewandelten Derivat des Tautomeren davon umsetzt, worin $X_1$ für die Variable $R_2$ oder für verethertes Hydroxy steht und $X_2$ verethertes Hydroxy bedeutet, oder
b) in einer Verbindung der Formel

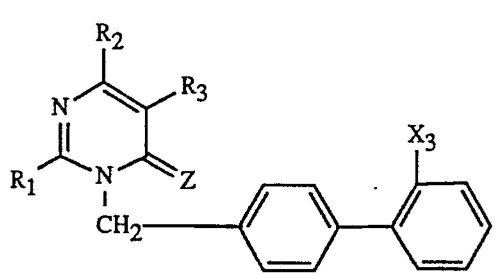

(III)

oder einem Salz davon, worin $X_3$ einen in die Variable $R_5$ überführbaren Rest bedeutet, $X_3$ in die Variable $R_5$ überführt, oder
c) eine Verbindung der Formel

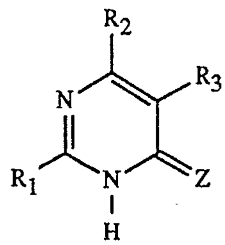

(IVa),

ein Tautomeres oder Salz davon mit einer Verbindung der Formel

$X_4$-$R_4$ (IVb)

oder einem Salz davon, worin $X_4$ für reaktionsfähiges verestertes Hydroxy steht, umsetzt, und, wenn erwünscht, eine verfahrensgemäss oder auf andere Weise erhältliche Verbindung der Formel I oder ein Salz davon in eine andere erfindungsgemässe Verbindung oder ein Salz davon überführt, eine verfahrensgemäss erhältliche freie Verbindung der Formel I in ein Salz überführt, ein verfahrensgemäss erhältliches Salz in die freie Verbindung der Formel I oder in ein anderes Salz überführt, ein verfahrensgemäss erhältliches Gemisch von Isomeren auftrennt und die gewünschte Verbindung isoliert.

2. Verfahren gemäss Anspruch 1 zur Herstellung von Verbindungen der Formel I, ihrer Tautomeren und Salze, worin

Z für O, S oder N(R) und R für Wasserstoff oder einen aliphatischen Kohlenwasserstoffrest steht,

$R_1$ einen gegebenenfalls durch Halogen oder Hydroxy substituierten aliphatischen Rest, eine cycloaliphatischen oder araliphatischen Kohlenwasserstoffrest oder einen aromatischen Rest bedeutet,

$R_2$ und $R_3$ unabhängig voneinander Halogen, Acyl, einen aromatischen Rest, gegebenenfalls verestertes oder amidiertes Carboxy bedeuten, oder

$R_2$ für -$Z_1$-$R'_2$ und $R_3$ für -$Z_2$-$R'_3$ stehen, wobei $Z_1$ und $Z_2$ unabhängig voneinander für eine Bindung stehen oder O, $S(O)_n$ oder NH bedeuten, n für 0, 1 oder 2 steht und $R'_2$ und $R'_3$ unabhängig voneinander für Wasserstoff oder einen aliphatischen Kohlenwasserstoffrest stehen, der gegebenenfalls durch Halogen, Hydroxy, gegebenenfalls substituiertes Amino, gegebenenfalls verestertes oder amidiertes Carboxy substituiert ist und der gegebenenfalls durch -O- oder $S(O)_n$- unterbrochen ist, wobei in für 0, 1 oder 2 steht,

$R_4$ für eine Gruppe der Formel

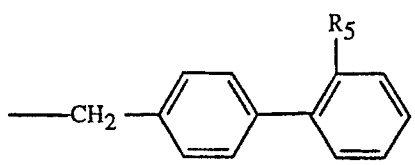

(Ib)

steht, in der

$R_5$ COOH, $SO_3H$, Halogenalkansulfamoyl, $PO_2H_2$, $PO_3H_2$ oder 5-Tetrazolyl bedeutet.

3. Verfahren gemäss Anspruch 1 zur Herstellung von Verbindungen der Formel I, ihrer Tautomeren und Salze, worin

Z für O, S oder N(R) steht und R Wasserstoff, Niederalkyl, Niederalkenyl oder Niederalkinyl bedeutet;

$R_1$ Niederalkyl, Niederalkenyl bzw. Niederalkinyl bedeutet, welche jeweils gegebenenfalls substituiert sind durch Substituenten ausgewählt aus Halogen, Hydroxy, Niederalkoxy, Niederalkenyloxy, Phenylniederalkoxy, Phenoxy, Mercapto, Niederalkylthio, Nieder alkansulfinyl, -sulfonyl, Niederalkenylthio, Niederalkenylsulfinyl, -sulfonyl, Niederalkinylthio, Niederalkinylsulfinyl, -sulfonyl, Amino, welches gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, Carboxy, Niederalkoxy-, Niederalkoxyniederalkoxy-oder Niederalkenyloxy-carbonyl und Carbamoyl, in welchem die Aminogruppe gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, oder jeweils 3- bis 7-gliedriges Cycloalkyl bzw. Cycloalkenyl, Phenylniederalkyl, Phenylniederalkenyl, Phenylniederalkinyl, Phenyl, Naphthyl, Pyrrolyl, Pyrazolyl, Imidazolyl, Tetrazolyl, Furyl, Thienyl oder Pyridyl bedeutet;

$R_2$ und $R_3$ unabhängig voneinander Halogen, Niederalkanoyl, Phenylniederalkanoyl, Benzoyl, Phenyl, Naphthyl, Pyrrolyl, Pyrazolyl, Imidazolyl, Triazolyl, Tetrazolyl, Furyl, Thienyl, Pyridyl, Amino, welches

gegebenenfalls durch Niederalkyl, Niederalkenyl, Niederalkinyl, Phenylniederalkyl, Phenylniederalkinyl unabhängig voneinander mono-oder disubstituiert oder durch Niederalkylen oder Niederalkylenoxyniederalkylen disubstituiert ist, Carboxy, Niederalkoxy-, Niederalkoxyniederalkoxy- oder Niederalkenyloxy-carbonyl, Carbamoyl, in welchem die Aminogruppe gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, bedeuten; oder $R_2$ für $-Z_1-R_2'$ und $R_3$ für $-Z_2-R_3'$ stehen, wobei $Z_1$ und $Z_2$ unabhängig voneinander für eine Bindung, O oder $S(O)_n$ stehen und n für 0, 1 oder 2 steht, und $R_2'$ und $R_3'$ unabhängig voneinander Wasserstoff, Phenylniederalkyl, Phenylniederalkenyl oder Phenylniederalkinyl bedeuten oder für Niederalkyl, Niederalkenyl, Niederalkinyl, Niederalkoxyniederalkyl, -niederalkenyl oder -niederalkinyl, Niederalkenyloxyniederalkyl, -niederalkenyl oder -niederalkinyl, Niederalkylthioniederalkyl, -niederalkenyl oder -niederalkinyl Niederalkansulfinylniederalkyl, -sulfonylniederalkyl, Niederalkenylthioniederalkyl, -sulfinylniederalkyl, -sulfonylniederalkyl, Niederalkinylthioniederalkyl, -sulfinylniederalkyl, -sulfonylniederalkyl stehen, welche jeweils unabhängig voneinander gegebenenfalls substituiert sind durch Substituenten ausgewählt aus Halogen, Hydroxy, Niederalkoxy, Niederalkenyloxy, Phenylniederalkoxy, Phenoxy, Mercapto, Niederalkylthio, Niederalkansulfinyl, -sulfonyl, Niederalkenylthio, Niederalkenylsulfinyl, -sulfonyl, Niederalkinylthio, Niederalkinylsulfinyl, -sulfonyl, Amino, welches gegebenenfalls durch Niederalkyl, Niederalkenyl, Niederalkinyl, Phenylniederalkyl, Phenylniederalkinyl unabhängig voneinander mono- oder disbustituiert oder durch Niederalkylen oder Niederalkylenoxyniederalkylen disubstituiert ist, Carboxy, Niederalkoxy-, Niederalkoxyniederalkoxy- oder Niederalkenyloxy-carbonyl, Carbamoyl, in welchem die Aminogruppe gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disbustituiert ist;

oder $R_2$ und $R_3$ gemeinsam für Propylen oder Butylen oder für die Partialstruktur der Formel -CH=CH-CH=CH- stehen, in der gegebenenfalls eine oder zwei der Methingruppen (-CH=) durch -N= ersetzt ist;

$R_4$ für eine Gruppe der Formel (Ia), insbesondere (Ib), steht, in der

Alk Niederalkylen oder Niederalkyliden, insbesondere Methylen, bedeutet;

$R_5$ COOH, $SO_3H$, Halogenniederalkansulfamoyl, $PO_2H_2$, $PO_3H_2$ oder 5-Tetrazolyl bedeutet;

die Ringe A und B sowie die (hetero-)aromatischen Reste bzw. der durch $R_2$ und $R_3$ gemeinsam gebildete (hetero-)aromatische Ring jeweils unabhängig voneinander gegebenenfalls substituiert sind durch Substituenten ausgewählt aus Halogen, Hydroxy, Niederalkoxy, Niederalkenyloxy, Phenylniederalkoxy, Phenoxy, Mercapto, Niederalkylthio, Niederalkansulfinyl, -sulfonyl, Niederalkenylthio, Niederalkenylsulfinyl, -sulfonyl, Niederalkinylthio, Niederalkinylsulfinyl, -sulfonyl, oder aus Niederalkyl, Niederalkenyl, Niederalkinyl, Niederalkoxyniederalkyl, -niederalkenyl, -niederalkinyl, Niederalkenyloxyniederalkyl, - niederalkenyl bzw. -niederalkinyl, welche jeweils gegebenenfalls substituiert sind durch Substituenten ausgewählt aus Halogen, Hydroxy, Niederalkoxy, Niederalkenyloxy, Phenylniederalkoxy, Phenoxy, Mercapto, Niederalkylthio, Niederalkansulfinyl, -sulfonyl, Niederalkenylthio, Niederalkenylsulfinyl, -sulfonyl, Niederalkinylthio, Niederalkinylsulfinyl, -sulfonyl, Amino, welches gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, Carboxy, Niederalkoxy-, Niederalkoxyniederalkoxy- oder Niederalkenyloxy-carbonyl, Carbamoyl, in welchem die Aminogruppe gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist.

4. Verfahren gemäss Anspruch 1 zur Herstellung von Verbindungen der Formel I, ihrer Tautomeren und Salze, worin

worin Z für O, S oder N(R) und R für Wasserstoff, Niederalkyl, Niederalkenyl oder Niederalkinyl steht,

$R_1$ gegebenenfalls durch Halogen oder Hydroxy substituiertes Niederalkyl, Niederalkenyl bzw. Niederalkinyl, jeweils 3- bis 7-gliedriges Cycloalkyl bzw. Cycloalkenyl, Phenylniederalkyl, Phenylniederalkenyl bzw. Phenylniederalkinyl oder Phenyl bedeutet, $R_2$ und $R_3$ unabhängig voneinander Halogen, Niederalkanoyl, Phenyl, Carboxy, welches gegebenenfalls durch einen Alkohol verestert ist, welcher sich von Niederalkyl, Niederalkenyl, Niederalkinyl, Niederalkoxyniederalkyl, -niederalkenyl, -niederalkinyl, Niederalkenyloxyniederalkyl, -niederalkenyl, -niederalkinyl ableitet, Carbamoyl, in welchem die Aminogruppe gegebenenfalls durch Niederalkyl, Niederalkenyl, Niederalkinyl, Phenylniederalkyl, -niederalkenyl oder -niederalkinyl unabhängig voneinander mono- oder disubstituiert oder durch Niederalkylen oder Niederalkylenoxyniederalkylen disubstituiert ist, oder $R_2$ für $-Z_1-R'_2$ und $R_3$ für $-Z_2-R'_3$ stehen, wobei $Z_1$ und $Z_2$ unabhängig voneinander für eine Bindung stehen oder O, $S(O)_n$ oder NH bedeuten, n für 0, 1 oder 2 steht, und $R'_2$ und $R'_3$ unabhängig voneinander Wasserstoff, gegebenenfalls durch Halogen, Hydroxy, Amino, welches gegebenenfalls wie vorstehend angegeben substituiert ist, Carboxy, welches gegebenenfalls wie vorstehend angegeben verestert ist, Carbamoyl, welches gegebenenfalls wie vorstehend angegeben substituiert ist, substituiertes

Niederalkyl, Niederalkenyl bzw. Niederalkinyl, Niederalkoxyniederalkyl, -niederalkenyl, -niederalkinyl, Niederalkenyloxyniederalkyl, -niederalkenyl, -niederalkinyl, Niederalkylthioniederalkyl, -niederalkenyl, -niederalkinyl, Niederalkansulfinylniederalkyl, -sulfonylniederalkyl, Niederalkenylthioniederalkyl, -sulfinylniederalkyl, -sulfonylniederalkyl, Niederalkinylthioniederalkyl, -sulfinylniederalkyl bzw. - sulfonylniederalkyl bedeuten, $R_4$ für die Gruppe der Formel Ib steht, in der $R_5$ COOH, $SO_3H$, Halogenniederalkansulfamoyl, $PO_2H_2$, $PO_3H_2$ oder 5-Tetrazolyl bedeutet, wobei Phenyl bzw. Phenyl in Phenyl aufweisenden Resten jeweils unsubstituiert oder durch Substituenten ausgewählt aus der Gruppe bestehend aus Niederalkyl, Niederalkoxy, Halogen, Trifluormethyl und Hydroxy substituiert ist.

5. Verfahren gemäss Anspruch 1 zur Herstellung von Verbindungen der Formel I, ihrer Tautomeren und Salze, worin

worin Z für O, S oder N(R) steht und R Wasserstoff oder Niederalkyl bedeutet, $R_1$ jeweils gegebenenfalls durch Halogen oder Hydroxy substituiertes Niederalkyl bzw. Niederalkenyl bedeutet oder $C_3$-$C_7$-Cycloalkyl, $C_3$-$C_7$-Cycloalkenyl, Phenylniederalkyl, Phenyl oder Pyridyl bedeutet;

$R_2$ und $R_3$ unabhängig voneinander Halogen, Niederalkanoyl, Phenylniederalkanoyl, Benzoyl, Phenyl, Tetrazolyl, Pyridyl, Amino, welches gegebenenfalls durch Niederalkyl oder Phenylniederalkyl mono- oder disubstituiert oder durch Niederalkylen oder Niederalkylenoxyniederalkylen disubstuiert ist, Carboxy, Niederalkoxy-, Niederalkoxyniederalkoxy- oder Niederalkenyloxy-carbonyl, Carbamoyl, in welchem die Aminogruppe gegebenenfalls durch Niederalkyl oder Phenylniederalkyl unabhängig voneinander mono- oder disubstituiert oder durch Niederalkylen oder Niederalkylenoxyniederalkylen disubstituiert ist, bedeuten; oder $R_2$ für $-Z_1-R_2'$ und $R_3$ für $-Z_2-R_3'$ stehen, wobei $Z_1$ und $Z_2$ unabhängig voneinander für eine Bindung, O oder $S(O)_n$ stehen und n für 0, 1 oder 2 steht; und $R_2'$ und $R_3'$ unabhängig voneinander Wasserstoff oder Phenylniederalkyl bedeuten oder für Niederalkyl, Niederalkenyl, Niederalkoxyniederalkyl, Niederalkylthioniederalkyl, Niederalkansulfinylniederalkyl oder -sulfonylniederalkyl stehen, welche jeweils unabhängig voneinander gegebenenfalls substituiert sind durch Substituenten ausgewählt aus Halogen, Hydroxy, Niederalkoxy, Phenylniederalkoxy, Phenoxy, Amino, welches gegebenenfalls durch Niederalkyl, Niederalkenyl, Niederalkinyl, Phenylniederalkyl, Phenylniederalkinyl unabhängig voneinander mono- oder disubstituiert oder durch Niederalkylen oder Niederalkylenoxyniederalkylen disubstituiert ist, Carboxy, Niederalkoxy-, Niederalkoxyniederalkoxy-carbonyl, Carbamoyl, in welchem die Aminogruppe gegebenenfalls durch Niederalkyl oder Phenylniederalkyl unabhängig voneinander mono- oder disubstituiert oder durch Niederalkylen oder Niederalkylenoxyniederalkylen disubstituiert ist; oder $R_2$ und $R_3$ gemeinsam für Propylen oder Butylen oder für die Partialstruktur der Formel -CH=CH-CH=CH- stehen, in der gegebenenfalls eine oder zwei Methingruppe(n) durch =N- ersetzt ist;

$R^4$ für eine Gruppe der Formel Ia, in erster Linie Ib, steht, in der Alk Niederalkylen oder Niederalkyliden, in erster Linie Methylen, bedeutet;

$R_5$ Carboxy, Halogenniederalkansulfamoyl oder 5-Tetrazolyl bedeutet;

die Ringe A und B sowie die (hetero-)aromatischen Reste bzw. der durch $R_2$ und $R_3$ gemeinsam gebildete (hetero-)aromatische Ring jeweils unabhängig voneinander unsubstituiert oder substituiert sind durch Substituenten ausgewählt aus Halogen, Hydroxy, Niederalkoxy, Phenylniederalkoxy, Mercapto, Niederalkylthio, Niederalkansulfinyl, -sulfonyl, gegebenenfalls durch Halogen, Hydroxy, Niederalkoxy, Phenylniederalkoxy, Phenoxy substituiertes Niederalkyl bzw. Niederalkoxyniederalkoxyniederalkyl.

6. Verfahren gemäss Anspruch 1 zur Herstellung von Verbindungen der Formel I, ihrer Tautomeren und Salze, worin

worin Z für O, S oder N(R) und R für Wasserstoff oder Niederalkyl, steht, $R_1$ Niederalkyl, $C_3$-$C_7$-Cycloalkyl, Phenylniederalkyl oder Phenyl bedeutet, $R_2$ und $R_3$ unabhängig voneinander Halogen, Phenyl, Niederalkanoyl, Carboxy, Niederalkoxy-, Niederalkoxy niederalkoxy-carbonyl, Carbamoyl, Niederalkyl-, Diniederalkylcarbamoyl, Niederalkylen-carbamoyl, Niederalkylenoxyniederalkylen-carbamoyl, bedeutet, oder $R_2$ für $-Z_1-R'_2$ und $R_3$ für $Z-R'_3$ stehen, wobei $Z_1$ und $Z_2$ unabhängig voneinander für eine Bindung stehen oder O, S oder NH bedeuten und $R'_2$ und $R'_3$ unabhängig voneinander Wasserstoff, Niederalkyl, Halogenniederalkyl, Hydroxyniederalkyl, Niederalkoxyniederalkyl, Aminoniederalkyl, Niederalkylamino-niederalkyl, Diniederalkylaminoniederalkyl, Niederalkylenamino-niederalkyl, Niederalkylenoxyniederalkylenaminoniederalkyl, Carboxyniederalkyl, Niederalkoxy-, Niederalkoxyniederalkoxy-carbonylniederalkyl, bedeuten, $R_4$ für die Gruppe der Formel Ib steht, in der $R_5$ COOH oder 5-Tetrazolyl bedeutet, wobei Phenyl bzw. Phenyl in Phenyl aufweisenden Resten jeweils unsubstituiert oder durch Niederalkyl, Niederalkoxy, Halogen, Trifluormethyl, und/oder Hydroxy substituiert ist.

7. Verfahren gemäss Anspruch 1 zur Herstellung von Verbindungen der Formel I, ihrer Tautomeren und Salze, worin

worin Z für O steht, $R_1$ Niederalkyl, insbesondere mit 3 bis und mit 5 C-Atomen, wie n-Butyl, bedeutet, einer der Reste $R_2$ und $R_3$ Halogen, insbesondere mit Atomnummer bis und mit 35, wie Chlor, Phenyl,

Carboxy, Niederalkoxycarbonyl, insbesondere mit 2 bis und mit 5 C-Atomen, wie Ethoxycarbonyl, Wasserstoff, Hydroxy, Niederalkylthio, insbesondere mit bis und mit 4 C-Atomen, wie Methylthio, Niederalkansulfonyl, insbesondere mit bis und mit 4 C-Atomen, wie Methansulfonyl, Amino, Diniederalkylamino, insbesondere mit bis und mit 4 C-Atomen je Niederalkylteil, wie Dimethylamino, Morpholino, Niederalkyl, insbesondere mit bis und mit 4 C-Atomen, wie Methyl, Hydroxyniederalkyl, insbesondere mit bis und mit 4 C-Atomen, wie Hydroxymethyl, Halogenniederalkyl, insbesondere mit Atomnummer bis und mit 35 und mit bis und mit 4 C-Atomen, wie Trifluormethyl, Diniederalkylaminoniederalkyl, insbesondere mit bis und mit 4 C-Atomen je Niederalkylteil, wie 2-Dimethylaminoethyl, Carboxyniederalkyl, insbesondere mit bis und mit 5 C-Atomen, wie Carboxymethyl, Niederalkoxycarbonylniederalkyl, insbesondere mit bis und mit 4 C-Atomen je Niederalkoxy- bzw. Niederalkylteil, wie Ethoxycarbonylmethyl, Niederalkoxy, insbesondere mit bis und mit 4 C-Atomen, wie Methoxy, Hydroxyniederalkoxy, insbesondere mit bis und mit 4 C-Atomen, wie 2-Hydroxyethoxy, Niederalkoxyniederalkoxy, insbesondere mit bis und mit 4 C-Atomen je Niederalkoxyteil, wie 2-Methoxyethoxy, Diniederalkylaminoniederalkoxy, insbesondere mit bis und mit 4 C-Atomen je Niederalkyl- bzw. Niederalkoxyteil, wie 2-Dimethylaminoethyl, Carboxyniederalkoxy, insbesondere mit bis und mit 5 C-Atomen, wie 2-Carboxyethoxy, oder Niederalkoxycarbonylniederalkoxy, insbesondere mit bis und mit 4 C-Atomen je Nieder alkoxyteil, wie 2-Ethoxycarbonylethoxy, bedeutet und der andere Wasserstoff oder Niederalkyl, insbesondere mit bis und mit 4 C-Atomen, wie Methyl, bedeutet, und $R_4$ für die Gruppe der Formel Ib steht, in der $R_5$ 5-Tetrazolyl bedeutet.

8. Verfahren gemäss Anspruch 1 zur Herstellung von Verbindungen der Formel I, ihrer Tautomeren und Salze, worin

worin Z für O steht, $R_1$ Niederalkyl, insbesondere mit 3 bis und mit 5 C-Atomen, wie n-Propyl oder n-Butyl, oder Niederalkenyl, insbesondere mit 3 bis und mit 5 C-Atomen, wie 2-Propenyl, bedeutet, $R_2$ und $R_3$ unabhängig voneinander Halogen, insbesondere mit Atomnummer bis und mit 35, wie Chlor, Tetrazolyl, wie 5-Tetrazolyl, Amino, welches gegebenenfalls durch Niederalkyl oder Phenylniederalkyl, insbesondere mit bis und mit 4 C-Atomen je Alkylteil, mono- oder disubstituiert oder durch Niederalkylenoxyniederalkylen, insbesondere mit bis und mit 4 C-Atomen je Niederalkylenteil, wie 4-Morpholinyl, disubstituiert ist, Carboxy, Niederalkoxycarbonyl, insbesondere mit 2 bis und mit 5 C-Atomen, wie Methoxy- oder Ethoxycarbonyl, Niederalkoxyniederalkoxycarbonyl, insbesondere mit bis und mit 4 C-Atomen im Alkoxyteil, wie 2-Methoxyethoxycarbonyl, bedeuten; oder $R_2$ für $-Z_1-R_2'$ und $R_3$ für $-Z_2-R_3'$ stehen, wobei $Z_1$ und $Z_2$ unabhängig voneinander für eine Bindung, O oder $S(O)_n$ stehen und n für 0, 1 oder 2 steht, und $R_2'$ und $R_3'$ unabhängig voneinander Wasserstoff oder Phenylniederalkyl, insbesondere mit bis und mit 4 C-Atomen im Alkylteil, wie Benzyl, bedeuten oder Niederalkyl oder Niederalkoxyniederalkyl, insbesondere jeweils mit bis und mit 4 C-Atomen im Alkylteil, bedeuten, welche jeweils gegebenenfalls durch Halogen, insbesondere mit Atomnummer bis und 35, wie Chlor, Hydroxy, Amino, welches gegebenenfalls durch Niederalkyl oder Phenylniederalkyl, insbesondere mit bis und mit 4 C-Atomen je Alkylteil, mono- oder disubstituiert oder durch Niederalkylenoxynieeralkyen, insbesondere mit bis und mit 4 C-Atomen je Niederalkyteil disubstituiert ist, wie Methyl-, Dimethyl-, Benzyl oder Dibenzylamino oder 4-Morpholinyl, Carboxy, Niederalkoxycarbonyl, insbesondere mit 2 bis und mit 5 C-Atomen, wie Methoxy- oder Ethoxycarbonyl, substituiert sind; oder $R_2$ und $R_3$ gemeinsam für Propylen oder Butylen oder für die Partialstruktur der Formel -CH = CH-CH = CH- stehen, in der gegebenenfalls eine oder zwei Methingruppe(n) durch = N- ersetzt ist;

$R_4$ für eine Gruppe der Formel Ia, insbesondere Ib, steht, in der Alk Niederalkylen oder Niederalkyliden, insbesondere jeweils mit bis und mit 4 C-Atomen, wie Methylen, Ethylen oder Ethyliden, in erster Linie Methylen, bedeutet;

$R_5$ Carboxy, Halogenniederalkansulfamoyl, insbesondere mit Atomnummer bis und mit 35 und bis und mit 4 C-Atomen, insbesondere Trifluormethansulfamoyl, oder 5-Tetrazolyl bedeutet;

die Ringe A und B sowie die aromatischen Reste bzw. der durch $R_2$ und $R_3$ gemeinsam gebildete (hetero-)aromatische Ring jeweils unabhängig voneinander unsubstituiert oder durch Hydroxy, Halogen, insbesondere mit Atomnummer bis und mit 35, wie Chlor, Trifluormethyl, Niederalkyl, insbesondere mit bis und mit 4 C-Atomen, wie Methyl, und/oder Niederalkoxy, insbesondere mit bis und mit 4 C-Atomen, wie Methoxy, substituiert sind.

9. Verfahren gemäss Anspruch 1 zur Herstellung von Verbindungen der Formel I, ihrer Tautomeren und Salze, worin

worin Z für O steht, $R_1$ $C_3$-$C_5$-Alkyl, wie n-Propyl oder n-Butyl, bedeutet, $R_2$ und $R_3$ unabhängig voneinander Halogen mit Atomnummer bis und mit 35, wie Chlor, Di-$C_1$-$C_4$-alkylamino, wie Dimethylamino, 4-Morpholinyl, Carboxy, $C_2$-$C_5$-Alkoxycarbonyl, wie Methoxy- oder Ethoxycarbonyl, bedeuten; oder $R_2$ für $-Z_1-R_2'$ und $R_3$ für $-Z_2-R_3'$ stehen, wobei $Z_1$ und $Z_2$ unabhängig voneinander für eine Bindung, O oder $S(O)_n$ stehen und n für 0, 1 oder 2 steht, und $R_2'$ und $R_3'$ unabhängig voneinander Wasserstoff, Phenyl-$C_2$-$C_5$-alkyl, wie Benzyl, oder $C_1$-$C_4$-Alkyl, wie Methyl, welches gegebenenfalls durch Hydroxy oder Di-$C_1$-$C_4$-

alkylamino, wie Dimethylamino, substituiert ist, oder $C_1$-$C_4$-Alkoxy-$C_1$-$C_4$-alkoxy, wie 2-Methoxyethoxy, bedeuten; oder $R_2$ und $R_3$ gemeinsam für Propylen oder Butylen oder für die Partialstruktur der Formel -CH = CH-CH = CH- stehen, in der gegebenenfalls eine oder zwei Methingruppe(n) durch = N- ersetzt ist;
$R_4$ für eine Gruppe der Formel Ib steht, in der $R_5$ Carboxy oder in erster Linie 5-Tetrazolyl bedeutet; und die Ringe A und B bzw. der durch $R_2$ und $R_3$ gemeinsam gebildete (hetero-)aromatische Ring in erster Linie unsubstituiert, ferner durch Hydroxy, Halogen, insbesondere mit Atomnummer bis und mit 35, wie Chlor, Trifluormethyl, Niederalkyl, insbesondere mit bis und mit 4 C-Atomen, wie Methyl, und/oder Niederalkoxy, insbesondere mit bis und mit 4 C-Atomen, wie Methoxy, substituiert sind.

10. Verfahren gemäss Anspruch 1 zur Herstellung von Verbindungen der Formel I, ihrer Tautomeren und Salze, worin
worin Z für O steht, $R_1$ $C_3$-$C_5$-Alkyl, wie n-Propyl oder n-Butyl, bedeutet, $R_2$ Halogen mit Atomnummer bis und mit 35, wie Chlor, Trifluormethyl, Carboxy, $C_2$-$C_5$-Alkoxycarbonyl, wie Ethoxycarbonyl, Wasserstoff, Hydroxy, $C_1$-$C_4$-Alkyl, wie Methyl oder n-Butyl, $C_1$-$C_4$-Alkoxy, wie Methoxy, Phenyl-$C_1$-$C_4$-alkoxy, wie Benzyloxy, Hydroxy-$C_1$-$C_4$-alkoxy, wie 2-Hydroxyethoxy, $C_1$-$C_4$-Alkoxy-$C_1$-$C_4$-alkoxy, wie 2-Methoxyethoxy, Di-$C_1$-$C_4$-alkylamino-$C_1$-$C_4$-alkoxy, wie 2-Dimethylaminoethoxy, bedeutet; $R_3$ Wasserstoff, $C_1$-$C_4$-Alkyl, wie Methyl oder n-Butyl, Carboxy, $C_2$-$C_5$-Alkoxycarbonyl, wie Ethoxycarbonyl, oder Hydroxy-$C_1$-$C_4$-alkyl, wie Hydroxymethyl, bedeutet; oder $R_2$ und $R_3$ gemeinsam für Propylen oder Butylen oder für die Partialstruktur der Formel -CH = CH-CH = CH- stehen, in der gegebenenfalls eine Methingruppe durch = N- ersetzt ist;
$R_4$ für eine Gruppe der Formel Ib steht, in der $R_5$ Carboxy oder in erster Linie 5-Tetrazolyl bedeutet; und die Ringe A und B in erster Linie unsubstituiert, ferner durch Hydroxy, Halogen mit Atomnummer bis und mit 35, wie Chlor, Trifluormethyl, $C_1$-$C_4$-Alkyl, wie Methyl, oder $C_1$-$C_4$-Alkoxy, wie Methoxy, substituiert sind.

11. Verfahren gemäss Anspruch 1 zur Herstellung von Verbindungen der Formel I, ihrer Tautomeren und Salze, worin
worin Z für O steht, $R_1$ $C_3$-$C_5$-Alkyl, wie n-Propyl oder n-Butyl, bedeutet, $R_2$ $C_1$-$C_5$-Alkyl, wie Methyl, n-Propyl oder n-Butyl, oder Hydroxy bedeutet, $R_3$ Wasserstoff oder $C_1$-$C_5$-Alkyl, wie n-Propyl oder n-Butyl, bedeutet; oder $R_2$ und $R_3$ gemeinsam für Propylen oder Butylen oder für die Partialstruktur der Formel -CH = CH-CH = CH- stehen, in der gegebenenfalls eine Methingruppe durch = N- ersetzt ist;
$R_4$ für eine Gruppe der Formel Ib stehet, in der $R_5$ Carboxy oder in erster Linie 5-Tetrazolyl bedeutet; und die Ringe A und B in erster Linie unsubstituiert oder ferner durch Hydroxy, Halogen mit Atomnummer bis und mit 35, wie Chlor, Trifluormethyl, $C_1$-$C_4$-Alkyl, wie Methyl, oder $C_1$-$C_4$-Alkoxy, wie Methoxy, substituiert sind.

12. Verfahren gemäss Anspruch 1 zur Herstellung von Verbindungen der Formel I, ihrer Tautomeren und Salze, worin
worin Z für O steht, $R_1$ $C_3$-$C_5$-Alkyl, wie n-Propyl oder n-Butyl, bedeutet, $R_2$ und $R_3$ gemeinsam für Propylen oder Butylen oder für die Partialstruktur der Formel -CH = CH-CH = CH- stehen, in der gegebenenfalls eine Methingruppe durch = N- ersetzt ist;
$R_4$ für eine Gruppe der Formel Ib steht, in der $R_5$ 5-Tetrazolyl bedeutet; und die Ringe A und B unsubstituiert sind.

13. Verfahren gemäss Anspruch 1 zur Herstellung von Verbindungen der Formel I, ihrer Tautomeren und Salze, worin
worin Z für O steht, $R_1$ Alkyl mit 3 bis und mit 5 C-Atomen, wie n-Butyl, bedeutet, $R_2$ Wasserstoff, Halogen mit Atomnummer bis und mit 35, wie Chlor, Alkyl mit bis und mit 4 C-Atomen, wie Methyl, Alkoxy mit bis und mit 4 C-Atomen, wie Methoxy, bedeutet und $R_3$ Wasserstoff bedeutet, und $R_4$ für die Gruppe der Formel Ia steht, in der $R_5$ 5-Tetrazolyl bedeutet.

14. Verfahren gemäss Anspruch 1 zur Herstellung von Verbindungen der Formel I, ihrer Tautomeren und Salze worin
worin Z für O steht, $R_1$ Alkyl mit 3 bis und mit 5 C-Atomen, wie n-Butyl, bedeutet, $R_2$ Wasserstoff, Halogen mit Atomnummer bis und mit 35, wie Chlor, oder Alkyl mit bis und mit 4 C-Atomen, wie Methyl, bedeutet, $R_3$ Wasserstoff ist, $R_4$ für die Gruppe der Formel Ia steht und $R_5$ 5-Tetrazolyl bedeutet.

15. Verfahren gemäss Anspruch 1 zur Herstellung von Verbindungen der Formel I, ihrer Tautomeren und Salze, worin
worin Z für O steht, $R_1$ $C_3$-$C_5$-Alkyl, wie n-Butyl, bedeutet, $R_2$ $C_1$-$C_4$-Alkyl, wie Methyl oder n-Butyl, und $R_3$ $C_1$-$C_4$-Alkyl, wie n-Butyl bedeuten, oder $R_2$ Hydroxy und $R_3$ Wasserstoff oder Methyl bedeuten, $R_4$ für die Gruppe der Formel Ib steht, in der $R_5$ 5-Tetrazolyl bedeutet.

16. Verfahren gemäss Anspruch 1 zur Herstellung von 2-n-Butyl-1,6-dihydro-1-[(2'-(1H-tetrazolyl-5-yl)-biphenyl-4-yl)-methyl]-6-oxopyrimidin oder eines Salzes davon.

17. Verfahren gemäss Anspruch 1 zur Herstellung von 2-n-Butyl-1,6-dihydro-4-methyl-1-[(2'-tetrazol-5-yl)-

biphenyl-4-yl)methyl]-6-oxo-pyrimidin oder eines Salzes davon.

18. Verfahren gemäss Anspruch 1 zur Herstellung von 2-n-Butyl-1,6-dihydro-4-butyl-1-[(2′-tetrazol-5-yl)-biphenyl-4-yl)methyl]-6-oxo-pyrimidin oder eines Salzes davon.

19. Verfahren gemäss Anspruch 1 zur Herstellung von 2-n-Butyl-1,6-dihydro-4,5-dimethyl-1-[(2′-tetrazol-5-yl)biphenyl-4-yl)methyl]-6-oxo-pyrimidin oder eines Salzes davon.

20. Verfahren gemäss Anspruch 1 zur Herstellung von 2-n-Butyl-1,6-dihydro-4-methyl-5-propyl-1-[(2′-tetrazol-5-yl)biphenyl-4-yl)methyl]-6-oxo-pyrimidin oder eines Salz davon.

21. Verfahren gemäss Anspruch 1 zur Herstellung von 2-n-Butyl-1,6-dihydro-4- hydroxy-1-[2′-(1H-tetrazol-5-yl)biphenyl-4-yl)methyl]-6-oxopyrimidin oder eines Salzes davon.

22. Verfahren gemäss Anspruch 1 zur Herstellung von 2-n-Butyl-1,6-dihydro-4-hydroxy-5-methyl-1-[2′-(1H-tetrazol-5-yl)biphenyl-4-yl)methyl]-6-oxopyrimidin oder eines Salzes davon.

23. Verfahren gemäss Anspruch 1 zur Herstellung von 2-n-Butyl-3-[(2′-(1H-tetrazol-5-yl)biphenyl-4-yl)-methyl]-3,5,6,7-tetrahydro-4H-cyclopentapyrimidin-4-on oder eines Salzes davon.

24. Verfahren gemäss Anspruch 1 zur Herstellung von 2-n-Butyl-3-[(2′-(1H-tetrazol-5-yl)biphenyl-4-yl)-methyl]-5,6,7,8-tetrahydro-3H-quinazolin-4-on oder eines Salzes davon.

25. Verfahren gemäss Anspruch 1 zur Herstellung einer Verbindung gemäss einem der Ansprüche 1-24 in Form eines pharmazeutisch verwendbaren Salzes.

26. Verfahren zur Herstellung von pharmazeutischen Präparaten enthaltend ein Verbindung gemäss einem der Ansprüche 1-25, dadurch gekennzeichnet, dass man den Wirkstoff mit geeigneten Hilfs- und Zusatzstoffen zu pharmazeutischen Präparaten verarbeitet.

27. Verwendung von Verbindungen gemäss einem der Ansprüche 1-25 zur Herstellung von Angiotensin-II-antagonisierend wirkenden pharmazeutischen Präparaten und von Antihypertensiva.